# EUROPEAN PATENT APPLICATION

(11) **EP 2 527 445 A2**
(43) Date of publication of application: **28.11.2012**
(21) Application number: 12181612.8
(22) Date of filing: 16.05.2008
(51) Int. Cl.: C12N 15/113, C12N 15/85, A61K 31/713, A61P 31/16

(54) **Treatment and prevention of influenza**

(30) Priority: 16.05.2007 US 938315 P; 16.05.2007 AU 2007902616
(62) Divisional of application: 08747962.2
(71) Applicant: MAT Malta Advanced Technologies Limited, St Julians ST J3311 (MT); Commonwealth Scientific and Industrial Research Organisation, Campbell, ACT 2612 (AU)
(72) Inventor: Doran, Timothy James, Ocen Grove Victoria 3226 (AU); McKay, James Climie, Geelong Victoria 3220 (AU); Moore, Robert John, Ascot Vale Victoria 3032 (AU); Lowenthal, John William, Belmont Victoria 3220 (AU); Tyack, Scott Geoffrey, Grovedale Victoria 3216 (AU)
(74) Representative: Almond-Martin, Carol

(57) **Abstract**

The invention relates to a nucleic acid construct encoding at least three RNA molecules comprising a double-stranded region, wherein the RNA molecules reduce influenza A virus replication in an animal cell and/or reduce production of infectious influenza A virus particles in an animal cell and/or reduce the expression of an influenza A virus polypeptide in an influenza A virus infected animal cell when compared to an isogenic influenza A virus infected animal cell lacking the RNA molecule, wherein the double-stranded regions comprise nucleotide sequences at least 95% identical to sequences selected from:
(i) SEQ ID NO:7, SEQ ID NO:9 and SEQ ID NO:12,
(ii) SEQ ID NO:6, SEQ ID NO:7 and SEQ ID NO:8, and
(iii) SEQ ID NO:9, SEQ ID NO:13 and SEQ ID NO:15.

SEQUENCE LISTING

<110>  MAT Malta Advanced Technologies Limited
       Commonwealth Scientific and Industrial Research
       Organisation

<120>  Treatment and prevention of influenza

<130>  B08595A

<150>  US 60/938,315
<151>  2007-05-16

<150>  AU 2007902616
<151>  2007-05-16

<160>  65

<170>  PatentIn version 3.5

<210>  1
<211>  2341
<212>  RNA
<213>  Artificial

<220>
<223>  Consensus sequence of Influenza A PB2 gene

<400>  1
agcraaagca ggucaaauau auucaauaug gagagaauaa aagaauuamg agaucuaaug      60

ucacaguccc gcacucgcga gauacuaaca aaaaccacug uggaccauau ggccauaauc     120

aagaaauaca caucaggaag acaagagaag aacccugcuc ucagaaugaa auggaugaug     180

gcaaugaaau auccaaucac agcggacaag agaauaauag agaugauucc ugaaaggaau     240

gaacaagggc agacgcucug gagcaagaca aaugaugcug gaucggacag ggugauggug     300

ucuccccuag cuguaacuug guggaauagg aaugggccgr cgacaagugc aguccauuau     360

ccaaagguuu acaaaacaua cuuugagaag guugaaaggu uaaaacaugg aaccuucggu     420

cccguucauu uccgaaacca aguuaaaaua cgccgccgag uugauauaaa uccuggccau     480

gcagaucuca gugcuaaaga agcacaagau gucaucaugg aggucguuuu cccaaaugaa     540

gugggagcua gaauauugac aucagagucg caauugacaa uaacgaaaga gaagaaagaa     600

gagcuccaag auuguaagau ugcucccuua augguugcau acauguugga aagggaacug     660

guccgcaaaa ccagauuccu accgguagca ggcggaacaa gcagugugua cauugaggua     720

uugcauuuga cucaagggac cugcugggaa cagauguaca cuccaggcgg agaagugaga     780

aaugacgaug uugaccagag uyugaucauc gcugccagaa acauuguuag gagagcaacg     840

guaucagcgg auccacuggc aucacugcug gagauguguc acagcacaca aauugguggg     900

auaaggaugg uggacauccu uaggcaaaau ccaacugagg aacaagcugu ggauauaugc     960

aaagcagcaa ugggucugag gaucaguucw uccuuuagcu uuggaggcuu cacuuucaaa    1020

agaacaagug gaucauccgu camgaaggaa gaggaagugc uuacaggcaa ccuccaaaca    1080

uugaaaauaa gaguacauga gggguaugag gaauucacaa ugguugggcg gagggcaaca 1140

gcuauccuga ggaaagcaac uagaaggcug auucaguuga uaguaagugg aagagacgaa 1200

caaucaaucg cugaggcaau cauuguagca augguguucu cacaggagga uugcaugaua 1260

aaggcagucc gaggcgaucu gaauuucgua aacagagcaa accaaagauu aaaccccaug 1320

caucaacucc ugagacauuu ucaaaaggay gcaaaagugc uauuucagaa uuggggaauu 1380

gaacccauug auaaugucau ggggaugauc ggaauauuac cugacaugac ucccagcaca 1440

gaaaugucac ugagaggagu aagaguuagu aaaaugggag uggaugaaua uuccagcacu 1500

gagagaguag uuguaaguau ugaccguuuc uuaaggguuc gagaucagcg ggggaacgua 1560

cucuuaucuc ccgaagaggu cagcgaaacc cagggaacag agaaauugac aauaacauau 1620

ucaucaucaa ugauguggga aaucaacggu ccugagucag ugcuuguuaa caccuaucaa 1680

uggaucauca gaaacuggga gacugugaag auucaauggu cucaagaccc cacgaugcug 1740

uacaauaaga uggaguuuga accguuccaa uccuugguac cyaaagcugc cagaggucaa 1800

uacaguggau uugugagaac auuauuccaa caaaugcgug acguacuggg gacauuugau 1860

acuguccaga uaauaaagcu gcuaccauuu gcagcagccc caccggagca gagcagaaug 1920

caguuuucuu cucuaacugu gaaugugaga ggcucaggaa ugagaauacu cguaaggggc 1980

aauuccccug uguucaacua caauaaggca accaaaaggc uuaccguucu uggaaaggac 2040

gcaggugcau uaacagagga uccagaugag gggacagccg gaguggaauc ugcaguacug 2100

aggggauucy uaauucuagg caaggaggac aaaagauaug gaccagcauu gagcaucaau 2160

gaacugagca aucuugcgaa aggggagaaa gcuaaugugc ugauagggca aggagacgug 2220

guguugguaa ugaaacgraa acgggacucu agcauacuua cugacagcca gacagcgacc 2280

aaaagaauuc ggauggccau caauuagugu cgaauuguuu aaaaacgacc uuguuucuac 2340

w 2341

<210> 2
<211> 2341
<212> RNA
<213> Artificial

<220>
<223> Consensus sequence of the Influenza A PB1 gene

<400> 2
   agcgaaagca ggcaaaccau uugaauggau gucaauccga cuuuacuuuu cuugaaagua 60
   ccagugcaaa augcuauaag uaccacauuc ccuuauacug gagacccucc auacagccau 120
   ggaacaggga caggauacac cauggacaca gucaacagaa cacaccaaua uucagaaaag 180
   gggaagugga caacaaacac agagacugga gcaccccaac ucaacccgau ugauggacca 240
   cuaccugagg auaaugagcc caguggguau gcacaaacag auuguguauu ggaagcaaug 300
   gcuuuccuug aagaauccca cccagggauc uuugaaaacu cgugucuuga aacgauggaa 360
   auuguucaac aaacaagagu ggauaaacug acccaagguc gccagaccua ugacuggaca 420
   uugaauagaa accaaccggc ugcaacugcu uuggccaaca cuauagaaau cuucagaucg 480
   aacggucuaa cagccaauga aucgggacgg cuaauagauu uccucaagga ugugauggaa 540
   ucaauggaua aggaagaaau ggagauaaca acacauuucc agagaaagag aagrgugagg 600
   gacaacauga ccaagaaaau ggucacacaa agaacaauag ggaagaaaaa acaaaggcug 660
   aacaaaaaga gcuaccugau aagagcacug acacugaaca caaugacaaa agaugcagaa 720
   agaggcaaau ugaagaggcg agcrauugca acacccggaa ugcaaaucag aggauucgus 780
   uacuuuguug aaacacuagc gaggaguauc ugugagaaac uugagcaauc uggacuccca 840
   gucggaggga augagaagaa ggcuaaauug gcaaacgucg ugaggaagau gaugacuaac 900
   ucacaagaua cugaacucuc cuuuacaauu acuggagaca auaccaaaug gaaugagaau 960
   cagaauccua ggauguuucu ggcaaugaua acruacauca caaggaacca gccagaaugg 1020
   uuucggaaug ucuuaagcau ugcyccuaua auguucucaa acaaaauggc gagayuagga 1080
   aaaggauaca uguucgaaag uaagagcaug aaguuacgaa cacaaauacc agcagaaaug 1140
   cuugcaaaca uugaucuuaa auacuucaau gaauuaacga aaaagaaaau ugagaaaaua 1200
   agrccucuau uaauagaugg uacagccuca uugagcccug gaaugaugau gggcauguuc 1260
   aacaugcuga guacaguccu aggaguyuca auccugaauc uuggacagaa aagguacacc 1320
   aaaaccacau auugguggga cggacuccaa uccucugaug auuucgcucu caucguaaau 1380
   gcaccgaauc augagggaau acaagcagga guggauaggu uuuauaggac uuguaaacua 1440
   guuggaauca auaugagcaa gaagaagucu uacauaaauc ggacagggac auuugaauuc 1500
   acgagcuuuu ucuaccgcua uggauuugua gccaauuuca guauggagcu gcccaguuuu 1560
   ggagugucug gaauuaauga aucggccgac augagcauug guguuacagu gauaaaraac 1620
   aauaugauaa acaacgaccu ugggccagca acagcucaga uggcucuuca gyuauucauc 1680
   aaggacuaca gauacacaua ccgaugccac agaggggaua cgcaaaucca aacraggaga 1740
   ucauucgagc ugaagaagcu gugggagcaa acccguucaa aggcaggacu guugguuuca 1800
   gauggaggac caaaucuaua caauauccga aaucuccaua uuccugargu cugcuuraaa 1860
   ugggaauuga uggaugaaga uuaccagggc agacugugua auccucugaa uccruucguc 1920
   agccauaagg aaauugaauc ugucaacaau gcuguaguaa ugccagcuca uggcccggcc 1980
   aagaguaugg aauaugaugc cguugcaacu acacauucau ggauuccuaa aaggaaycgu 2040
   uccauucuca auacgaguca aaggggaauu cuugaggaug aacagaugua ccagaagugc 2100
   ugcaaucuau ucgagaaauu cuuccccagc aguucauauc ggaggccagu uggaauuucc 2160
   agcauggugg aggccauggu gucuagggcc cgaauugacg cacgaauuga uuucgagucu 2220
   ggaaggauua agaaagaaga guuugcugag aucaugaaga ucuguuccac cauugaagar 2280
   cucagacggc aaaaauagug aauuuagcuu guccuucgug aaaaaaugcc uuguuucuac 2340
   u 2341

<210> 3
<211> 2181
<212> RNA
<213> Artificial

<220>
<223> Consensus sequence of the Influenza A PA gene

<400> 3
auggaagacu uugugcgaca augcuucaau ccaaugauug ucgarcuugc ggaaaaggca 60

augaaagaau auggggaaga uccgaaaauc gaaacgaaca aguuugcugc aauaugcaca 120

cacuuggagg ucuguuucau guauucggau uuucacuuua uugaugaacg gagugaauca 180

auaauuguag aaucuggaga uccgaaugca uuauugaaac accgauuuga aauaauugaa 240

ggaagagacc gaacgauggc cuggacugug gugaauagua ucugcaacac cacaggaguu 300

gagaaaccua aauuucuccc agauuuguau gacuacaaag agaaccgauu caucgaaauu 360

ggagugacac ggagggaagu ucauacauac uaucuggaga aagccaacaa gauaaaaucc 420

gagaagacac auauucacau auucucauuc acaggggagg aaauggccac caaagcggac 480

uacacccuug augaagagag cagggcaaga auuaaaacca ggcuguucac cauaaggcag 540

gaaauggcca guaggggucu augggauucc uuucgucaau ccgagagagg cgaagagaca 600

auugaagara aauuugaaau cacuggaacc augcgcagac uugcagacca aagucuccca 660

ccgaacuucu ccagccuuga aaacuuuaga gccuaugugg auggauucga accgaacggc 720

ugcauugagg gcaagcuuuc ucaaauguca aaagaaguga augcuagaau ugagccauuu 780

uugaagacaa cgccacgccc ucucagacua ccugaugggc cuccuugcuc ucagcggucg 840

aaguucuugc ugauggaugc ccuuaaauua agcaucgaag acccgaguca ugagggggag 900

gggauaccac uauacgaugc aaucaaaugc augaagacau uuuucggcug gaaagagccc 960

aacaucguga aaccacauga aaaagguaua aaccccaauu accuccuggc uuggaagcaa 1020

gugcuggcag aacuccaaga uauugaaaau gaggagaaaa ucccaaaaac aaagaacaug 1080

aaaaaaacaa gccaguugaa gugggcacum ggugagaaca uggcaccaga gaaaguagac 1140

uuugaggacu gcaaagaugu uagcgaucua agacaguaug acagugauga accagagucu 1200

agaucacuag caagcuggau ucagagugaa uucaacaagg caugugaauu gacagauucg 1260

aguuggauug aacuugayga aauaggagaa gacguagcuc caauugagca cauugcaagu 1320

augagaagga acuauuuuac agcggaagua ucccauugca gggccacuga auacauaaug 1380

aagggagugu acauaaacac agcccuguug aaugcauccu gugcagccau ggaugacuuu 1440

caacugauuc caaugauaag caaaugcaga accaaagaag gaagacggaa aacuaaucug 1500

uauggauuca uuauaaaagg gagaucccac uugaggaaug auaccgaugu gguaaauuuu 1560

gugaguaugg aauucucucu uacugauccg agrcuggagc cacacaagug ggaaaaguac 1620

uguguccucg agauaggaga caugcuccuc cggacugcag uaggccaagu uucamggccc 1680

auguuccugu auguaagaac caauggaacc uccaagauca aaaugaaaug gggcauggaa 1740

augaggcgau gccuucuuca aucccuucaa caaauugaaa gcaugauuga agccgagucu 1800

ucugucaaag agaaggacau gaccaaagaa uucuuugaaa ayaaaucaga aacauggccg 1860

auuggagagu cccccaaggg aguggaggaa ggcuccaucg gaaaggugug cagaaccuug 1920

cuggcgaagu cuguguucaa caguuuauau gcaucuccac aacucgaggg guuuucagcu 1980

gaaucaagaa aauugcuucu cauugcucag gcacuuaggg acaaccugga accuggaccc 2040

uucgaucuug gagggcuaua ugaagcaauu gaggagugcc ugauuaacga ucccuggguu 2100

uugcuuaaug cgucuugguu caacuccuuc cucgcacaug cacugaaaua guuguggcaa 2160

ugcuacuauu ugcuauccau a 2181

<210> 4
<211> 1565
<212> RNA
<213> Artificial

<220>
<223> Consensus sequence of the Influenza A NP

<400> 4
agcaaaagca ggguagauaa ucacucaccg agugacauca acaucauggc gucucaaggc 60

accaaacgau cuuaugaaca gauggaaacu gguggagaac gccagaaugc uacugagauc 120

agggcaucug uuggaagaau gguuaguggc auugggaggu ucuacauaca gaugugcaca 180

gaacucaaac ucagugacua ugaagggagg cugauccaga acagcauaac aauagagaga 240

augguacucu cugcauuuga ugaaagaagg aacagauacc uggaagaaca ccccagugcg 300

gggaaggacc cgaagaarac uggaggucca auuuaucgga ggagagacgg gaaaugggug 360

agagagcura uucuguacga caaagaggag aucaggagga uuuggcguca agcgaacaau 420

ggagaggacg caacugcugg ucuyacccac cugaugauau ggcauuccaa ucuaaaugau 480

gccacauauc agagaacgag agcucucgug cguacuggaa uggaccccag gaugugcucu 540

cugaugcaag grucaacucu cccgaggaga ucuggagcug ccggugcagc aguraagggg 600

guagggacaa uggugaugga gcugauucgg augauaaaac gagggaucaa cgaccggaau 660

uucuggagag gcgaaaaugg aagaagaaca aggauugcau augagagaau gugcaacauc 720

cucaaaggga aauuccaaac agcagcacaa agagcaauga uggaucaagu gcgagagagc 780

agaaauccug ggaaugcuga aauugaagau cucauuuuuc uggcacgguc ugcacucauc 840

cugagaggau caguggccca uaaguccugc uugccugcuu guguguacgg acuugcagug 900

gccaguggau augacuuuga gagagaaggg uacucucugg uuggaauaga uccuuuccgy 960

cugcuucaaa acagccaggu cuuuagucuc auuagaccaa augagaaucc agcacauaag 1020

agucaauuag uguggauggc augccacucu gcagcauuug aggaccuuag agucucaagu 1080

uucaucagag ggacaagagu ggucccaaga ggacarcuau ccaccagagg gguucaaauu 1140

gcuucaaaug agaacaugga rgcaauggac uccaacacuc uugaacugag aagyagauau 1200

ugggcuauaa gaaccagaag cggaggaaac accaaccagc agagggcauc ugcaggacag 1260

aucagcguuc agcccacuuu cucgguacag agaaaccuuc ccuucgaaag agcgaccauu 1320

auggcagcau uuacaggraa uacugagggc agaacgucug acaugaggac ugaaaucaua 1380

agaaugaugg aaagugccag accagaagau gugucauucc aggggcgggg agucuucgag 1440

cucucggacg aaaaggcaac gaacccgauc gugccuuccu uugacaugar uaaugaagga 1500

ucuuauuucu ucggagacaa ugcagaggag uaugacaauu aaagaaaaau acccuuguuu 1560

cuacu 1565

<210> 5
<211> 1122
<212> RNA
<213> Artificial

<220>
<223> Consensus sequence of the Influenza A M1 gene

<400> 5
   ugcaggaauu cgauagcaga aagcagguag auguugaaag augagucuuc uaaccgaggu 60
   cgaaacguac guucucucua ucaucccguc aggcccccuc aaagccgaga ucgcgcagaa 120
   acuugaagau gucuuugcag gaaagaacac cgaucucgag gcucucaugg aguggcuaaa 180
   gacaagacca auccugucac cucugacuaa agggauuuug ggauuuguau ucacgcucac 240
   cgugcccagu gagcgaggac ugcagcguag acgcuuuguc cagaaugccc uaaauggaaa 300
   uggagaucca aauaauaugg auagggcagu uaagcuauau aagaagcuga aaagagaaau 360
   aacauuccau ggggcuaagg aggucgcacu cagcuacuca acyggugcac uugccaguug 420
   caugggucuc auauacaaca ggaugggaac ggugacuacg gaaguggcuu uuggccuagu 480
   gugugccacu ugugagcaga uugcagauuc acagcaucgg ucucacagac agauggcaac 540
   uaucaccaac ccacuaauca grcaugagaa cagaauggug cuggccagca cuacagcuaa 600
   ggcuauggag cagauggcgg gaucaaguga gcaggcagcg gaagccaugg agrucgcuaa 660
   ucaggcuagg cagauggugc aggcaaugag gacaauuggg acucauccua acucuagugc 720
   uggucugaga gauaaucuuc uugaaaauuu gcaggccuac cagaaacgaa ugggagugca 780
   gaugcagcga uucaagugau ccumuuguug uugccgcaar uaucauuggg aucuugcacu 840
   ugauauugug gauucuugau cgucuuuucu ucaaaugcau uuaucgucgc cuuaaauacg 900
   guuugaaaag agggccukcu acggcaggrg uaccugaguc uaugagggaa gaguaccggc 960
   aggaacagca gagugcugug gauguugacg auggucauuu ugucaacaua gaauuggagu 1020
   aaaaaacuac cuuguuucua curmkrccgs wwkacuugua cagcucgucc augccgagag     1080
   ugaucccggc ggcggucacg aacuccagca ggacccgaug uu                        1122


<210>  6
<211>  19
<212>  RNA
<213>  Artificial

<220>
<223>  strand of dsRNA

<400>  6
caggauacac cauggauac                                                    19


<210>  7
<211>  19
<212>  RNA
<213>  Artificial

<220>
<223>  strand of dsRNA

<400>  7
gaucuguucc accauugaa                                                    19


<210>  8
<211>  19
<212>  RNA
<213>  Artificial

<220>
<223>  strand of dsRNA

<400>  8
cgggacucua gcauacuua                                                    19


<210>  9
<211>  19
<212>  RNA
<213>  Artificial

<220>
<223>  strand of dsRNA

<400>  9
gcaauugagg agugccuga                                                    19


<210>  10
<211>  21
<212>  RNA
<213>  Artificial

<220>
<223>  strand of dsRNA

<400>  10
ccaggaaaug cugagaucga a                                                 21

<210>  11
<211>  21
<212>  RNA
<213>  Artificial

<220>
<223>  strand of dsRNA

<400>  11
gaguaaugaa ggaucuuauu u                                                21


<210>  12
<211>  21
<212>  RNA
<213>  Artificial

<220>
<223>  strand of dsRNA

<400>  12
aucuuauuuc uucggagaca a                                                21


<210>  13
<211>  19
<212>  RNA
<213>  Artificial

<220>
<223>  strand of dsRNA

<400>  13
ggaucuuauu ucuucggag                                                   19


<210>  14
<211>  19
<212>  RNA
<213>  Artificial

<220>
<223>  strand of dsRNA

<400>  14
cacuaaucag acaugagaa                                                   19


<210>  15
<211>  19
<212>  RNA
<213>  Artificial

<220>
<223>  strand of dsRNA

<400>  15
cuacagcuaa ggcuaugga                                                   19


<210>  16
<211>  4749
<212>  DNA
<213>  Artificial

<220>
<223>  Nucleic acid construct

<400>  16
agcttgcatg catttaaatg acagcagcag gtgaaagaca gacataacac caggcacagc      60

actcctgtgt acctcaaaag gcactagttg cctacatgcc acttcaaggg aagtgcttgt     120

cccactctgc tctgcactgg tgcagcctca cctcgagcac tgtgtgcagt tctgggagcc     180

acagtatatg aaggacataa aactagtata gagcacccaa aggaaggcta tgaagatggg     240

gaaggtgagt gaggaatggc tgaggtccct tggtctgcac agcccagagc agaggaggtc     300

tgaggagacg actcatggca gcctatggct cctcaagagg ggagtagagg ggaagtgctg     360

agatgtgctc cctgatgaca gcaacaggac ttgaacagac agtgggggtt aggaaaaggt     420

tcttcaccag agggcggtgg gcatggaaca ggctccctag ggcagtggac atgaccctga     480

gttactgtag ttcaaggaac agagggttgg acatcatgat ttcttgatgc cccttccagc     540

ccctgaaatc ctgtgtgtga ttctgtgaga gtgtttgggc aatactctca ggcaaagggt     600

ttgaattttg ggtggtgctg tgtggagtca ggagttggac tcagtgatcc ttgttggttc     660

cttccaactc acaatattct gtgattctat gatacatgag ccagtacatc aagatccaaa     720

agtccttttg attcccaatc ctcatgtccc actaaaatca tcctactatg aatccttcat     780

attattacag aaatgctcaa gctcaaacta accaaaaaat tgcaagaact ggatctgtat     840

gctttgaatc agagctaggt gacttaaaag tagattccaa ataatcttaa agacatggtg     900

tcatttgcaa actgcatcag aaaaggagaa agaacccccc agcctccacc tcccaaatcc     960

acagaccttt acagctttcc tgcagtgcct gaatctgaga taccactcag ggaacagtta    1020

tgttagtgta agatctgtag agatacctat ccaattactt tcaatgtgag tacactaata    1080

ttgctgtctg gcatattgct acatgtttca gcatcaacta caatttctaa tcttttcctc    1140

tactgttcct gtcccttgta atacaacagt aatacagggt tggcataaaa gccctttggt    1200

gagagctggt gacaatgtgc tctgcaggtc cctatgtccc agtaggacac ctgagtctac    1260

tgaggagctg cctgtggaag tctcccagga catgctgagc accacagaac agttgcactg    1320

ctgaaccccc agagctgata agctcagcgc cctgcaagac atcagatctg ctcgaggtcc    1380

tcagcacatg ctgccattga gctgcacaac ctggggcact tccaagaagg aggtgactgc    1440

tgtgtgaaac caaggactgc agacagaaac ctgggggaaa aagggcaccg agggcttcct    1500

gagcacccac ttgaggatcc ttaattaact ggaaactagg acgtggaagt catatacaat    1560

tcaagatgca agttaatttt atctgcagag aaattgtgtt tcaagaagga agaatacaca    1620

gggtaatgtc ataacctctt cacctaacca catggtaggt aaccatgagc taccactgag    1680

ctcacatcct tgcccccaaa cagcaaacat tcgacgtagc tgcagtaagc agcacgtgga    1740

tggtctcgga attcgattgt cgaccagaca gacgtcaggc tttctaagcc tggactgagt    1800

aagagcggaa gagctccaca gcactctgag tgcgcacaga ccgcgcgtac agcgcacagc 1860

cgcgcggccg ctccttcagg cactgccgac gacagcccag gcggaggtcc tgagcgccgg 1920

cgctaaattt gcataaagaa ctacccagga gccctcgcgc gcggaaacgg gcaaaaaggg 1980

gcttctaata tggaaatatt acgccgaatc gcgttacaaa tcggctaagc gggcctaaga 2040

gttaacaaga tgtgctatta agcggagcct tttggtggga agaaatggag tagtcactgt 2100

gttctaaaag aacttgcaga atgagccttt aaataccgca gtctcgatgc tcttagtcca 2160

ctacagctaa ggctatggag caaatttcaa gagaatttgc tccatagcct tagctgtagt 2220

gttttttgga actcgaccga agaaccgagc gctgctggcc ttaaagtccc accaaaactc 2280

tgaagaaacg aagccagacc cggcactcag cgggcagccc gcgcctcccg ccgccccaca 2340

gtgccgcgcg cgtgcatttg catagcgcgg tgctcgcagg gggaaactca ccccctcaag 2400

tccgcccccc gcttcccgcc cgctgtcccg cacctcatca gtgctgtgcg ctgtctgtgt 2460

cccccagcac gcactctttg ctgttcttac ccggaggctt gccctatcct tgaggtttct 2520

attttttagg ctataaatac cgcctaggag gtagagatat tcgcaattga ggagtgcctg 2580

attcaagaga tcaggcactc ctcaattgct tttttggaac tcgacgaatt gtgggacggc 2640

ggaagacggg ctcccgcccc gcccctatat gcaaagcaga gaacttcccg ccgtgcaccg 2700

cgcgcaatcg gaagagaatt tgggcacttc agacccaaaa aaaaacccaa aacttctgcg 2760

aaaaagaaag aatctcagcg gagtaaatag ggatttttgt taaagaggtg ataccagaag 2820

aagaaatatg caaatacaac gccagctcac cgctacttaa aaatcatgat ataatagagg 2880

cttaaatact gtccgagaga cactggggtt tggatcttat ttcttcggag ttcaagagac 2940

tccgaagaaa taagatcctt ttttggaact cgagaatcac tagtgaattc cataccactg 3000

cgagggtgcc aagtcatggg actgatactc tttaacgtct ttatcaatga cctagaaagt 3060

gttattgaat gaacctgtag tgagttgctg atgactgaac tgagtgctac agttaatatg 3120

agagaaggga gagaaatatg agagaaatgt tgagggaact agacttgttc agaagaaaag 3180

gttccaggca gatcttactg caaccttcca gttaattaag atatcttgtc ccttccagga 3240

acaggaagat tattaaaaaa aaaaaagtca ccataaaaaa aaaaaaaata ctatgctgaa 3300

gtaaggaaat tcagtaaaca agaaaaagag atattaataa tatggattac tttaagtttt 3360

gaattatttt cctttcaata gagtaactgt tagaaacatt ctgaatagtg cctctatact 3420

cagtgcccta tgttcacgtt cactaattca aaacagaaaa aaaaaaaaag attcctgtca 3480

tggttttttt ttgtctgttt gtttgtttgt ttgtttttcc tacttaagat cttgccctcc 3540

catttacact ggaatttaat tcagtacctc cggtaattct ggtattctac attcaatatc 3600

tttagcaaat ctttctgctg tagccagatt actccaacat aagatttctg gtttctggga 3660

aggatgccca tccctgagat atgcaaggtc aggctggatg gggtgctggg cacctgatgg 3720

agctgtagat gtccctgttc attgcagggg atttgaactg aatggccttt aagggtcctt 3780

ttcaactcaa acagttgaat gattctgtgg tttccaagga tagggagggt cctaacattc 3840

aaagctagac tgggaaagaa gaaaaagaca aaaattcttg gttatttcat tgctttggta 3900

gttccatcag cagataaaat tataggtctc ttcttggtct agtgtaagaa atgcctggga 3960

atcattttat cacaaaaatc tttctgtttg catgttttct tctgtactta ccaatcacac 4020

taaatgagtt ggattaaaat tttgggaggt tgagcagaaa atgttcttaa gtattaatat 4080

caattcttca catttaaaat gtgcaaatat tttgaggggt gttagtaggc tttgctcctc 4140

tgaaatttac ctttatggtt taatcaggtg atgcctccag ttgtgcaagt agccactaac 4200

aataatgttt tttcctacta atgggaaggt ggcaaacatt atattcagtg gattactaat 4260

aattttaagg aggaagtctt ttcaatctaa ttaacaagaa atgtgagtaa caaatgacgg 4320

atatttgtca taaaacaaca gtcatcttct cttacatgtg taaatataac attgtatttt 4380

acataagcca gtcagaaatc ttcacaagtc ttggatagtt ccagattgta actcctgatg 4440

cttagctcta tcaggatgat atgactcaga aacgttagtt ctatcaacct agtttccaac 4500

tcttccttgc ttttaatgat ttgtggagct ctactctgaa ccagcctggc tttgaagtaa 4560

atctccttca ctcatcatca gcacactttt tttgaaaagc cacgtcactt atagaatcct 4620

acttatgttg tggctcgatg aacatgtgtt aacactgtgg atcaggggag cgggagtgag 4680

cagtacagga gggaaaatat gattggaaac tccttgtgct gcaatctatt taaatctcga 4740

gctcgcgaa 4749

<210> 17
<211> 4700
<212> DNA
<213> Artificial

<220>
<223> Nucleic acid construct

<400> 17
   agcttgcatg catttaaatg acagcagcag gtgaaagaca gacataacac caggcacagc 60
   actcctgtgt acctcaaaag gcactagttg cctacatgcc acttcaaggg aagtgcttgt 120
   cccactctgc tctgcactgg tgcagcctca cctcgagcac tgtgtgcagt tctgggagcc 180
   acagtatatg aaggacataa aactagtata gagcacccaa aggaaggcta tgaagatggg 240
   gaaggtgagt gaggaatggc tgaggtccct tggtctgcac agcccagagc agaggaggtc 300
   tgaggagacg actcatggca gcctatggct cctcaagagg ggagtagagg ggaagtgctg 360
   agatgtgctc cctgatgaca gcaacaggac ttgaacagac agtgggggtt aggaaaaggt 420
   tcttcaccag agggcggtgg gcatggaaca ggctccctag ggcagtggac atgaccctga 480
   gttactgtag ttcaaggaac agagggttgg acatcatgat ttcttgatgc cccttccagc 540
   ccctgaaatc ctgtgtgtga ttctgtgaga gtgtttgggc aatactctca ggcaaagggt 600
   ttgaattttg ggtggtgctg tgtggagtca ggagttggac tcagtgatcc ttgttggttc 660
   cttccaactc acaatattct gtgattctat gatacatgag ccagtacatc aagatccaaa 720
   agtccttttg attcccaatc ctcatgtccc actaaaatca tcctactatg aatccttcat 780
   attattacag aaatgctcaa gctcaaacta accaaaaaat tgcaagaact ggatctgtat 840
   gctttgaatc agagctaggt gacttaaaag tagattccaa ataatcttaa agacatggtg 900
   tcatttgcaa actgcatcag aaaaggagaa agaacccccc agcctccacc tcccaaatcc 960
   acagaccttt acagctttcc tgcagtgcct gaatctgaga taccactcag ggaacagtta 1020
   tgttagtgta agatctgtag agatacctat ccaattactt tcaatgtgag tacactaata 1080
   ttgctgtctg gcatattgct acatgtttca gcatcaacta caatttctaa tcttttcctc 1140
   tactgttcct gtcccttgta atacaacagt aatacagggt tggcataaaa gccctttggt 1200
   gagagctggt gacaatgtgc tctgcaggtc cctatgtccc agtaggacac ctgagtctac 1260
   tgaggagctg cctgtggaag tctcccagga catgctgagc accacagaac agttgcactg 1320
   ctgaaccccc agagctgata agctcagcgc cctgcaagac atcagatctg ctcgaggtcc 1380
   tcagcacatg ctgccattga gctgcacaac ctggggcact tccaagaagg aggtgactgc 1440
   tgtgtgaaac caaggactgc agacagaaac ctgggggaaa aagggcaccg agggcttcct 1500
   gagcacccac ttgaggatcc ttaattaact ggaaactagg acgtggaagt catatacaat 1560
   tcaagatgca agttaatttt atctgcagag aaattgtgtt tcaagaagga agaatacaca 1620
   gggtaatgtc ataacctctt cacctaacca catggtaggt aaccatgagc taccactgag 1680
   ctcacatcct tgcccccaaa cagcaaacat tcgacgtagc tgcagtaagc agcacgtgga 1740
   tggtctcgga attgcggccg cgggaattcg attgtcgacg aattgtggga cggcggaaga 1800
   cgggctcccg ccccgcccct atatgcaaag cagagaactt cccgccgtgc accgcgcgca 1860
   atcggaagag aatttgggca cttcagaccc aaaaaaaaac ccaaaacttc tgcgaaaaag 1920
   aaagaatctc agcggagtaa atagggattt ttgttaaaga ggtgatacca gaagaagaaa 1980
   tatgcaaata caacgccagc tcaccgctac ttaaaaatca tgatataata gaggcttaaa 2040
   tactgtccga gagacactgg ggtttgatct gttccaccat tgaattcaag agattcaatg 2100
   gtggaacaga tcttttttgg aactcgaccg aagaaccgag cgctgctggc cttaaagtcc 2160
   caccaaaact ctgaagaaac gaagccagac ccggcactca gcgggcagcc cgcgcctccc 2220
   gccgccccac agtgccgcgc gcgtgcattt gcatagcgcg gtgctcgcag gggggaactc 2280
   accccctcaa gtccgccccc cgcttcccgc ccgctgtccc gcacctcatc agtgctgtgc 2340
   gctgtctgtg tcccccagca cgcactcttt gctgttctta cccggaggct tgccctatcc 2400
   ttgaggtttc tattttttag gctataaata ccgcctagga ggtagagata ttccgggact 2460
   ctagcatact tattcaagag ataagtatgc tagagtcccg ttttttggaa ctcgacgagg 2520
   ctcagtgtca cgcagagcgc gggacgagcg ctccgagccc tcccagtgcc gcccccaagg 2580
   cagggcggcc ggcgcagctc cccgcagccc gccagtggga aggctctgct ttgcataacg 2640
   cgcaaggcct gctgggagga aagcggagcg agaaagagcg ttaacgtgcg ccgagtgttt 2700
   tagagcaaaa gcattcagac ctgaagcagc gctgagagat gcctctgccg cccatttact 2760
   ggaacgttca gacccaccgc aagtcaccgt gaccttgagg acactgagct gttggccgtt 2820
   atatagcact tggggcagct cgtagctttc aggatacacc atggatactt caagagagta 2880
   tccatggtgt atcctgtttt tggaactcga gaatcactag tgaattcgcg gccgcgaatt 2940
   ccataccact gcgagggtgc caagtcatgg gactgatact ctttaacgtc tttatcaatg 3000
   acctagaaag tgttattgaa tgaacctgta gtgagttgct gatgactgaa ctgagtgcta 3060
   cagttaatat gagagaaggg agagaaatat gagagaaatg ttgagggaac tagacttgtt 3120
   cagaagaaaa ggttccaggc agatcttact gcaaccttcc agttaattaa gatatcttgt 3180
   cccttccagg aacaggaaga ttattaaaaa aaaaaaagtc accataaaaa aaaaaaaaat 3240
   actatgctga agtaaggaaa ttcagtaaac aagaaaaaga gatattaata atatggatta 3300
   ctttaagttt tgaattattt tcctttcaat agagtaactg ttagaaacat tctgaatagt 3360
   gcctctatac tcagtgccct atgttcacgt tcactaattc aaaacagaaa aaaaaaaaaa 3420
   gattcctgtc atggtttttt tttgtctgtt tgtttgtttg tttgtttttc ctacttaaga 3480
   tcttgccctc ccatttacac tggaatttaa ttcagtacct ccggtaattc tggtattcta 3540
   cattcaatat ctttagcaaa tctttctgct gtagccagat tactccaaca taagatttct 3600
   ggtttctggg aaggatgccc atccctgaga tatgcaaggt caggctggat ggggtgctgg 3660
   gcacctgatg gagctgtaga tgtccctgtt cattgcaggg gatttgaact gaatggcctt 3720
   taagggtcct tttcaactca aacagttgaa tgattctgtg gtttccaagg atagggaggg 3780
   tcctaacatt caaagctaga ctgggaaaga agaaaaagac aaaaattctt ggttatttca 3840
   ttgctttggt agttccatca gcagataaaa ttataggtct cttcttggtc tagtgtaaga 3900
   aatgcctggg aatcatttta tcacaaaaat ctttctgttt gcatgttttc ttctgtactt 3960
   accaatcaca ctaaatgagt tggattaaaa ttttgggagg ttgagcagaa aatgttctta 4020
   agtattaata tcaattcttc acatttaaaa tgtgcaaata ttttgagggg tgttagtagg 4080
   ctttgctcct ctgaaattta cctttatggt ttaatcaggt gatgcctcca gttgtgcaag 4140
   tagccactaa caataatgtt ttttcctact aatgggaagg tggcaaacat tatattcagt 4200
   ggattactaa taattttaag gaggaagtct tttcaatcta attaacaaga aatgtgagta 4260
   acaaatgacg gatatttgtc ataaaacaac agtcatcttc tcttacatgt gtaaatataa 4320
   cattgtattt tacataagcc agtcagaaat cttcacaagt cttggatagt tccagattgt 4380
   aactcctgat gcttagctct atcaggatga tatgactcag aaacgttagt tctatcaacc 4440
   tagtttccaa ctcttccttg cttttaatga tttgtggagc tctactctga accagcctgg 4500
   ctttgaagta aatctccttc actcatcatc agcacacttt ttttgaaaag ccacgtcact 4560
   tatagaatcc tacttatgtt gtggctcgat gaacatgtgt taacactgtg gatcagggga 4620
   gcgggagtga gcagtacagg agggaaaata tgattggaaa ctccttgtgc tgcaatctat 4680
   ttaaatctcg agctcgcgaa 4700

<210> 18
<211> 4706
<212> DNA
<213> Artificial

<220>
<223> Nucleic acid construct

<400> 18
agcttgcatg catttaaatg acagcagcag gtgaaagaca gacataacac caggcacagc 60

actcctgtgt acctcaaaag gcactagttg cctacatgcc acttcaaggg aagtgcttgt 120

cccactctgc tctgcactgg tgcagcctca cctcgagcac tgtgtgcagt tctgggagcc 180

acagtatatg aaggacataa aactagtata gagcacccaa aggaaggcta tgaagatggg 240

gaaggtgagt gaggaatggc tgaggtccct tggtctgcac agcccagagc agaggaggtc 300

tgaggagacg actcatggca gcctatggct cctcaagagg ggagtagagg ggaagtgctg 360

agatgtgctc cctgatgaca gcaacaggac ttgaacagac agtgggggtt aggaaaaggt 420

tcttcaccag agggcggtgg gcatggaaca ggctccctag ggcagtggac atgaccctga 480

gttactgtag ttcaaggaac agagggttgg acatcatgat ttcttgatgc cccttccagc 540

ccctgaaatc ctgtgtgtga ttctgtgaga gtgtttgggc aatactctca ggcaaagggt 600

ttgaattttg ggtggtgctg tgtggagtca ggagttggac tcagtgatcc ttgttggttc 660

cttccaactc acaatattct gtgattctat gatacatgag ccagtacatc aagatccaaa 720

agtccttttg attcccaatc ctcatgtccc actaaaatca tcctactatg aatccttcat 780

attattacag aaatgctcaa gctcaaacta accaaaaaat tgcaagaact ggatctgtat 840

gctttgaatc agagctaggt gacttaaaag tagattccaa ataatcttaa agacatggtg 900

tcatttgcaa actgcatcag aaaaggagaa agaacccccc agcctccacc tcccaaatcc 960

acagaccttt acagctttcc tgcagtgcct gaatctgaga taccactcag ggaacagtta 1020

tgttagtgta agatctgtag agatacctat ccaattactt tcaatgtgag tacactaata 1080

ttgctgtctg gcatattgct acatgtttca gcatcaacta caatttctaa tcttttcctc 1140

tactgttcct gtcccttgta atacaacagt aatacagggt tggcataaaa gccctttggt 1200

gagagctggt gacaatgtgc tctgcaggtc cctatgtccc agtaggacac ctgagtctac 1260

tgaggagctg cctgtggaag tctcccagga catgctgagc accacagaac agttgcactg 1320

ctgaaccccc agagctgata agctcagcgc cctgcaagac atcagatctg ctcgaggtcc 1380

tcagcacatg ctgccattga gctgcacaac ctggggcact tccaagaagg aggtgactgc 1440

tgtgtgaaac caaggactgc agacagaaac ctgggggaaa aagggcaccg agggcttcct 1500
gagcacccac ttgaggatcc ttaattaact ggaaactagg acgtggaagt catatacaat 1560
tcaagatgca agttaatttt atctgcagag aaattgtgtt tcaagaagga agaatacaca 1620
gggtaatgtc ataacctctt cacctaacca catggtaggt aaccatgagc taccactgag 1680
ctcacatcct tgcccccaaa cagcaaacat tcgacgtagc tgcagtaagc agcacgtgga 1740
tggtctcgga attgcggccg cgggaattcg attgtcgacg aattgtggga cggcggaaga 1800
cgggctcccg ccccgcccct atatgcaagg cagagaactt cccgccgtgc accgcgcgca 1860
atcggaagag aatttgggca cttcagaccc aaaaaaaaac ccaaaacttc tgcgaaaaag 1920
aaagaatctc agcggagtaa atagggattt ttgttaaaga ggtgatacca gaagaagaaa 1980
tatgcaaata caacgccagc tcaccgctac ttaaaaatca tgatataata gaggcttaaa 2040
tactgtccga gagacactgg ggtttatctt atttcttcgg agacaattca agagattgtc 2100
tccgaagaaa taagattttt ttggaactcg accgaagaac cgagcgctgc tggccttaaa 2160
gtcccaccaa aactctgaag aaacgaagcc agacccggca ctcagcgggc agcccgcgcc 2220
tcccgccgcc ccacagtgcc gcgcgcgtgc atttgcatag cgcggtgctc gcagggggaa 2280
actcaccccc tcaagtccgc cccccgcttc ccgcccgctg tcccgcacct catcagtgct 2340
gtgcgctgtc tgtgtccccc agcacgcact ctttgctgtt cttacccgga ggcttgccct 2400
atccttgagg tttctatttt ttaggctata aataccgcct aggaggtaga gatattcgca 2460
attgaggagt gcctgattca agagatcagg cactcctcaa ttgctttttt ggaagtcgac 2520
gaggctcagt gtcacgcaga gcgcgggacg agcgctccga gccctcccag tgccgccccc 2580
caaggcaggg cggccggcgc agctccccgc agcccgccag tgggaaggct ctgctttgca 2640
taacgcgcaa ggcctgctgg gaggaaagcg gagcgagaaa gagcgttaac gtgcgccgag 2700
tgttttagag caaaagcatt cagacctgaa gcagcgctga gagatgcctc tgccgcccat 2760
ttactggaac gttcagaccc accgcaagtc accgtgacct tgaggacact gagctgttgg 2820
ccgttatata gcacttgggg cagctcgtag ctttgatctg ttccaccatt gaattcaaga 2880
gattcaatgg tggaacagat cttttttgga actcgagaat cactagtgaa ttcgcggccg 2940
cgaattccat accactgcga gggtgccaag tcatgggact gatactcttt aacgtcttta 3000
tcaatgacct agaaagtgtt attgaatgaa cctgtagtga gttgctgatg actgaactga 3060
gtgctacagt taatatgaga gaagggagag aaatatgaga gaaatgttga gggaactaga 3120
cttgttcaga agaaaaggtt ccaggcagat cttactgcaa ccttccagtt aattaagata 3180
tcttgtccct tccaggaaca ggaagattat taaaaaaaaa aaagtcacca taaaaaaaaa 3240
aaaaatacta tgctgaagta aggaaattca gtaaacaaga aaaagagata ttaataatat 3300
ggattacttt aagttttgaa ttattttcct ttcaatagag taactgttag aaacattctg 3360

aatagtgcct ctatactcag tgccctatgt tcacgttcac taattcaaaa cagaaaaaaa 3420

aaaaaagatt cctgtcatgg tttttttttg tctgtttgtt tgtttgtttg tttttcctac 3480

ttaagatctt gccctcccat ttacactgga atttaattca gtacctccgg taattctggt 3540

attctacatt caatatcttt agcaaatctt tctgctgtag ccagattact ccaacataag 3600

atttctggtt tctgggaagg atgcccatcc ctgagatatg caaggtcagg ctggatgggg 3660

tgctgggcac ctgatggagc tgtagatgtc cctgttcatt gcaggggatt tgaactgaat 3720

ggcctttaag ggtccttttc aactcaaaca gttgaatgat tctgtggttt ccaaggatag 3780

ggagggtcct aacattcaaa gctagactgg gaaagaagaa aaagacaaaa attcttggtt 3840

atttcattgc tttggtagtt ccatcagcag ataaaattat aggtctcttc ttggtctagt 3900

gtaagaaatg cctgggaatc attttatcac aaaaatcttt ctgtttgcat gttttcttct 3960

gtacttacca atcacactaa atgagttgga ttaaaatttt gggaggttga gcagaaaatg 4020

ttcttaagta ttaatatcaa ttcttcacat ttaaaatgtg caaatatttt gaggggtgtt 4080

agtaggcttt gctcctctga aatttacctt tatggtttaa tcaggtgatg cctccagttg 4140

tgcaagtagc cactaacaat aatgtttttt cctactaatg ggaaggtggc aaacattata 4200

ttcagtggat tactaataat tttaaggagg aagtcttttc aatctaatta acaagaaatg 4260

tgagtaacaa atgacggata tttgtcataa aacaacagtc atcttctctt acatgtgtaa 4320

atataacatt gtattttaca taagccagtc agaaatcttc acaagtcttg gatagttcca 4380

gattgtaact cctgatgctt agctctatca ggatgatatg actcagaaac gttagttcta 4440

tcaacctagt ttccaactct tccttgcttt taatgatttg tggagctcta ctctgaacca 4500

gcctggcttt gaagtaaatc tccttcactc atcatcagca cacttttttt gaaaagccac 4560

gtcacttata gaatcctact tatgttgtgg ctcgatgaac atgtgttaac actgtggatc 4620

aggggagcgg gagtgagcag tacaggaggg aaaatatgat tggaaactcc ttgtgctgca 4680

atctatttaa atctcgagct cgcgaa 4706

<210> 19
<211> 1242
<212> DNA
<213> Artificial

<220>
<223> Multiple shRNA construct 1

<400> 19
gaattcgatt gtcgaccaga cagacgtcag gctttctaag cctggactga gtaagagcgg 60

aagagctcca cagcactctg agtgcgcaca gaccgcgcgt acagcgcaca gccgcgcggc 120

cgctccttca ggcactgccg acgacagccc aggcggaggt cctgagcgcc ggcgctaaat 180

ttgcataaag aactacccag gagccctcgc gcgcggaaac gggcaaaaag gggcttctaa 240

tatggaaata ttacgccgaa tcgcgttaca aatcggctaa gcgggcctaa gagttaacaa 300

gatgtgctat taagcggagc cttttggtgg gaagaaatgg agtagtcact gtgttctaaa 360

agaacttgca gaatgagcct ttaaataccg cagtctcgat gctcttagtc cactacagct 420

aaggctatgg agcaaatttc aagagaattt gctccatagc cttagctgta gtgttttttg 480

gaactcgacc gaagaaccga gcgctgctgg ccttaaagtc ccaccaaaac tctgaagaaa 540

cgaagccaga cccggcactc agcgggcagc ccgcgcctcc cgccgcccca cagtgccgcg 600

cgcgtgcatt tgcatagcgc ggtgctcgca gggggaaact caccccctca agtccgcccc 660

ccgcttcccg cccgctgtcc cgcacctcat cagtgctgtg cgctgtctgt gtcccccagc 720

acgcactctt tgctgttctt acccggaggc ttgccctatc cttgaggttt ctatttttta 780

ggctataaat accgcctagg aggtagagat attcgcaatt gaggagtgcc tgattcaaga 840

gatcaggcac tcctcaattg cttttttgga actcgacgaa ttgtgggacg gcggaagacg 900

ggctcccgcc ccgcccctat atgcaaagca gagaacttcc cgccgtgcac cgcgcgcaat 960

cggaagagaa tttgggcact tcagacccaa aaaaaaaccc aaaacttctg cgaaaaagaa 1020

agaatctcag cggagtaaat agggattttt gttaaagagg tgataccaga agaagaaata 1080

tgcaaataca acgccagctc accgctactt aaaaatcatg atataataga ggcttaaata 1140

ctgtccgaga gacactgggg tttggatctt atttcttcgg agttcaagag actccgaaga 1200

aataagatcc ttttttggaa ctcgagaatc actagtgaat tc 1242

<210> 20
<211> 1181
<212> DNA
<213> Artificial

<220>
<223> Multiple shRNA construct 2

<400> 20
gcggccgcgg gaattcgatt gtcgacgaat tgtgggacgg cggaagacgg gctcccgccc 60

cgcccctata tgcaaagcag agaacttccc gccgtgcacc gcgcgcaatc ggaagagaat 120

ttgggcactt cagacccaaa aaaaaaccca aaacttctgc gaaaaagaaa gaatctcagc 180

ggagtaaata gggatttttg ttaaagaggt gataccagaa gaagaaatat gcaaatacaa 240

cgccagctca ccgctactta aaaatcatga tataatagag gcttaaatac tgtccgagag 300

acactggggt ttgatctgtt ccaccattga attcaagaga ttcaatggtg gaacagatct 360

tttttggaac tcgaccgaag aaccgagcgc tgctggcctt aaagtcccac caaaactctg 420

aagaaacgaa gccagacccg gcactcagcg ggcagcccgc gcctcccgcc gccccacagt 480

gccgcgcgcg tgcatttgca tagcgcggtg ctcgcagggg gaaactcacc ccctcaagtc 540

cgccccccgc ttcccgcccg ctgtcccgca cctcatcagt gctgtgcgct gtctgtgtcc 600

cccagcacgc actctttgct gttcttaccc ggaggcttgc cctatccttg aggtttctat 660

tttttaggct ataaataccg cctaggaggt agagatattc cgggactcta gcatacttat 720

tcaagagata agtatgctag agtcccgttt tttggaactc gacgaggctc agtgtcacgc 780

agagcgcggg acgagcgctc cgagccctcc cagtgccgcc cccaaggcag ggcggccggc 840

gcagctcccc gcagcccgcc agtgggaagg ctctgctttg cataacgcgc aaggcctgct 900

gggaggaaag cggagcgaga aagagcgtta acgtgcgccg agtgttttag agcaaaagca 960

ttcagacctg aagcagcgct gagagatgcc tctgccgccc atttactgga acgttcagac 1020

ccaccgcaag tcaccgtgac cttgaggaca ctgagctgtt ggccgttata tagcacttgg 1080

ggcagctcgt agctttcagg atacaccatg gatacttcaa gagagtatcc atggtgtatc 1140

ctgtttttgg aactcgagaa tcactagtga attcgcggcc g 1181

<210> 21
<211> 1196
<212> DNA
<213> Artificial

<220>
<223> Multiple shRNA construct 3

<400> 21
gcggccgcgg gaattcgatt gtcgacgaat tgtgggacgg cggaagacgg gctcccgccc 60

cgcccctata tgcaaggcag agaacttccc gccgtgcacc gcgcgcaatc ggaagagaat 120

ttgggcactt cagacccaaa aaaaaaccca aaacttctgc gaaaaagaaa gaatctcagc 180

ggagtaaata gggatttttg ttaaagaggt gataccagaa gaagaaatat gcaaatacaa 240

cgccagctca ccgctactta aaaatcatga tataatagag gcttaaatac tgtccgagag 300

acactggggt ttatcttatt tcttcggaga caattcaaga gattgtctcc gaagaaataa 360

gatttttttg gaactcgacc gaagaaccga gcgctgctgg ccttaaagtc ccaccaaaac 420

tctgaagaaa cgaagccaga cccggcactc agcgggcagc ccgcgcctcc cgccgcccca 480

cagtgccgcg cgcgtgcatt tgcatagcgc ggtgctcgca gggggaaact caccccctca 540

agtccgcccc ccgcttcccg cccgctgtcc cgcacctcat cagtgctgtg cgctgtctgt 600

gtcccccagc acgcactctt tgctgttctt acccggaggc ttgccctatc cttgaggttt 660

ctatttttta ggctataaat accgcctagg aggtagagat attcgcaatt gaggagtgcc 720

tgattcaaga gatcaggcac tcctcaattg cttttttgga agtcgacgag gctcagtgtc 780

acgcagagcg cgggacgagc gctccgagcc ctcccagtgc cgccccccaa ggcagggcgg 840

ccggcgcagc tccccgcagc ccgccagtgg gaaggctctg ctttgcataa cgcgcaaggc 900

ctgctgggag gaaagcggag cgagaaagag cgttaacgtg cgccgagtgt tttagagcaa 960

aagcattcag acctgaagca gcgctgagag atgcctctgc cgcccattta ctggaacgtt 1020

cagacccacc gcaagtcacc gtgaccttga ggacactgag ctgttggccg ttatatagca 1080

cttggggcag ctcgtagctt tgatctgttc caccattgaa ttcaagagat tcaatggtgg 1140

aacagatctt ttttggaact cgagaatcac tagtgaattc gcggccgcga attcca 1196

<210> 22
<211> 325
<212> DNA
<213> Artificial

<220>
<223> cU6-1 promoter sequence

<400> 22
cgaagaaccg agcgctgctg gccttaaagt cccaccaaaa ctctgaagaa acgaagccag 60

acccggcact cagcgggcag cccgcgcctc ccgccgcccc acagtgccgc gcgcgtgcat 120

ttgcatagcg cggtgctcgc agggggaaac tcaccccctc aagtccgccc cccgcttccc 180

gcccgctgtc ccgcacctca tcagtgctgt gcgctgtctg tgtcccccag cacgcactct 240

ttgctgttct tacccggagg cttgccctat ccttgaggtt tctatttttt aggctataaa 300

taccgcctag gaggtagaga tattc 325

<210> 23
<211> 394
<212> DNA
<213> Artificial

<220>
<223> cU6-3 promoter sequence

<400> 23
cagacagacg tcaggctttc taagcctgga ctgagtaaga gcggaagagc tccacagcac 60

tctgagtgcg cacagaccgc gcgtacagcg cacagccgcg cggccgctcc ttcaggcact 120

gccgacgaca gcccaggcgg aggtcctgag cgccggcgct aaatttgcat aaagaactac 180

ccaggagccc tcgcgcgcgg aaacgggcaa aaagggcctt ctaatatgga aatattacgc 240

cgaatcgcgt tacaaatcgg ctaagcgggc ctaagagtta acaagatgtg ctattaagcg 300

gagccttttg gtgggaagaa atggagtagt cactgtgttc taaaagaact tgcagaatga 360

gcctttaaat accgcagtct cgatgctctt agtc 394

<210> 24
<211> 287
<212> DNA
<213> Artificial

<220>
<223> cU6-4 promoter sequence

<400> 24
tgaattgtgg gacggcggaa gacgggctcc cgccccgccc ctatatgcaa agcagagaac 60

ttcccgccgt gcaccgcgcg caatcggaag agaatttggg cacttcagac ccaaaaaaaa 120

acccaaaact tctgcgaaaa agaaagaatc tcagcggagt aaatagggat ttttgttaaa 180

gaggtgatac cagaagaaga aatatgcaaa tacaacgcca gctcaccgct acttaaaaat 240

catgatataa tagaggctta aatactgtcc gagagacact ggggttt 287

<210> 25
<211> 783
<212> DNA
<213> Artificial

<220>
<223> 7SK promoter sequence

<400> 25
gtccagccat ccacctccca ccaatacttc cccactgaac catgtccctc agtagcacag 60

ggtttgtgga acgcctcctg ggacggtgcc tcccccacct gccacgcagc ccattccagc 120

acctgacact tctggagacg aaatttttcc taacgtccaa cctgagtctc ccctggtgca 180

acttgaggct gttcccctga ctcccatcgc tagttacgtg ggaagaaaag acccctaaga 240

ccaccccgtg caaccaccag cccatcccca ccacgcccac tgaccgggcc cctcagtgcc 300

acagcagcac ggttctcgag cgcttcgcag gacggtgagc actgcccgga acctctgcac 360

ggcctcagca acgcgacttt cagccggggt cgctgcccag gagccggcgg cttcggagcg 420

cagagcgagg cgggagagct ccggccgcgg gaggctcagt gtcacgcaga gcgcgggacg 480

agcgctccga gccctcccag tgccgccccc aaggcagggc ggccggcgca gctccccgca 540

gcccgccagt gggaaggctc tgctttgcat aacgcgcaag gcctgctggg aggaaagcgg 600

agcgagaaag agcgttaacg tgcgccgagt gttttagagc aaaagcattc agacctgaag 660

cagcgctgag agatgcctct gccgcccatt tactggaaac gttcagaccc accgcaagtc 720

accgtgacct tgagacactg agctgttggc cgttatatag cacttggggc agctcgtagc 780

ttt 783

<210> 26
<211> 18
<212> DNA
<213> Artificial

<220>
<223> oligonucleotide primer

<400> 26
cgaagaaccg agcgctgc 18

<210> 27
<211> 85
<212> DNA
<213> Artificial

<220>
<223> oligonucleotide primer

<400> 27
gggctcgagt tccaaaaaag cgcagtgtta ctccacttct cttgaaagtg gagtaacact 60

gcgctgaata ccgcttcctc ctgag                                                85

<210>  28
<211>  20
<212>  DNA
<213>  Artificial

<220>
<223>  oligonucleotide primer

<400>  28
gaattgtggg acggcggaag                                                      20

<210>  29
<211>  20
<212>  DNA
<213>  Artificial

<220>
<223>  oligonucleotide primer

<400>  29
cagacagacg tcaggctttc                                                      20

<210>  30
<211>  83
<212>  DNA
<213>  Artificial

<220>
<223>  oligonucleotide primer

<400>  30
ctcgagttcc aaaaaaggat cttatttctt cggagtctct tgaactccga agaaataaga          60

tccaaacccc agtgtctctc gga                                                  83

<210>  31
<211>  99
<212>  DNA
<213>  Artificial

<220>
<223>  oligonucleotide primer

<400>  31
ctcgagttcc aaaaaacact acagctaagg ctatggagca aattctcttg aaatttgctc          60

catagcctta gctgtagtgg actaagagca tcgagactg                                 99

<210>  32
<211>  83
<212>  DNA
<213>  Artificial

<220>
<223>  oligonucleotide primer

<400>  32

ctcgagttcc aaaaaagcaa ttgaggagtg cctgatctct tgaatcaggc actcctcaat 60

tgcgaatatc tctacctcct agg 83

<210> 33
<211> 24
<212> DNA
<213> Artificial

<220>
<223> oligonucleotide primer

<400> 33
gtcgaccgaa gaaccgagcg ctgc 24

<210> 34
<211> 26
<212> DNA
<213> Artificial

<220>
<223> oligonucleotide primer

<400> 34
gtcgacgaat tgtgggacgg cggaag 26

<210> 35
<211> 26
<212> DNA
<213> Artificial

<220>
<223> oligonucleotide primer

<400> 35
gtcgaccaga cagacgtcag gctttc 26

<210> 36
<211> 20
<212> DNA
<213> Artificial

<220>
<223> oligonucleotide primer

<400> 36
gaggctcagt gtcacgcaga 20

<210> 37
<211> 83
<212> DNA
<213> Artificial

<220>
<223> oligonucleotide primer

<400> 37
ctcgagttcc aaaaaagatc tgttccacca ttgaatctct tgaattcaat ggtggaacag 60

atcaaacccc agtgtctctc gga 83

<210>  38
<211>  83
<212>  DNA
<213>  Artificial

<220>
<223>  oligonucleotide primer

<400>  38
ctcgagttcc aaaaaacggg actctagcat acttatctct tgaataagta tgctagagtc      60

ccggaatatc tctacctcct agg                                              83


<210>  39
<211>  87
<212>  DNA
<213>  Artificial

<220>
<223>  oligonucleotide primer

<400>  39
ctcgagttcc aaaaaaatct tatttcttcg gagacaatct cttgaattgt ctccgaagaa      60

ataagataaa ccccagtgtc tctcgga                                          87


<210>  40
<211>  25
<212>  DNA
<213>  Artificial

<220>
<223>  oligonucleotide primer

<400>  40
gtcgacgagg ctcagtgtca cgcag                                            25


<210>  41
<211>  83
<212>  DNA
<213>  Artificial

<220>
<223>  oligonucleotide primer

<400>  41
ctcgagttcc aaaaaacagg atacaccatg gatactctct tgaagtatcc atggtgtatc      60

ctgaaagcta cgagctgccc caa                                              83


<210>  42
<211>  83
<212>  DNA
<213>  Artificial

<220>
<223>  oligonucleotide primer

<400>  42

ctcgagttcc aaaaaagatc tgttccacca ttgaatctct tgaattcaat ggtggaacag 60

atcaaagcta cgagctgccc caa 83

<210> 43
<211> 46
<212> DNA
<213> Artificial

<220>
<223> oligonucleotide primer

<400> 43
gaattccata ccactgcgag ggtgccaagt catgggactg atactc 46

<210> 44
<211> 32
<212> DNA
<213> Artificial

<220>
<223> oligonucleotide primer

<400> 44
gatatcttaa ttaactggaa ggttgcagta ag 32

<210> 45
<211> 25
<212> DNA
<213> Artificial

<220>
<223> oligonucleotide primer

<400> 45
gatatcttgt cccttccagg aacag 25

<210> 46
<211> 32
<212> DNA
<213> Artificial

<220>
<223> oligonucleotide primer

<400> 46
ctcgagattt aaatagattg cagcacaagg ag 32

<210> 47
<211> 35
<212> DNA
<213> Artificial

<220>
<223> oligonucleotide primer

<400> 47
ggatccttaa ttaactggaa actaggacgt ggaag 35

<210> 48
<211> 47
<212> DNA
<213> Artificial

<220>
<223> oligonucleotide primer

<400> 48
gaattccgag accatccacg tgctgcttac tgcagctacg tcgaatg 47

<210> 49
<211> 34
<212> DNA
<213> Artificial

<220>
<223> oligonucleotide primer

<400> 49
gcatgcattt aaatgacagc agcaggtgaa agac 34

<210> 50
<211> 26
<212> DNA
<213> Artificial

<220>
<223> oligonucleotide primer

<400> 50
ggatcctcaa gtgggtgctc aggaag 26

<210> 51
<211> 87
<212> DNA
<213> Artificial

<220>
<223> Oligonucleotide primer

<400> 51
ctcgagttcc aaaaaaatct tatttcttcg gagacaatct cttgaattgt ctccgaagaa 60

ataagatgac taagagcatc gagactg 87

<210> 52
<211> 21
<212> RNA
<213> Artificial

<220>
<223> dsNA coding sequence

<400> 52
cagcgaccaa aagaauucgg a 21

<210> 53
<211> 21
<212> RNA

<213>  Artificial Sequence

<220>
<223>  dsNA coding sequence

<400>  53
aagaauucgg auggccauca a                                                21


<210>  54
<211>  19
<212>  RNA
<213>  Artificial Sequence

<220>
<223>  dsNA coding sequence

<400>  54
guggauucuu gaucgucuu                                                   19


<210>  55
<211>  17
<212>  DNA
<213>  Artificial Sequence

<220>
<223>  Oligonucleotide primer

<400>  55
ttgcccccaa acagcaa                                                     17


<210>  56
<211>  20
<212>  DNA
<213>  Artificial Sequence

<220>
<223>  Oligonucleotide primer

<400>  56
gaccatccac gtgctgctta                                                  20


<210>  57
<211>  17
<212>  DNA
<213>  Artificial Sequence

<220>
<223>  Oligonucoleotide primer

<400>  57
cattcgacgt agctgca                                                     17


<210>  58
<211>  19
<212>  DNA
<213>  Artificial Sequence

<220>
<223>  Oligonucleotide primer

<400>  58
gcagcacgtg gatggtctc                                                    19


<210>  59
<211>  20
<212>  DNA
<213>  Artificial Sequence

<220>
<223>  Oligonucleotide primer

<400>  59
tcttccgccg tcccacaatt                                                   20


<210>  60
<211>  20
<212>  DNA
<213>  Artificial Sequence

<220>
<223>  Oligonucleotide primer

<400>  60
gcttagaaag cctgacgtct                                                   20


<210>  61
<211>  1206
<212>  DNA
<213>  Artificial Sequence

<220>
<223>  Nucleic acid construct

<400>  61
gacgtcgacg aattgtggga cggcggaaga cgggctcccg ccccgcccct atatgcaaag      60

cagagaactt cccgccgtgc accgcgcgca atcggaagag aatttgggca cttcagaccc     120

aaaaaaaaac ccaaaacttc tgcgaaaaag aaagaatctc agcggagtaa atagggattt     180

ttgttaaaga ggtgatacca gaagaagaaa tatgcaaata caacgccagc tcaccgctac     240

ttaaaaatca tgatataata gaggcttaaa tactgtccga gagacactgg ggtttgatct     300

gttccaccat tgaattcaag agattcaatg gtggaacaga tcttttttgg aactcgacca     360

gacagacgtc aggctttcta agcctggact gagtaagagc ggaagagctc cacagcactc     420

tgagtgcgca cagaccgcgc gtacagcgca cagccgcgcg gccgctcctt caggcactgc     480

cgacgacagc ccaggcggag gtcctgagcg ccggcgctaa atttgcataa agaactaccc     540

aggagccctc gcgcgcggaa acgggcaaaa aggggcttct aatatggaaa tattacgccg     600

aatcgcgtta caaatcggct aagcgggcct aagagttaac aagatgtgct attaagcgga     660

gccttttggt gggaagaaat ggagtagtca ctgtgttcta aaagaacttg cagaatgagc     720

ctttaaatac cgcagtctcg atgctcttag tctcttattt cttcggagac aattcaagag     780

attgtctccg aagaaataag atttttttgg aactcgaccg aagaaccgag cgctgctggc     840

cttaaagtcc caccaaaact ctgaagaaac gaagccagac ccggcactca gcgggcagcc 900

cgcgcctccc gccgccccac agtgccgcgc gcgtgcattt gcatagcgcg gtgctcgcag 960

ggggaaactc accccctcaa gtccgccccc cgcttcccgc ccgctgtccc gcacctcatc 1020

agtgctgtgc gctgtctgtg tcccccagca cgcactcttt gctgttctta cccggaggct 1080

tgccctatcc ttgaggtttc tattttttag gctataaata ccgcctagga ggtagagata 1140

ttcgcaattg aggagtgcct gattcaagag atcaggcact cctcaattgc ttttttggaa 1200

ctcgag 1206

<210> 62
<211> 1664
<212> DNA
<213> Artificial Sequence

<220>
<223> Nucleic acid construct

<400> 62
cagaggtgta aagtacttga gtaattttac ttgattactg tacttaagta ttatttttgg 60

ggatttttac tttacttgag tacaattaaa aatcaatact tttactttta cttaattaca 120

tttttttaga aaaaaaagta ctttttactc cttacaattt tatttacagt caaaaagtac 180

ttattttttg gagatcactt cattctattt tcccttgcta ttaccaaacc aattgaattg 240

cgctgatgcc cagtttaatt taaatagatc tctcgacgaa ttgtgggacg gcggaagacg 300

ggctcccgcc ccgcccctat atgcaaggca gagaacttcc cgccgtgcac cgcgcgcaat 360

cggaagagaa tttgggcact tcagacccaa aaaaaaaccc aaaacttctg cgaaaaagaa 420

agaatctcag cggagtaaat agggattttt gttaaagagg tgataccaga agaagaaata 480

tgcaaataca acgccagctc accgctactt aaaaatcatg atataataga ggcttaaata 540

ctgtccgaga gacactgggg tttatcttat ttcttcggag acaattcaag agattgtctc 600

cgaagaaata agattttttt ggaactcgac cgaagaaccg agcgctgctg gccttaaagt 660

cccaccaaaa ctctgaagaa acgaagccag acccggcact cagcgggcag cccgcgcctc 720

ccgccgcccc acagtgccgc gcgcgtgcat ttgcatagcg cggtgctcgc agggggaaac 780

tcaccccctc aagtccgccc cccgcttccc gcccgctgtc ccgcacctca tcagtgctgt 840

gcgctgtctg tgtcccccag cacgcactct ttgctgttct tacccggagg cttgccctat 900

ccttgaggtt tctatttttt aggctataaa taccgcctag gaggtagaga tattcgcaat 960

tgaggagtgc ctgattcaag agatcaggca ctcctcaatt gcttttttgg aagtcgagga 1020

ggctcagtgt cacgcagagc gcgggacgag cgctccgagc cctcccagtg ccgcccccca 1080

aggcagggcg gccggcgcag ctccccgcag cccgccagtg ggaaggctct gctttgcata 1140

acgcgcaagg cctgctggga ggaaagcgga gcgagaaaga gcgttaacgt gcgccgagtg 1200

ttttagagca aaagcattca gacctgaagc agcgctgaga gatgcctctg ccgcccattt 1260

actggaacgt tcagacccac cgcaagtcac cgtgaccttg aggacactga gctgttggcc 1320

gttatatagc acttggggca gctcgtagct ttgatctgtt ccaccattga attcaagaga 1380

ttcaatggtg gaacagatct tttttggaac tcgaggtcga ctctagagcg gccgcgcgca 1440

ctagtgaatt ccatggatat caagcttaaa caagaatctc tagttttctt tcttgctttt 1500

acttttactt ccttaatact caagtacaat tttaatggag tactttttta cttttactca 1560

agtaagattc tagccagata cttttacttt taattgagta aaattttccc taagtacttg 1620

tactttcact tgagtaaaat ttttgagtac tttttacacc tctg 1664

<210> 63
<211> 1723
<212> DNA
<213> Artificial Sequence

<220>
<223> Nucleic acid construct

<400> 63
   cagaggtgta aagtacttga gtaattttac ttgattactg tacttaagta ttatttttgg 60
   ggatttttac tttacttgag tacaattaaa aatcaatact tttactttta cttaattaca 120
   tttttttaga aaaaaaagta ctttttactc cttacaattt tatttacagt caaaaagtac 180
   ttattttttg gagatcactt cattctattt tcccttgcta ttaccaaacc aattgaattg 240
   cgctgatgcc cagtttaatt taaatagatc tctcgacgaa ttgtgggacg gcggaagacg 300
   ggctcccgcc ccgcccctat atgcaaagca gagaacttcc cgccgtgcac cgcgcgcaat 360
   cggaagagaa tttgggcact tcagacccaa aaaaaaaccc aaaacttctg cgaaaaagaa 420
   agaatctcag cggagtaaat agggattttt gttaaagagg tgataccaga agaagaaata 480
   tgcaaataca acgccagctc accgctactt aaaaatcatg atataataga ggcttaaata 540
   ctgtccgaga gacactgggg tttgatctgt tccaccattg aattcaagag attcaatggt 600
   ggaacagatc ttttttggaa ctcgaccaga cagacgtcag gctttctaag cctggactga 660
   gtaagagcgg aagagctcca cagcactctg agtgcgcaca gaccgcgcgt acagcgcaca 720
   gccgcgcggc cgctccttca ggcactgccg acgacagccc aggcggaggt cctgagcgcc 780
   ggcgctaaat ttgcataaag aactacccag gagccctcgc gcgcggaaac gggcaaaaag 840
   gggcttctaa tatggaaata ttacgccgaa tcgcgttaca aatcggctaa gcgggcctaa 900
   gagttaacaa gatgtgctat taagcggagc cttttggtgg gaagaaatgg agtagtcact 960
   gtgttctaaa agaacttgca gaatgagcct ttaaataccg cagtctcgat gctcttagtc 1020
   tcttatttct tcggagacaa ttcaagagat tgtctccgaa gaaataagat ttttttggaa 1080
   ctcgaccgaa gaaccgagcg ctgctggcct taaagtccca ccaaaactct gaagaaacga 1140
   agccagaccc ggcactcagc gggcagcccg cgcctcccgc cgccccacag tgccgcgcgc 1200
   gtgcatttgc atagcgcggt gctcgcaggg ggaaactcac cccctcaagt ccgccccccg 1260
   cttcccgccc gctgtcccgc acctcatcag tgctgtgcgc tgtctgtgtc ccccagcacg 1320
   cactctttgc tgttcttacc cggaggcttg ccctatcctt gaggtttcta ttttttaggc 1380
   tataaatacc gcctaggagg tagagatatt cgcaattgag gagtgcctga ttcaagagat 1440
   caggcactcc tcaattgctt ttttggaact cgaggtcgac tctagagcgg ccgcgcgcac 1500
   tagtgaattc catggatatc aagcttaaac aagaatctct agttttcttt cttgctttta 1560
   cttttacttc cttaatactc aagtacaatt ttaatggagt acttttttac ttttactcaa 1620
   gtaagattct agccagatac ttttactttt aattgagtaa aattttccct aagtacttgt 1680
   actttcactt gagtaaaatt tttgagtact ttttacacct ctg 1723

<210> 64
<211> 3459
<212> DNA
<213> Artificial Sequence

<220>
<223> pminiTol2

<400> 64
   gggcgaattg ggcccagagg tgtaaagtac ttgagtaatt ttacttgatt actgtactta 60
   agtattattt ttggggattt ttactttact tgagtacaat taaaaatcaa tacttttact 120
   tttacttaat tacatttttt tagaaaaaaa agtacttttt actccttaca attttattta 180
   cagtcaaaaa gtacttattt tttggagatc acttcattct attttccctt gctattacca 240
   aaccaattga attgcgctga tgcccagttt aatttaaata gatctggcca tctagagcgg 300
   ccgcgcgcac tagtgaattc catggatatc aagcttaaac aagaatctct agttttcttt 360
   cttgctttta cttttacttc cttaatactc aagtacaatt ttaatggagt acttttttac 420
   ttttactcaa gtaagattct agccagatac ttttactttt aattgagtaa aattttccct 480
   aagtacttgt actttcactt gagtaaaatt tttgagtact ttttacacct ctgctcgacc 540
   atatgggaga gctcccaacg cgttggatgc atagcttgag tattctatag tgtcacctaa 600
   atagcttggc gtaatcatgg tcatagctgt ttcctgtgtg aaattgttat ccgctcacaa 660
   ttccacacaa catacgagcc ggaagcataa agtgtaaagc ctggggtgcc taatgagtga 720
   gctaactcac attaattgcg ttgcgctcac tgcccgcttt ccagtcggga aacctgtcgt 780
   gccagctgca ttaatgaatc ggccaacgcg cggggagagg cggtttgcgt attgggcgct 840
   cttccgcttc ctcgctcact gactcgctgc gctcggtcgt tcggctgcgg cgagcggtat 900
   cagctcactc aaaggcggta atacggttat ccacagaatc aggggataac gcaggaaaga 960
   acatgtgagc aaaaggccag caaaaggcca ggaaccgtaa aaaggccgcg ttgctggcgt 1020
   ttttccatag gctccgcccc cctgacgagc atcacaaaaa tcgacgctca agtcagaggt 1080
   ggcgaaaccc gacaggacta taaagatacc aggcgtttcc ccctggaagc tccctcgtgc 1140
   gctctcctgt tccgaccctg ccgcttaccg gatacctgtc cgcctttctc ccttcgggaa 1200
   gcgtggcgct ttctcatagc tcacgctgta ggtatctcag ttcggtgtag gtcgttcgct 1260
   ccaagctggg ctgtgtgcac gaaccccccg ttcagcccga ccgctgcgcc ttatccggta 1320
   actatcgtct tgagtccaac ccggtaagac acgacttatc gccactggca gcagccactg 1380
   gtaacaggat tagcagagcg aggtatgtag gcggtgctac agagttcttg aagtggtggc 1440
   ctaactacgg ctacactaga agaacagtat ttggtatctg cgctctgctg aagccagtta 1500
   ccttcggaaa aagagttggt agctcttgat ccggcaaaca aaccaccgct ggtagcggtg 1560
   gtttttttgt ttgcaagcag cagattacgc gcagaaaaaa aggatctcaa gaagatcctt 1620
   tgatcttttc tacggggtct gacgctcagt ggaacgaaaa ctcacgttaa gggattttgg 1680
   tcatgagatt atcaaaaagg atcttcacct agatcctttt aaattaaaaa tgaagtttta 1740
   aatcaatcta aagtatatat gagtaaactt ggtctgacag ttaccaatgc ttaatcagtg 1800
   aggcacctat ctcagcgatc tgtctatttc gttcatccat agttgcctga ctccccgtcg 1860
   tgtagataac tacgatacgg gagggcttac catctggccc cagtgctgca atgataccgc 1920
   gagacccacg ctcaccggct ccagatttat cagcaataaa ccagccagcc ggaagggccg 1980
   agcgcagaag tggtcctgca actttatccg cctccatcca gtctattaat tgttgccggg 2040
   aagctagagt aagtagttcg ccagttaata gtttgcgcaa cgttgttgcc attgctacag 2100
   gcatcgtggt gtcacgctcg tcgtttggta tggcttcatt cagctccggt tcccaacgat 2160
   caaggcgagt tacatgatcc cccatgttgt gcaaaaaagc ggttagctcc ttcggtcctc 2220
   cgatcgttgt cagaagtaag ttggccgcag tgttatcact catggttatg gcagcactgc 2280
   ataattctct tactgtcatg ccatccgtaa gatgcttttc tgtgactggt gagtactcaa 2340
   ccaagtcatt ctgagaatag tgtatgcggc gaccgagttg ctcttgcccg gcgtcaatac 2400
   gggataatac cgcgccacat agcagaactt taaaagtgct catcattgga aaacgttctt 2460
   cggggcgaaa actctcaagg atcttaccgc tgttgagatc cagttcgatg taacccactc 2520
   gtgcacccaa ctgatcttca gcatctttta ctttcaccag cgtttctggg tgagcaaaaa 2580
   caggaaggca aaatgccgca aaaaagggaa taagggcgac acggaaatgt tgaatactca 2640
   tactcttcct ttttcaatat tattgaagca tttatcaggg ttattgtctc atgagcggat 2700
   acatatttga atgtatttag aaaaataaac aaataggggt tccgcgcaca tttccccgaa 2760
   aagtgccacc tgatgcggtg tgaaataccg cacagatgcg taaggagaaa ataccgcatc 2820
   aggaaattgt aagcgttaat attttgttaa aattcgcgtt aaatttttgt taaatcagct 2880
   cattttttaa ccaataggcc gaaatcggca aaatccctta taaatcaaaa gaatagaccg 2940
   agatagggtt gagtgttgtt ccagtttgga acaagagtcc actattaaag aacgtggact 3000
   ccaacgtcaa agggcgaaaa accgtctatc agggcgatgg cccactacgt gaaccatcac 3060
   cctaatcaag ttttttgggg tcgaggtgcc gtaaagcact aaatcggaac cctaaaggga 3120
   gcccccgatt tagagcttga cggggaaagc cggcgaacgt ggcgagaaag gaagggaaga 3180
   aagcgaaagg agcgggcgct agggcgctgg caagtgtagc ggtcacgctg cgcgtaacca 3240
   ccacacccgc cgcgcttaat gcgccgctac agggcgcgtc cattcgccat tcaggctgcg 3300
   caactgttgg gaagggcgat cggtgcgggc ctcttcgcta ttacgccagc tggcgaaagg 3360
   gggatgtgct gcaaggcgat taagttgggt aacgccaggg ttttcccagt cacgacgttg 3420
   taaaacgacg gccagtgaat tgtaatacga ctcactata 3459

<210> 65
<211> 5693
<212> DNA
<213> Artificial Sequence

<220>
<223> pCMV-Tol2

<400> 65
   ggccgccacc atggaggaag tatgtgattc atcagcagct gcgagcagca cagtccaaaa 60
   tcagccacag gatcaagagc acccgtggcc gtatcttcgc gaattctttt ctttaagtgg 120
   tgtaaataaa gattcattca agatgaaatg tgtcctctgt ctcccgctta ataaagaaat 180
   atcggccttc aaaagttcgc catcaaacct aaggaagcat attgagagaa tgcacccaaa 240
   ttacctcaaa aactactcta aattgacagc acagaagaga aagatcggga cctccaccca 300
   tgcttccagc agtaagcaac tgaaagttga ctcagttttc ccagtcaaac atgtgtctcc 360
   agtcactgtg aacaaagcta tattaaggta catcattcaa ggacttcatc ctttcagcac 420
   tgttgatctg ccatcattta aagagctgat tagtacactg cagcctggca tttctgtcat 480
   tacaaggcct actttacgct ccaagatagc tgaagctgct ctgatcatga aacagaaagt 540
   gactgctgcc atgagtgaag ttgaatggat tgcaaccaca acggattgtt ggactgcacg 600
   tagaaagtca ttcattggtg taactgctca ctggatcaac cctggaagtc ttgaaagaca 660
   ttccgctgca cttgcctgca aaagattaat gggctctcat acttttgagg tactggccag 720
   tgccatgaat gatatccact cagagtatga aatacgtgac aaggttgttt gcacaaccac 780
   agacagtggt tccaacttta tgaaggcttt cagagttttt ggtgtggaaa acaatgatat 840
   cgagactgag gcaagaaggt gtgaaagtga tgacactgat tctgaaggct gtggtgaggg 900
   aagtgatggt gtggaattcc aagatgcctc acgagtcctg gaccaagacg atggcttcga 960
   attccagcta ccaaaacatc aaaagtgtgc ctgtcactta cttaacctag tctcaagcgt 1020
   tgatgcccaa aaagctctct caaatgaaca ctacaagaaa ctctacagat ctgtctttgg 1080
   caaatgccaa gctttatgga ataaaagcag ccgatcggct ctagcagctg aagctgttga 1140
   atcagaaagc cggcttcagc ttttaaggcc aaaccaaacg cggtggaatt caacttttat 1200
   ggctgttgac agaattcttc aaatttgcaa agaagcagga gaaggcgcac ttcggaatat 1260
   atgcacctct cttgaggttc caatgtttaa tccagcagaa atgctgttct tgacagagtg 1320
   ggccaacaca atgcgtccag ttgcaaaagt actcgacatc ttgcaagcgg aaacgaatac 1380
   acagctgggg tggctgctgc ctagtgtcca tcagttaagc ttgaaacttc agcgactcca 1440
   ccattctctc aggtactgtg acccacttgt ggatgcccta caacaaggaa tccaaacacg 1500
   attcaagcat atgtttgaag atcctgagat catagcagct gccatccttc tccctaaatt 1560
   tcggacctct tggacaaatg atgaaaccat cataaaacga ggcatggact acatcagagt 1620
   gcatctggag cctttggacc acaagaagga attggccaac agttcatctg atgatgaaga 1680
   ttttttcgct tctttgaaac cgacaacaca tgaagccagc aaagagttgg atggatatct 1740
   ggcctgtgtt tcagacacca gggagtctct gctcacgttt cctgctattt gcagcctctc 1800
   tatcaagact aatacacctc ttcccgcatc ggctgcctgt gagaggcttt tcagcactgc 1860
   aggattgctt ttcagcccca aaagagctag gcttgacact aacaattttg agaatcagct 1920
   tctactgaag ttaaatctga ggttttacaa ctttgagtag actagtctga agggcgaatt 1980
   ctgcagatat ccatcacact ggcggccgcg ggatccagac atgataagat tacattgatg 2040
   agtttggaca aaccacaact agaatgcagt gaaaaaaatg ctttatttgt gaaatttgtg 2100
   atgctattgc tttatttgta accattataa gctgcaataa acaagttaac aacaacaatt 2160
   gcattcattt tatgtttcag gttcaggggg aggtgtggga ggttttttcg gatcctctag 2220
   agtcgacctg caggcatgca agcttggcgt aatcatggtc atagctgttt cctgtgtgaa 2280
   attgttatcc gctcacaatt ccacacaaca tacgagccgg aagcataaag tgtaaagcct 2340
   ggggtgccta atgagtgagc taactcacat taattgcgtt gcgctcactg cccgctttcc 2400
   agtcgggaaa cctgtcgtgc cagctgcatt aatgaatcgg ccaacgcgcg gggagaggcg 2460
   gtttgcgtat tgggcgctct tccgcttcct cgctcactga ctcgctgcgc tcggtcgttc 2520
   ggctgcggcg agcggtatca gctcactcaa aggcggtaat acggttatcc acagaatcag 2580
   gggataacgc aggaaagaac atgtgagcaa aaggccagca aaaggccagg aaccgtaaaa 2640
   aggccgcgtt gctggcgttt ttccataggc tccgcccccc tgacgagcat cacaaaaatc 2700
   gacgctcaag tcagaggtgg cgaaacccga caggactata aagataccag gcgtttcccc 2760
   ctggaagctc cctcgtgcgc tctcctgttc cgaccctgcc gcttaccgga tacctgtccg 2820
   cctttctccc ttcgggaagc gtggcgcttt ctcatagctc acgctgtagg tatctcagtt 2880
   cggtgtaggt cgttcgctcc aagctgggct gtgtgcacga accccccgtt cagcccgacc 2940
   gctgcgcctt atccggtaac tatcgtcttg agtccaaccc ggtaagacac gacttatcgc 3000
   cactggcagc agccactggt aacaggatta gcagagcgag gtatgtaggc ggtgctacag 3060
   agttcttgaa gtggtggcct aactacggct acactagaag gacagtattt ggtatctgcg 3120
   ctctgctgaa gccagttacc ttcggaaaaa gagttggtag ctcttgatcc ggcaaacaaa 3180
   ccaccgctgg tagcggtggt ttttttgttt gcaagcagca gattacgcgc agaaaaaaag 3240
   gatctcaaga agatcctttg atcttttcta cggggtctga cgctcagtgg aacgaaaact 3300
   cacgttaagg gattttggtc atgagattat caaaaaggat cttcacctag atccttttaa 3360
   attaaaaatg aagttttaaa tcaatctaaa gtatatatga gtaaacttgg tctgacagtt 3420
   accaatgctt aatcagtgag gcacctatct cagcgatctg tctatttcgt tcatccatag 3480
   ttgcctgact ccccgtcgtg tagataacta cgatacggga gggcttacca tctggcccca 3540
   gtgctgcaat gataccgcga gacccacgct caccggctcc agatttatca gcaataaacc 3600
   agccagccgg aagggccgag cgcagaagtg gtcctgcaac tttatccgcc tccatccagt 3660
   ctattaattg ttgccgggaa gctagagtaa gtagttcgcc agttaatagt ttgcgcaacg 3720
   ttgttgccat tgctacaggc atcgtggtgt cacgctcgtc gtttggtatg gcttcattca 3780
   gctccggttc ccaacgatca aggcgagtta catgatcccc catgttgtgc aaaaaagcgg 3840
   ttagctcctt cggtcctccg atcgttgtca gaagtaagtt ggccgcagtg ttatcactca 3900
   tggttatggc agcactgcat aattctctta ctgtcatgcc atccgtaaga tgcttttctg 3960
   tgactggtga gtactcaacc aagtcattct gagaatagtg tatgcggcga ccgagttgct 4020
   cttgcccggc gtcaatacgg gataataccg cgccacatag cagaacttta aaagtgctca 4080
   tcattggaaa acgttcttcg gggcgaaaac tctcaaggat cttaccgctg ttgagatcca 4140
   gttcgatgta acccactcgt gcacccaact gatcttcagc atcttttact ttcaccagcg 4200
   tttctgggtg agcaaaaaca ggaaggcaaa atgccgcaaa aaagggaata agggcgacac 4260
   ggaaatgttg aatactcata ctcttccttt ttcaatatta ttgaagcatt tatcagggtt 4320
   attgtctcat gagcggatac atatttgaat gtatttagaa aaataaacaa ataggggttc 4380
   cgcgcacatt tccccgaaaa gtgccacctg acgtctaaga aaccattatt atcatgacat 4440
   taacctataa aaataggcgt atcacgaggc cctttcgtct cgcgcgtttc ggtgatgacg 4500
   gtgaaaacct ctgacacatg cagctcccgg agacggtcac agcttgtctg taagcggatg 4560
   ccgggagcag acaagcccgt cagggcgcgt cagcgggtgt tggcgggtgt cggggctggc 4620
   ttaactatgc ggcatcagag cagattgtac tgagagtgca ccatatgcgg tgtgaaatac 4680
   cgcacagatg cgtaaggaga aaataccgca tcaggcgcca ttcgccattc aggctgcgca 4740
   actgttggga agggcgatcg gtgcgggcct cttcgctatt acgccagctg gcgaaagggg 4800
   gatgtgctgc aaggcgatta agttgggtaa cgccagggtt ttcccagtca cgacgttgta 4860
   aaacgacggc cagtgaattc gagcttgcat gcctgcaggt cgttacataa cttacggtaa 4920
   atggcccgcc tggctgaccg cccaacgacc cccgcccatt gacgtcaata atgacgtatg 4980
   ttcccatagt aacgccaata gggactttcc attgacgtca atgggtggag tatttacggt 5040
   aaactgccca cttggcagta catcaagtgt atcatatgcc aagtacgccc cctattgacg 5100
   tcaatgacgg taaatggccc gcctggcatt atgcccagta catgacctta tgggactttc 5160
   ctacttggca gtacatctac gtattagtca tcgctattac catggtgatg cggttttggc 5220
   agtacatcaa tgggcgtgga tagcggtttg actcacgggg atttccaagt ctccacccca 5280
   ttgacgtcaa tgggagtttg ttttggcacc aaaatcaacg ggactttcca aaatgtcgta 5340
   acaactccgc cccattgacg caaatgggcg gtaggcgtgt acggtgggag gtctatataa 5400
   gcagagctcg tttagtgaac cgtcagatcg cctggagacg ccatccacgc tgttttgacc 5460
   tccatagaag acaccgggac cgatccagcc tccggactct agaggatccg gtactcgagg 5520
   aactgaaaaa ccagaaagtt aactggtaag tttagtcttt ttgtctttta tttcaggtcc 5580
   cggatccggt ggtggtgcaa atcaaagaac tgctcctcag tggatgttgc ctttacttct 5640
   aggcctgtac ggaagtgtta cttctgctct aaaagctgcg gaattgtacc cgc 5693

## Description

### FIELD OF INVENTION

The present invention relates to nucleic acid molecules comprising a double-stranded region, and nucleic acid constructs encoding therfor, that are useful for the treatment and/or prevention of influenza. In particular, the present invention relates to nucleic acid constructs encoding a double stranded RNA molecule(s) that can be used to produce transgenic animals, for example chickens, such that they are at least less susceptible to an avian influenza infection. Also provided are nucleic acid molecules comprising a double-stranded region that can be used as a therapeutic to treat and/or prevent, for example, avian influenza in poultry.

### BACKGROUND OF THE INVENTION

Three types of influenza viruses, types A, B, and C are known and they belong to a family of single-stranded negative-sense enveloped RNA viruses called Orthomyxoviridae. The viral genome is approximately 12000 to 15000 nucleotides in length and comprises eight RNA segments (seven in Type C) that encode eleven proteins.

Influenza A virus infects many animals such as humans, pigs, horses, marine mammals, and birds (Nicholson et al., 2005). Its natural reservoir is in aquatic birds, and in avian species most influenza virus infections cause mild localized infections of the respiratory and intestinal tract. However, the virus can have a highly pathogenic effect in poultry, with sudden outbreaks causing high mortality rates in affected poultry populations.

Influenza A viruses can be classified into subtypes based on allelic variations in antigenic regions of two genes that encode surface glycoproteins, namely, hemagglutinin (HA) and neuraminidase (NA) which are required for viral attachment and cellular release. Other major viral proteins include the nucleoprotein, the nucleocapsid structural protein, matrix proteins (Ml and M2), polymerases (PA, PB1 and PB2), and non-structural proteins (NS1 and NS2).

At least sixteen subtypes of HA (H1 to H16) and nine NA (N1 to N9) antigenic variants are known in influenza A virus. Avian influenza strains can also be characterized as low pathogenic and highly pathogenic strains. Low pathogenic strains typically only have two basic amino acids at positions -1 and -3 of the cleavage site of the HA precursor, while highly pathogenic strains have a multi-basic cleavage site. Subtypes H5 and H7 can cause highly pathogenic infections in poultry and certain subtypes have been shown to cross the species barrier to humans. Highly pathogenic H5 and H7 viruses can also emerge from low pathogenic precursors in domestic poultry. Symptoms of avian influenza infection range from typical influenza type symptoms (fever, cough, sore throat and muscle aches) to conjunctivitis, pneumonia, acute respiratory distress, and other life-threatening complications.

There is a need to develop ways of controlling influenza virus survival and/or replication in animals such as poultry not only to improve productivity and welfare in the livestock industry, but to also reduce health risks to humans.

### SUMMARY OF THE INVENTION

The present inventors have identified nucleic acid molecules comprising double-stranded regions that are capable of reducing influenza virus replication and/or production in infected animal cells.

Accordinlgy, the present invention provides a nucleic acid construct encoding an RNA molecule comprising a double-stranded region, wherein the RNA molecule reduces influenza A virus replication in an animal cell and/or reduces production of infectious influenza A virus particles in an animal cell and/or reduces the expression of an influenza A virus polypeptide in an influenza A virus infected animal cell when compared to an isogenic influenza A virus infected animal cell lacking the RNA molecule.

Preferably, the double-stranded region comprises a sequence of nucleotides selected from:
(i) nucleotides within positions 2240 to 2341 of SEQ ID NO:1,
(ii) nucleotides within positions 2257 to 2341 of SEQ ID NO:2,
(iii) nucleotides within positions 2087 to 2233 of SEQ ID NO:3,
(iv) nucleotides within positions 1484 to 1565 of SEQ ID NO:4,
(v) a nucleotide sequence of any one of SEQ ID NOs:6 to 15 or 52 to 54,
(vi) a nucleotide sequence which is at least 90% identical to any one of (i) to (v),
(vii) a nucleotide sequence which hybridizes to any one of (i) to (v) under stringent conditions.

In one embodiment, the RNA molecule reduces influenza A virus replication in an animal cell when compared to an isogenic influenza A virus infected animal cell lacking the RNA molecule. In another embodiment, the RNA molecule reduces production of infectious influenza A virus particles in an animal cell when compared to an isogenic influenza A virus infected animal cell lacking the RNA molecule. In yet another embodiment, the RNA molecule reduces the expression of an influenza A virus polypeptide in an influenza A virus infected animal cell when compared to an isogenic infected animal cell lacking the RNA molecule.

Preferably, the influenza A virus is an avian influenza virus.

The nucleic acid construct of the invention may encode any type of RNA molecule comprising a double stranded region. Preferably, the double-stranded region is at least 19 basepairs in length. It is also preferable that the double-stranded region is less than 100 basepairs in length.

In a particularly preferred embodiment, the encoded RNA molecule is a short hairpin RNA.

In one prefered embodiment, the double-stranded region encoded by the RNA molecule comprises the sequence of nucleotides of SEQ ID NO:7.

In another preferred embodiment, the double-stranded region encoded by the RNA molecule comprises the sequence of nucleotides of SEQ ID NO:9.

In another prefered embodiment, the double-stranded region encoded by the RNA molecule comprises the sequence of nucleotides of SEQ ID NO:12.

In yet another prefered embodiment, the double-stranded region encoded by the RNA molecule comprises the sequence of nucleotides of SEQ ID NO:6.

In one prefered embodiment, the double-stranded region encoded by the RNA molecule comprises the sequence of nucleotides of SEQ ID NO:8.

In yet another prefered embodiment, the double-stranded region encoded by the RNA molecule comprises the sequence of nucleotides of SEQ ID NO:13.

In yet another prefered embodiment, the double-stranded region encoded by the RNA molecule comprises the sequence of nucleotides of SEQ ID NO:15.

In some instances it may be desirable that the nucleic acid construct encodes more than one RNA molecule, for example 2, 3, 4, 5 or more RNA molecules. Accordingly, the present invention provides a nucleic acid construct which encodes two or more RNA molecules. The encoded RNA molecules may be different or the same, or a combination thereof. Furthermore, the encoded RNA molecules may target the same or different influenza A virus genes, or a combination thereof. In one embodiment, each RNA molecule comprises a nucleotide sequence corresponding to a different influenza A virus gene.

The present invention further provides a nucleic acid construct of the invention, wherein each RNA molecule is encoded by a nucleotide sequence operably linked to a RNA polymerase II promoter or a RNA polymerase III promoter. In a preferred embodiment, the promoters are RNA polymerase III promoters.

In some instances it may be desirable for the promoter sequence to be the same as a naturally occurring promoter sequence from an animal and/or cell, or progeny thereof, into which the nucleic acid construct is transfected/transformed. In one embodiment, the promoter is a chicken, turkey and/or duck promoter.

Preferably, the promoter is selected from a U6, 7SK and/or H1 promoter.

In one particular embodiment, the U6 promoter is cU6-1, cU6-2, cU6-3, and/or cU6-4.

In a further embodiment, the promoter comprises a nucleotide sequence selected from any one of SEQ ID NOs:22 to 25.

The present inventors have unexpectedly found that nucleic acid constructs comprising U6 promoters containing the minimum amount of promoter sequence required to elicit transcription of the shRNAs were at least equally as effective at transcribing shRNAs as constructs comprising U6 promoters with an additional 100bp of upstream sequence. Accordingly, in one embodiment of the invention, the promoter consists of a nucleotide sequence selected from any one of SEQ ID NOs:22 to 25.

In yet another embodiment, each nucleotide sequence encoding a RNA molecule is operably linked to a different RNA polymerase III promoter.

In one embodiment, the RNA molecule reduces the expression of an influenza A virus polypeptide encoded by any one of SEQ ID NOs:1 to 5.

In one particular embodiment, the influenza A virus polypeptide may be selected from PB1, PB2, PA, NP and/or M1. Preferably, the polypeptide is an avian influenza polypeptide.

In one embodiment, the avian influenza is a highly pathogenic strain.

Preferably, the avian influenza is H5N1.

In another embodiment, the construct comprises only influenza A virus sequences and naturally occurring host animal sequences. For example, when a construct is designed for transfection into a chicken, the nucleic acid construct will desirably consist of chicken and influenza A virus sequences. Therefore, in one embodiment the present invention provides a nucleic acid construct of the invention, wherein the construct consists of chicken and influenza A virus nucleotide sequences.

In a preferred embodiment the nucleic acid construct of the invention encodes three RNA molecules comprising a double-stranded region, wherein the double-stranded regions comprise nucleotide sequences selected from:
(i) SEQ ID NO:9, SEQ ID NO:13 and SEQ ID NO:15,
(ii) SEQ ID NO:6, SEQ ID NO:7 and SEQ ID NO:8, and
(iii) SEQ ID NO:7, SEQ ID NO:9 and SEQ ID NO:12.

In another preferred embodiment, the nucleic acid construct comprises a nucleotide sequence selected from SEQ ID NOs:16 to 21 and 61 to 63, or a fragment thereof, or a sequence that is at least 95% identical to a nucleotide sequence selected from SEQ ID NOs:16 to 21 and 61 to 63. Preferably the fragment comprises the nucleotide sequence missing 100 nucleotides or less from the 5' and/or 3' end of the sequence, more preferably 50 nucleotides or less from the 5' and/or 3' end of the sequence, more preferably 20 nucleotides or less from the 5' and/or 3' end of the sequence, more preferably 10 nucleotides or less from the 5' and/or 3' end of the sequence.

The present invention further provides an isolated and/or exogenous nucleic acid molecule comprising a double-stranded region, wherein the double-stranded region comprises a sequence of nucleotides selected from:
(i) nucleotides within positions 2240 to 2341 of SEQ ID NO:1,
(ii) nucleotides within positions 2257 to 2341 of SEQ ID NO:2,
(iii) nucleotides within positions 2087 to 2233 of SEQ ID NO:3,
(iv) nucleotides within positions 1484 to 1565 of SEQ ID NO:4,
(v) a nucleotide sequence of any one of SEQ ID NOs:6 to 15 or 52 to 54,
(vi) a nucleotide sequence which is at least 90% identical to any one of (i) to (v),
(vii) a nucleotide sequence which hybridizes to any one of (i) to (v) under stringent conditions.

In one embodiment, the isolated and/or exogenous nucleic acid molecule reduces influenza A virus replication in an animal cell when compared to an isogenic influenza A virus infected animal cell lacking the nucleic acid molecule. In another embodiment, the isolated and/or exogenous nucleic acid molecule reduces the production of infectious influenza A virus particles in an animal cell when compared to an isogenic influenza A virus infected animal cell lacking the nucleic acid molecule. In yet another embodiment, the isolated and/or exogenous nucleic acid molecule reduces the expression of an influenza A virus polypeptide in an influenza A virus infected animal cell when compared to an isogenic influenza A virus infected animal cell lacking the nucleic acid molecule.

In another embodiment, the isolated and/or exogenous nucleic acid molecule of the invention reduces the expression of an influenza A virus polypeptide encoded by any one of SEQ ID NOs:1 to 5.

Preferably, the double-stranded region of the isolated and/or exogenous nucleic acid molecule is at least 19 nucleotides in length. In some embodiments, it is preferred that the double-stranded region is less than 100 nucleotides in length.

In one embodiment, the isolated and/or exogenous nucleic acid molecule comprises double-stranded RNA.

Preferably, the isolated and/or exogenous nucleic acid molecule is a short interfering RNA or a short hairpin RNA.

In one preferred embodiment, the isolated and/or exogenous nucleic acid molecule comprises a nucleotide sequence selected from SEQ ID NOs:16 to 21 and 61 to 63, or a fragment thereof, or a sequence that is at least 95% identical to a nucleotide sequence selected from SEQ ID NOs:16 to 21 and 61 to 63. Preferably the fragment comprises the nucleotide sequence missing 100 nucleotides or less from the 5' and/or 3' end of the sequence, more preferably 50 nucleotides or less from the 5' and/or 3' end of the sequence, more preferably 20 nucleotides or less from the 5' and/or 3' end of the sequence, more preferably 10 nucleotides or less from the 5' and/or 3' end of the sequence.

In one embodiment of the present invention, the nucleic acid construct and/or the isolated and/or exogenous nucleic acid molecule is present in the genome of a cell.

The present invention further provides a vector comprising the nucleic acid construct of the invention and/or the isolated and/or exogenous nucleic acid molecule of the invention.

The present invention further provides a cell comprising the nucleic acid construct, the isolated and/or exogenous nucleic acid molecule and/or the vector of the invention.

In one embodiment of the invention, the cell is a primordial germ cell, for example a chicken primordial germ cell.

The present invention further provides a transgenic non-human organism comprising the nucleic acid construct, the isolated and/or exogenous nucleic acid molecule, the vector and/or the cell of the invention. The transgenic organism may be any organism, for example, an animal or a plant.

In one embodiment, the transgenic organism is a non-human animal.

In another embodiment, the transgenic organism is avian. In a preferred embodiment, the transgenic organism is poultry. In an even more preferred embodiment, the transgenic organism is a chicken, turkey or duck.

In one embodiment, the transgenic organism of the invention comprises a nucleotide sequence selected from SEQ ID Nos:16 to 21 and 61 to 63, or a fragment thereof, or a sequence that is at least 95% identical to a nucleotide sequence selected from SEQ ID NOs:16 to 21 and 61 to 63, encoding at least one RNA molecule comprising a double-stranded region. Fragments and/or sequences closely related to SEQ ID Nos:16 to 21 and 61 to 63 are encompassed by this embodiment as 5' and/or 3' regions of the construct may be lost when being integrated into the genome of the organism, and/or minor mutations during cell division over many generations may result in one or a few nucleotide differences when compared to the original construct. Preferably the fragment comprises the nucleotide sequence missing 100 nucleotides or less from the 5' and/or 3' end of the sequence, more preferably 50 nucleotides or less from the 5' and/or 3' end of the sequence, more preferably 20 nucleotides or less from the 5' and/or 3' end of the sequence, more preferably 10 nucleotides or less from the 5' and/or 3' end of the sequence.

In a further embodiment, the transgenic organism comprises two or more nucleic acid constructs of the invention.

The present invention further provides a composition comprising the nucleic acid construct of the invention, the isolated and/or exogenous nucleic acid molecule of the invention, the vector of the invention, the cell of the invention and/or the nucleic acid molecule of the invention.

The nucleic acid constructs, isolated and/or exogenous nucleic acid molecules, vectors and host cells of the invention may be used to treat and/or prevent influenza A virus infection in a subject. For example, the nucleic acid constructs, isolated and/or exogenous nucleic acid molecules, vectors and host cells may be used to protect poultry such as, but not limited to, a chicken, turkey or duck from avian influenza. In addition, in the event of a localised outbreak of avian influenza in a population of birds, it may be desirable to protect birds in surrounding areas, e.g., surrounding farms, from infection in an effort to contain the avian influenza outbreak.

Thus, the present invention further provides a method of treating and/or preventing an influenza A virus infection in a subject, the method comprising administering the nucleic acid construct of the invention, the isolated and/or exogenous nucleic acid of the invention, the vector of the invention, and/or the cell of the invention to the subject.

In one embodiment, the method comprises administering the nucleic acid construct, the isolated and/or exogenous nucleic acid, the vector, and/or the cell in drinking water or in an aerosol.

In one particular embodiment, the influenza A virus is avian influenza.

Preferably, the avian influenza is a highly pathogenic strain.

In a most preferred embodiment, the avian influenza is H5N1.

Preferably, the subject is avian, more preferably poultry. In a most preferred embodiment, the subject is a chicken, turkey or duck.

The present invention further provides a method of reducing the expression of one or more influenza A virus genes in a cell, the method comprising administering to the cell the isolated and/or exogenous nucleic acid molecule of the invention.

The present invention further provides use of the nucleic acid construct of the invention, the isolated and/or exogenous nucleic acid molecule of the invention, the vector of the invention, and/or the cell of the invention in the manufacture of a medicament for treating and/or preventing influenza A virus infection.

The present invention further provides a method of identifying an animal comprising the nucleic acid construct of the invention or the isolated and/or exogenous nucleic acid molecule of the invention, the method comprising:
determining the presence or absence of the nucleic acid construct of the invention and/or the isolated and/or exogenous nucleic acid molecule of the invention in a sample obtained from the animal.

In one embodiment, the method comprises amplifying the nucleic acid construct or a fragment thereof, or the isolated and/or exogenous nucleic acid molecule or a fragment thereof.

In another embodiment, the method comprises:
contacting a sample obtained from the animal with a probe that hybridizes under stringent conditions with the nucleic acid construct or the isolated and/or exogenous nucleic acid molecule to form a complex, and
determining the presence or absence of the complex.

The present invention further provides a method of breeding a non-human transgenic animal resistant to influenza, the method comprising:
(i) introducing the nucleic acid construct of the invention into a non-human animal cell,
(ii) selecting a transgenic non-human cell comprising the nucleic acid construct,
(iii) regenerating a transgenic non-human animal from the transgenic non-human cell,
(iv) breeding the transgenic non-human animal to produce transgenic progeny, and
(v) selecting transgenic progeny that are resistant to influenza.

The present invention further provides a method of producing food, the method comprising
(i) introducing the nucleic acid construct of the invention into an animal cell,
(ii) selecting a transgenic cell comprising the nucleic acid construct,
(iii) regenerating a transgenic animal from the transgenic cell,
(iv) breeding the transgenic animal to produce transgenic progeny, and
(v) producing food from the transgenic progeny.

In one embodiment, the food is selected from meat and eggs.

The present invention further provides a method of making a transgenic non-human animal resistant to influenza, the method comprising:
(i) introducing a first nucleic acid comprising a transposon into a cell, wherein the nucleic acid encodes a double-stranded RNA molecule,
(ii) introducing a second nucleic acid encoding a transposase into the cell,
(ii) selecting a transgenic cell comprising the first nucleic acid in the genome of the cell,
(iii) regenerating a transgenic non-human animal from the cell, and
(iv) breeding the transgenic non-human animal.

The first and second nucleic acids may be introduced into the cell on a single nucleic acid molecule, or alternatively, may be introduced into the cell on separate nucleic acid molecules. Preferably, the first and second nucleic acids are introduced into the cell on separate nucleic acid molecules.

In one embodiment, the transposon is a Tol2 transposon and the transposase is Tol2 transposase.

In another embodiment, the cell is a chicken primordial germ cell.

The present invention further provides a transgenic non-human animal resistant to influenza.

In one embodiment, the transgenic animal comprises a nucleic acid construct encoding an RNA molecule comprising a double-stranded region, wherein the RNA molecule reduces influenza virus replication in a cell of the animal when compared to an isogenic influenza virus infected animal cell lacking the RNA molecule.

In another embodiment, the transgenic animal comprises a nucleic acid construct encoding an RNA molecule comprising a double-stranded region, wherein the RNA molecule reduces production of infectious influenza virus particles in a cell of the animal when compared to an isogenic influenza virus infected animal cell lacking the RNA molecule.

In another embodiment, the transgenic animal comprises a nucleic acid construct encoding an RNA molecule comprising a double-stranded region, wherein the RNA molecule reduces the expression of an influenza virus polypeptide in an influenza virus infected cell of the animal when compared to an isogenic influenza virus infected animal cell lacking the RNA molecule.

In yet another embodiment, the transgenic non-human animal is a chicken and the influenza is influenza A.

Preferably the transgenic non-human organism comprises the nucleic acid construct of the invention, the isolated or exogenous nucleic acid molecule of the invention, the vector of the invention and/or the cell of the invention.

The present invention further provides use of the non-human transgenic animal of the invention or the transgenic non-human organism of the invention for breeding.

The present invention further provides use of the non-human transgenic animal of the invention or the non-human transgenic organism of the invention for food production.

As will be apparent, preferred features and characteristics of one aspect of the invention are applicable to many other aspects of the invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention is hereinafter described by way of the following non-limiting Examples and with reference to the accompanying Figures.

### BRIEF DESCRIPTION OF THE FIGURES

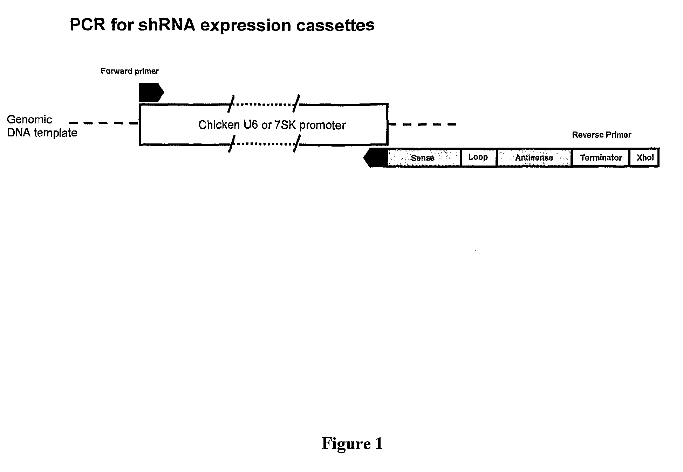

**Figure 1**. PCR for shRNA expression cassettes. Schematic representation of the PCR strategy used to produce shRNA expression vectors. PCR used forward primers paired with reverse primers comprising all shRNA components. All final PCR products consisted of a chicken U6 or 7SK promoter, shRNA sense, loop, shRNA antisense, termination sequence and *Xho*I site.

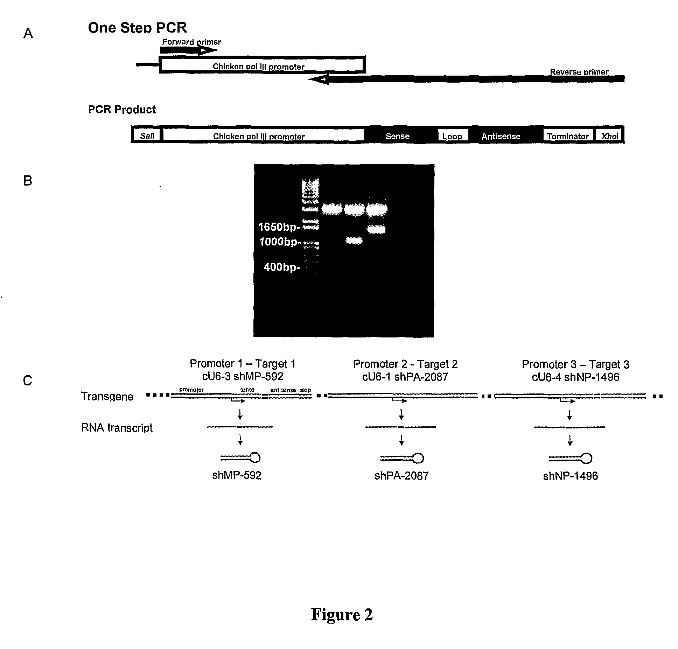

**Figure 2**. Construction of MWH-1 transgene. A, individual transcription units were produced using a one step PCR approach. The PCR products contain a chicken pol III promoter and shRNA components (sense, loop, antisense and terminator sequences). B, the three transcription units were ligated together using the compatible *Sal*I and *Xho*I sites on the 5' and 3' ends of the PCR products. C, The final transgene contains 3 transcription units that express the 3 individual shRNAs to target selected Influenza A virus genes.

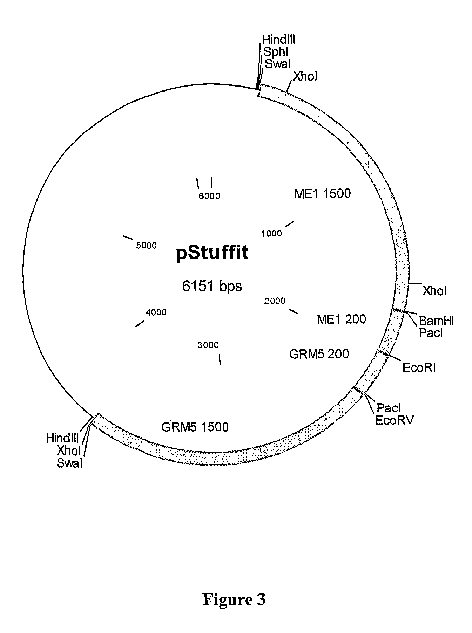

**Figure 3****.** Plasmid map of pStuffit (6151 basepairs). The four cloned regions of the chicken genome are labelled and shaded on the map. Cloning restriction sites as well as other relevant introduced restriction sites are indicated. MWH transgenes are inserted into the unique *Eco*RI site positioned between ME1 200 and GRM5 200. The *Hind*III enzyme sites of pIC20H can be used to excise the entire MWH transgenes including stuffer/buffer flanking sequence as a single fragment for insertion into the chicken genome.

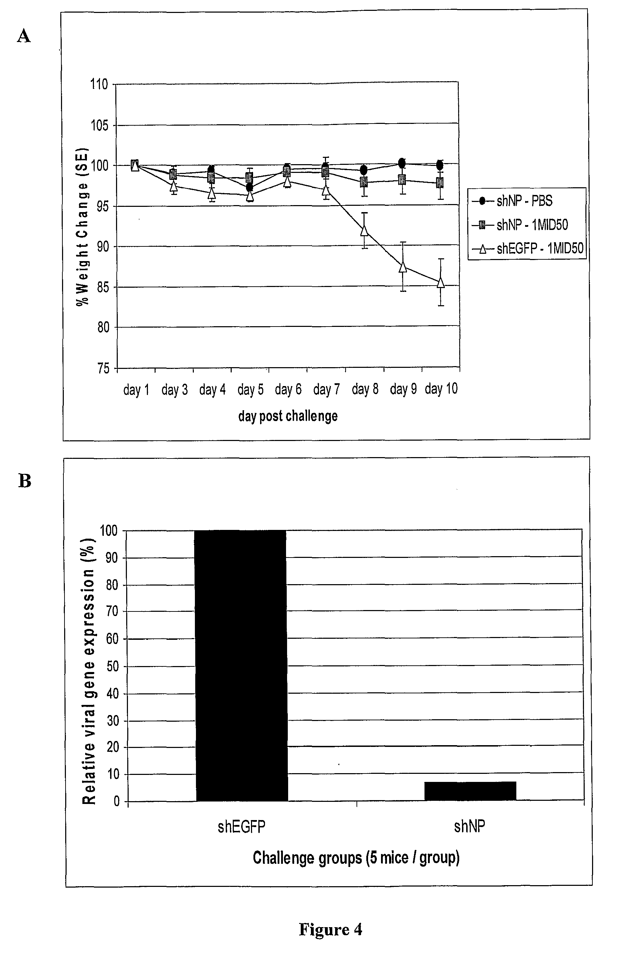

**Figure 4****.** Influenza challenge of transgenic mice. A, % weight change in mice expressing shNP-1496 versus mice expressing shEGFP. B, Relative viral gene expression in mice expressing shNP-1496 versus mice expressing shEGFP.

### KEY TO THE SEQUENCE LISTING

SEQ ID NO:1 - Consensus nucleotide sequence of the Influenza A PB2 gene.
SEQ ID NO:2 - Consensus nucleotide sequence of the Influenza A PB1 gene.
SEQ ID NO:3 - Consensus nucleotide sequence of the Influenza A PA gene.
SEQ ID NO:4 - Consensus nucleotide sequence of the Influenza A NP gene.
SEQ ID NO:5 - Consensus nucleotide sequence of the Influenza A M1 gene.
SEQ ID NOs:6 to 15 - Nucleotide sequences of nucleic acid molecules that target Influenza A genes and/or the mRNA encoded thereby.
SEQ ID NO:16 - Nucleotide sequence of MWH1 and stuffer sequences. 5' stuffer (nucleotides 1-1748); cU6-3 shMP-592 (nucleotides 1759-2234); cU6-1 shPA-2087 (nucleotides 2235-2622); cU6-4 shNP-1496 (nucleotides 2623-2974); 3' stuffer (nucleotides 2985-4745).
SEQ ID NO:17 - Nucleotide sequence of MWH2 and stuffer sequences. 5' stuffer (nucleotides 1-1748); cU6-4 shPB1-2257 (nucleotides 1774-2125); cU6-1 shPB2-2240 (nucleotides 2126-2513); c7SK shPB1-129 (nucleotides 2514-2911); 3' stuffer (nucleotides 2936-4696).
SEQ ID NO:18 - Nucleotide sequence of MWH3 and stuffer sequences. 5' stuffer (nucleotides 1-1748); cU6-4 shNP-1484 (nucleotides 1774-2129); cU6-1 shPA-2087 (nucleotides 2130-2517); c7SK shPB1-2257 (nucleotides 2518-2917); 3' stuffer (nucleotides 2947-4702).
SEQ ID NO:19 - Nucleotide sequence of MWH1.
SEQ ID NO:20 - Nucleotide sequence of MWH2.
SEQ ID NO:21 - Nucleotide sequence of MWH3.
SEQ ID NO:22 - Nucleotide sequence of chicken U6-1 promoter (cU6-1).
SEQ ID NO:23 - Nucleotide sequence of chicken U6-3 promoter (cU6-3).
SEQ ID NO:24 - Nucleotide sequence of chicken U6-4 promoter (cU6-4).
SEQ ID NO:25 - Nucleotide sequence of chicken 7SK promoter.
SEQ ID NOs:26 to 51 - Oligonucleotide primers.
SEQ ID NO:52 to 54 - Nucleotide sequences of nucleic acid molecules that target Influenza A genes and/or the mRNA encoded thereby.
SEQ ID NOs:55 to 60 - Oligonucleotide primers
SEQ ID NO:61 - Nucleotide sequence of MWH4.
SEQ ID NO:62 - Nucleotide sequence of MWH3 and Tol2 transposon.
SEQ ID NO:63 - Nucleotide sequence of MWH4 and Tol2 transposon.

### DETAILED DESCRIPTION OF THE INVENTION

### General Techniques and Selected Definitions

Unless specifically defined otherwise, all technical and scientific terms used herein shall be taken to have the same meaning as commonly understood by one of ordinary skill in the art (e.g., in cell culture, molecular genetics, virology, immunology, immunohistochemistry, protein chemistry, and biochemistry).

Unless otherwise indicated, the molecular biology, virology, cell culture, and immunological techniques utilized in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present), and are incorporated herein by reference.

As used herein the terms "treating", "treat" or "treatment" include administering a therapeutically effective amount of a nucleic acid construct, vector, cell and/or nucleic acid molecule of the invention sufficient to reduce or eliminate at least one symptom of an influenza A virus, especially avian influenza virus, infection.

The term "preventing" refers to protecting a subject that is exposed to influenza A virus from developing at least one symptom of an influenza A virus infection, or reducing the severity of a symptom of infection in a subject exposed to influenza A virus.

As used herein, an animal that is "resistant" to a viral pathogen exhibits reduced or no symptoms of disease compared to a susceptible animal when exposed to the viral pathogen, for example when exposed to influenza virus.

The term "avian" as used herein refers to any species, subspecies or race of organism of the taxonomic Class *Aves,* such as, but not limited to, such organisms as chicken, turkey, duck, goose, quail, pheasants, parrots, finches, hawks, crows and ratites including ostrich, emu and cassowary. The term includes the various known strains of *Gallus gallus* (chickens), for example, White Leghorn, Brown Leghorn, Barred-Rock, Sussex, New Hampshire, Rhode Island, Australorp, Cornish, Minorca, Amrox, California Gray, Italian Partidge-coloured, as well as strains of turkeys, pheasants, quails, duck, ostriches and other poultry commonly bred in commercial quantities.

The term "poultry" includes all avians kept, harvested, or domesticated for meat or eggs, for example chicken, turkey, ostrich, game hen, squab, guinea fowl, pheasant, quail, duck, goose, and emu.

As used herein, "avian influenza virus" refers to any influenza A virus that may infect birds. Examples of avian influenza viruses include, but are not limited to, any one or more of subtypes H1 to H16, and N1 to N9, and include highly pathogenic and low pathogenic strains. In one embodiment, the avian influenza virus is of the H5 subtype. In another embodiment, the avian influenza virus is of the H7 subtype. In another embodiment, the avian influenza virus is of the H5N1 subtype.

The term "influenza A virus polypeptide" refers to any protein that is encoded by an influenza A virus gene, for example, PB1, PB1-F2, PB2, polymerase PA (PA), haemagglutinin (HA), nucleocapsid protein (NP), neuraminidase (NA), matrix protein 1 (M1), matrix protein 2 (M2), non-structural protein 1 (NS1) and non-structural protein 2 (NS2).

"Virus replication" as used herein refers to the amplification of the viral genome in a host cell.

The term "virus particle" as used herein is to be understood as relating to the entire virus structure, comprising nucleic acid surrounded by a protein shell or capsid. Some particles of virus also include a glycoprotein envelope surrounding the protein shell, in which case the term virus particle also includes the virus envelope. An "infectious virus particle" is capable of entering and replicating in a cell of an organism.

By "reduces the expression of" or "reducing the expression of" a polypeptide or gene is meant that the translation of a polypeptide sequence and/or transcription of a nucleotide sequence in a host cell is down-regulated or inhibited. The degree of down-regulation or inhibition will vary with the nature and quantity of the nucleic acid construct or nucleic acid molecule provided to the host cell, the identity, nature, and level of RNA molecule(s) expressed from the construct, the time after administration, etc., but will be evident e.g., as a detectable decrease in target gene protein expression and/or related target or cellular function, or e.g., decrease in level of viral replication, etc.; desirably a degree of inhibition greater than 10%, 33%, 50%, 75%, 90%, 95% or 99% as compared to a cell not treated according to the present invention will be achieved.

As used herein, the term "subject" refers to an animal, e.g., a bird or mammal. In one embodiment, the subject is a human. In other embodiments, the subject may be avian, for example poultry such as a chicken, turkey or a duck.

The "sample" refers to a material suspected of containing the nucleic acid constructs, nucleic molecules, vectors, and/or cells of the invention. The sample can be used as obtained directly from the source or following at least one step of (partial) purification. The sample can be prepared in any convenient medium which does not interfere with the method of the invention. Typically, the sample is an aqueous solution or biological fluid as described in more detail below. The sample can be derived from any source, such as a physiological fluid, including blood, serum, plasma, saliva, sputum, ocular lens fluid, sweat, faeces, urine, milk, ascites fluid, mucous, synovial fluid, peritoneal fluid, transdermal exudates, pharyngeal exudates, bronchoalveolar lavage, tracheal aspirations, cerebrospinal fluid, semen, cervical mucus, vaginal or urethral secretions, amniotic fluid, and the like. In one embodiment, the sample is blood or a fraction thereof. Pretreatment may involve, for example, preparing plasma from blood, diluting viscous fluids, and the like. Methods of treatment can involve filtration, distillation, separation, concentration, inactivation of interfering components, and the addition of reagents. The selection and pretreatment of biological samples prior to testing is well known in the art and need not be described further.

As used herein, the term "transposon" refers to a genetic element that can move (transpose) from one position to another within the genome of an organism by processes which do not require extensive DNA sequence homology between the transposon and the site of insertion nor the recombination enzymes need for classical homologous crossing over.

As used herein, "isogenic" refers to organisms or cells that are characterised by essentially identical genomic DNA, for example the genomic DNA is at least about 92%, preferably at least about 98%, and most preferably at least about 99%, identical to the genomic DNA of an isogenic organism or cell.

As used herein, the term " introducing" as it relates to a nucleic acid construct or nucleic acid molecule is to be taken in the broadest possible sense and include any method resulting in the nucleic acid construct or nucleic acid molecule being present in a cell or organism. For example, the nucleic acid construct or nucleic acid molecule may be delivered to a cell as naked DNA via any suitable transfection or transformation technique such as, for example, electroporation. Alternatively, the nucleic acid construct or nucleic acid molecule may be inserted into the genome and/or be expressed by a transgene in a cell.

### RNA Interference

The terms "RNA interference", "RNAi" or "gene silencing" refer generally to a process in which a double-stranded RNA molecule reduces the expression of a nucleic acid sequence with which the double-stranded RNA molecule shares substantial or total homology. However, it has more recently been shown that RNA interference can be achieved using non-RNA double stranded molecules (see, for example, US 20070004667).

The present invention includes nucleic acid molecules comprising and/or encoding double-stranded regions for RNA interference. The nucleic acid molecules are typically RNA but may comprise chemically-modified nucleotides and non-nucleotides.

The double-stranded regions should be at least 19 contiguous nucleotides, for example about 19 to 23 nucleotides, or may be longer, for example 30 or 50 nucleotides, or 100 nucleotides or more. The full-length sequence corresponding to the entire gene transcript may be used. Preferably, they are about 19 to about 23 nucleotides in length.

The degree of identity of a double-stranded region of a nucleic acid molecule to the targeted transcript should be at least 90% and more preferably 95-100%. The nucleic acid molecule may of course comprise unrelated sequences which may function to stabilize the molecule.

The term "short interfering RNA" or "siRNA" as used herein refers to a nucleic acid molecule which comprises ribonucleotides capable of inhibiting or down regulating gene expression, for example by mediating RNAi in a sequence-specific manner, wherein the double stranded portion is less than 50 nucleotides in length, preferably about 19 to about 23 nucleotides in length. For example the siRNA can be a nucleic acid molecule comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof. The siRNA can be assembled from two separate oligonucleotides, where one strand is the sense strand and the other is the antisense strand, wherein the antisense and sense strands are self-complementary.

As used herein, the term siRNA is meant to be equivalent to other terms used to describe nucleic acid molecules that are capable of mediating sequence specific RNAi, for example micro-RNA (miRNA), short hairpin RNA (shRNA), short interfering oligonucleotide, short interfering nucleic acid (siNA), short interfering modified oligonucleotide, chemically-modified siRNA, post-transcriptional gene silencing RNA (ptgsRNA), and others. In addition, as used herein, the term RNAi is meant to be equivalent to other terms used to describe sequence specific RNA interference, such as post transcriptional gene silencing, translational inhibition, or epigenetics. For example, siRNA molecules of the invention can be used to epigenetically silence genes at both the post-transcriptional level or the pre-transcriptional level. In a non-limiting example, epigenetic regulation of gene expression by siRNA molecules of the invention can result from siRNA mediated modification of chromatin structure to alter gene expression.

By "shRNA" or "short-hairpin RNA" is meant an RNA molecule where less than about 50 nucleotides, preferably about 19 to about 23 nucleotides, is base paired with a complementary sequence located on the same RNA molecule, and where said sequence and complementary sequence are separated by an unpaired region of at least about 4 to about 15 nucleotides which forms a single-stranded loop above the stem structure created by the two regions of base complementarity. An Example of a sequence of a single-stranded loop includes: 5' UUCAAGAGA 3'.

Included shRNAs are dual or bi-finger and multi-finger hairpin dsRNAs, in which the RNA molecule comprises two or more of such stem-loop structures separated by single-stranded spacer regions.

Once designed, the nucleic acid molecules comprising a double-stranded region can be generated by any method known in the art, for example, by *in vitro* transcription, recombinantly, or by synthetic means.

Modifications or analogs of nucleotides can be introduced to improve the properties of the nucleic acid molecules of the invention. Improved properties include increased nuclease resistance and/or increased ability to permeate cell membranes. Accordingly, the terms "nucleic acid molecule" and "double-stranded RNA molecule" includes synthetically modified bases such as, but not limited to, inosine, xanthine, hypoxanthine, 2-aminoadenine, 6-methyl-, 2-propyl- and other alkyl- adenines, 5-halo uracil, 5-halo cytosine, 6-aza cytosine and 6-aza thymine, pseudo uracil, 4-thiuracil, 8-halo adenine, 8-aminoadenine, 8-thiol adenine, 8-thiolalkyl adenines, 8-hydroxyl adenine and other 8-substituted adenines, 8-halo guanines, 8-amino guanine, 8-thiol guanine, 8-thioalkyl guanines, 8-hydroxyl guanine and other substituted guanines, other aza and deaza adenines, other aza and deaza guanines, 5-trifluoromethyl uracil and 5-trifluoro cytosine.

### Nucleic Acids

By "isolated nucleic acid molecule" we mean a nucleic acid molecule which has generally been separated from the nucleotide sequences with which it is associated or linked in its native state (if it exists in nature at all). Preferably, the isolated nucleic acid molecule is at least 60% free, more preferably at least 75% free, and more preferably at least 90% free from other components with which it is naturally associated. Furthermore, the term "nucleic acid molecule" is used interchangeably herein with the term "polynucleotide".

The term "exogenous" in the context of a nucleic acid refers to the nucleic acid (including a nucleic acid construct of the invention) when present in a cell, or in a cell-free expression system, in an altered amount compared to its native state. In a particularly preferred embodiment, the cell is a cell that does not naturally comprise the nucleic acid or nucleic acid construct.

The terms "nucleic acid molecule" or "polynucleotide" refer to an oligonucleotide, polynucleotide or any fragment thereof. It may be DNA or RNA of genomic or synthetic origin, and combined with carbohydrate, lipids, protein, or other materials to perform a particular activity defined herein.

The % identity of a nucleic acid molecule is determined by GAP (Needleman and Wunsch, 1970) analysis (GCG program) with a gap creation penalty=5, and a gap extension penalty=0.3. The query sequence is at least 19 nucleotides in length, and the GAP analysis aligns the two sequences over a region of at least 19 nucleotides. Alternatively, the query sequence is at least 150 nucleotides in length, and the GAP analysis aligns the two sequences over a region of at least 150 nucleotides. Alternatively, the query sequence is at least 300 nucleotides in length and the GAP analysis aligns the two sequences over a region of at least 300 nucleotides. Preferably, the two sequences are aligned over their entire length.

With regard to the defined nucleic acid molecules, it will be appreciated that % identity figures higher than those provided above will encompass preferred embodiments. Thus, where applicable, in light of the minimum % identity figures, it is preferred that the nucleic acid molecule comprises a nucleotide sequence which is at least 90%, more preferably at least 91 %, more preferably at least 92%, more preferably at least 93%, more preferably at least 94%, more preferably at least 95%, more preferably at least 96%, more preferably at least 97%, more preferably at least 98%, more preferably at least 99%, more preferably at least 99.1 %, more preferably at least 99.2%, more preferably at least 99.3%, more preferably at least 99.4%, more preferably at least 99.5%, more preferably at least 99.6%, more preferably at least 99.7%, more preferably at least 99.8%, and even more preferably at least 99.9% identical to the relevant nominated SEQ ID NO.

A nucleic acid molecule of the present invention may selectively hybridise to a polynucleotide that encodes an influenza A virus polypeptide under stringent conditions. As used herein, under stringent conditions are those that (1) employ low ionic strength and high temperature for washing, for example, 0.015 M NaCl/0.0015 M sodium citrate/0.1% NaDodSO4 at 50°C; (2) employ during hybridisation a denaturing agent such as formamide, for example, 50% (vol/vol) formamide with 0.1% bovine serum albumin, 0.1% Ficoll, 0.1% polyvinylpyrrolidone, 50 mM sodium phosphate buffer at pH 6.5 with 750 mM NaCl, 75 mM sodium citrate at 42°C; or (3) employ 50% formamide, 5 x SSC (0.75 M NaCl, 0.075 M sodium citrate), 50 mM sodium phosphate (pH 6.8), 0.1 % sodium pyrophosphate, 5 x Denhardt's solution, sonicated salmon sperm DNA (50 g/ml), 0.1% SDS and 10% dextran sulfate at 42°C in 0.2 x SSC and 0.1% SDS.

Nucleic acid molecules of the present invention may possess, when compared to naturally occurring molecules, regions (for example naturally occurring promoters) which have one or more mutations which are deletions, insertions, or substitutions of nucleotide residues. Mutants can be either naturally occurring (that is to say, isolated from a natural source) or synthetic (for example, by performing site-directed mutagenesis on the nucleic acid as described above). It is thus apparent that polynucleotides of the invention can be either naturally occurring or recombinant.

Usually, monomers of a nucleic acid are linked by phosphodiester bonds or analogs thereof to form oligonucleotides ranging in size from a relatively short monomeric units, e.g., 12-18, to several hundreds of monomeric units. Analogs of phosphodiester linkages include: phosphorothioate, phosphorodithioate, phosphoroselenoate, phosphorodiselenoate, phosphoroanilothioate, phosphoranilidate, phosphoramidate.

### Nucleic Acid Constructs

As used herein, "nucleic acid construct" refers to any nucleic acid molecule that encodes a double-stranded RNA molecule as defined herein and includes the nucleic acid molecule in a vector, the nucleic acid molecule when present in a cell as an extrachromosomal nucleic acid molecule, and a nucleic acid molecule that is integrated into the genome. Typically, the nucleic acid construct will be double stranded DNA or double-stranded RNA, or a combination thereof. Furthermore, the nucleic acid construct will typically comprise a suitable promoter operably linked to the open reading frame encoding the double-stranded RNA. The nucleic acid construct may comprise a first open reading frame encoding a first single strand of the double-stranded RNA molecule, with the complementary (second) strand being encoded by a second open reading frame by a different, or preferably the same, nucleic acid construct. The nucleic acid construct may be a linear fragment or a circular molecule and it may or may not be capable of replication. The skilled person will understand that the nucleic acid construct of the invention may be included within a suitable vector. Transfection or transformation of the nucleic acid construct into a recipient cell allows the cell to express an RNA molecule encoded by the nucleic acid construct.

The nucleic acid construct of the invention may express multiple copies of the same, and/or one or more (e.g. 1, 2, 3, 4, 5, or more) including multiple different, RNA molecules comprising a double-stranded region, for example a short hairpin RNA. RNA molecules considered to be the "same" as each other are those that comprise only the same double-stranded sequence, and RNA molecules considered to be "different" from each other will comprise different double-stranded sequences, regardless of whether the sequences to be targeted by each different double-stranded sequence are within the same, or a different gene, or sequences of two different genes.

The nucleic acid construct also may contain additional genetic elements. The types of elements that may be included in the construct are not limited in any way and may be chosen by one with skill in the art. In some embodiments, the nucleic acid construct is inserted into a host cell as a transgene. In such instances it may be desirable to include "stuffer" fragments in the construct which are designed to protect the sequences encoding the RNA molecule from the transgene insertion process and to reduce the risk of external transcription read through. Stuffer fragments may also be included in the construct to increase the distance between, e.g., a promoter and a coding sequence and/or terminator component. The stuffer fragment can be any length from 5-5000 or more nucleotides. There can be one or more stuffer fragments between promoters. In the case of multiple stuffer fragments, they can be the same or different lengths. The stuffer DNA fragments are preferably different sequences. Preferably, the stuffer sequences comprise a sequence identical to that found within a cell, or progeny thereof, in which they have been inserted. In a further embodiment, the nucleic acid construct comprises stuffer regions flanking the open reading frame(s) encoding the double stranded RNA(s).

Alternatively, the nucleic acid construct may include a transposable element, for example a transposon characterized by terminal inverted repeat sequences flanking the open reading frames encoding the double stranded RNA(s). Examples of suitable transposons include Tol2, mini-Tol, Sleeping Beauty, Mariner and Galluhop.

Other examples of an additional genetic element which may be included in the nucleic acid construct include a reporter gene, such as one or more genes for a fluorescent marker protein such as GFP or RFP; an easily assayed enzyme such as beta-galactosidase, luciferase, beta-glucuronidase, chloramphenical acetyl transferase or secreted embryonic alkaline phosphatase; or proteins for which immunoassays are readily available such as hormones or cytokines. Other genetic elements that may find use in embodiments of the present invention include those coding for proteins which confer a selective growth advantage on cells such as adenosine deaminase, aminoglycodic phosphotransferase, dihydrofolate reductase, hygromycin-B-phosphotransferase, or drug resistance.

Where the nucleic acid construct is to be transfected into an animal, it is desirable that the promoter and any additional genetic elements consist of nucleotide sequences that naturally occur in the animal's genome. It is further desirable that the sequences encoding RNA molecules consist of influenza A virus sequences.

### Promoters

As used herein "promoter" refers to a nucleic acid sequence which is able to direct transcription of an operably linked nucleic acid molecule and includes, for example, RNA polymerase II and RNA polymerase III promoters. Also included in this definition are those transcriptional regulatory elements (e.g., enhancers) that are sufficient to render promoter-dependent gene expression controllable in a cell type-specific, tissue-specific, or temporal-specific manner, or that are inducible by external agents or signals.

When a nucleic acid construct comprising a promoter is transfected into a host animal, it is desirable that the promoter is one that naturally occurs in the animal's genome. For example, where the transgenic animal is a chicken, the promoter is preferably a chicken promoter; wherein the transgenic animal is a turkey, the promoter is preferably a turkey promoter; and wherein the transgenic animal is a duck, the promoter is preferably a duck promoter.

"Operably linked" as used herein refers to a functional relationship between two or more nucleic acid (e.g., DNA) segments. Typically, it refers to the functional relationship of a transcriptional regulatory element to a transcribed sequence. For example, a promoter is operably linked to a coding sequence, such as an open reading frame encoding a double-stranded RNA molecule defined herein, if it stimulates or modulates the transcription of the coding sequence in an appropriate cell. Generally, promoter transcriptional regulatory elements that are operably linked to a transcribed sequence are physically contiguous to the transcribed sequence, i.e., they are *cis*-acting. However, some transcriptional regulatory elements, such as enhancers, need not be physically contiguous or located in close proximity to the coding sequences whose transcription they enhance.

By "RNA polymerase III promoter" or "RNA pol III promoter" or "polymerase III promoter" or "pol III promoter" is meant any invertebrate, vertebrate, or mammalian promoter, e.g., chicken, human, murine, porcine, bovine, primate, simian, etc. that, in its native context in a cell, associates or interacts with RNA polymerase III to transcribe its operably linked gene, or any variant thereof, natural or engineered, that will interact in a selected host cell with an RNA polymerase III to transcribe an operably linked nucleic acid sequence. By U6 promoter (e.g., chicken U6, human U6, murine U6), H1 promoter, or 7SK promoter is meant any invertebrate, vertebrate, or mammalian promoter or polymorphic variant or mutant found in nature to interact with RNA polymerase III to transcribe its cognate RNA product, i.e., U6 RNA, H1 RNA, or 7SK RNA, respectively. Examples of suitable promoters include cU6-1 (SEQ ID NO:22), cU6-3 (SEQ ID NO:23), cU6-4 (SEQ ID NO:24) and c7SK (SEQ ID NO:25).

Preferred in some applications are the Type III RNA pol III promoters including U6, H1, and 7SK which exist in the 5' flanking region, include TATA boxes, and lack internal promoter sequences. Internal promoters occur for the pol III 5S rRNA, tRNA or VA RNA genes. The 7SK RNA pol III gene contains a weak internal promoter and a sequence in the 5' flanking region of the gene necessary for transcription. Pol III promoters for utilization in a nucleic acid construct for a particular application, e.g., to express double stranded RNA molecules such as hairpin RNAs against an avian or human virus may advantageously be selected for optimal binding and transcription by the host cell RNA polymerase III, e.g., including avian pol III promoters in an expression construct designed to transcribe a plurality of hairpin dsRNAs against an avian virus such as avian influenza virus (H5N1) in avian host cells.

By "different" polymerase promoters is meant any two RNA polymerase promoters, such as RNA polymerase II or RNA polymerase III promoters, including variants such as polymorphisms and mutants thereof, which in a particular species will drive transcription of different cognate transcripts, such as, e.g., the human 7SK promoter, the human U6 promoter, and the human H1 promoter, which are considered three "different" polymerase promoters. "Different" polymerase promoters also refers to individual members of a family of promoters such as, e.g., the chicken U6 family of promoters in which the cU6-1, cU6-2, cU6-3 and cU6-4 promoters are considered "different" promoters. The use of different polymerase promoters in the constructs of the present invention will reduce the possibility of intra- and/or intermolecular recombination events such as rearrangements or deletions.

In some aspects, multiple copies of the "same" RNA polymerase II or RNA polymerase III promoter may be included in a nucleic acid construct of the invention. In some embodiments, the nucleic acid constructs of the invention may contain multiple copies of the same polymerase promoter without a "different" polymerase promoter; e.g., three, four, five, or more U6 promoters each operably linked to a sequence encoding a RNA molecule such as a shRNA. Optionally, in some embodiments, other promoters may be included in addition to the two or more polymerase promoters, e.g., one or more polymerase I promoters, one or more mitochondrial promoters, etc. In one aspect, an expression construct comprising multiple polymerase promoters (2, 3, 4, 5, or more) is engineered to express multiple dsRNA hairpins or shRNAs, in which case 2, 3, 4, 5, or more copies of the same polymerase promoter may be used, irrespective of whether or not a "different" RNA polymerase promoter is also included.

In some instances it may also be desirable that the nucleic acid construct comprise a tissue-specific or cell-specific promoter. The term "tissue specific" as it applies to a promoter refers to a promoter that is capable of directing selective expression of a nucleotide sequence of interest to a specific type of tissue (e.g., lungs) in the relative absence of expression of the same nucleotide sequence of interest in a different type of tissue (e.g., brain). Such tissue specific promoters include promoters such as Ick, myogenin, or thyl. The term "cell-specific" as applied to a promoter refers to a promoter which is capable of directing selective expression of a nucleotide sequence of interest in a specific type of cell in the relative absence of expression of the same nucleotide sequence of interest in a different type of cell within the same tissue (see, e.g., Higashibata, et al. (2004); Hoggatt, et al. (2002); Sohal, et al., (2001); and Zhang, et al., (2004)). The term "cell-specific" when applied to a promoter also means a promoter capable of promoting selective expression of a nucleotide sequence of interest in a region within a single tissue. Alternatively, promoters may be constitutive or regulatable. Additionally, promoters may be modified so as to possess different specificities.

### Amplification of Nucleic Acid

The "polymerase chain reaction" ("PCR") is a reaction in which replicate copies are made of a target polynucleotide using a "pair of primers" or "set of primers" consisting of "upstream" and a "downstream" primer, and a catalyst of polymerization, such as a DNA polymerase, and typically a thermally-stable polymerase enzyme. Methods for PCR are known in the art, and are taught, for example, in "PCR" (Ed. M.J. McPherson and S.G Moller (2000) BIOS Scientific Publishers Ltd, Oxford). PCR can be performed on cDNA obtained from reverse transcribing mRNA isolated from biological samples.

A primer is often an oligonucleotide, generally of about 20 nucleotides long, with a minimum of about 15 nucleotides, that is capable of hybridising in a sequence specific fashion to the target sequence and being extended during the PCR. Longer nucleic acid moleucules, for example nucleic acid molecules at least 50 or 100 or more nucleotides in length may also be used as a primer. Amplicons or PCR products or PCR fragments or amplification products are extension products that comprise the primer and the newly synthesized copies of the target sequences. Multiplex PCR systems contain multiple sets of primers that result in simultaneous production of more than one amplicon. Primers may be perfectly matched to the target sequence or they may contain internal mismatched bases that can result in the introduction of restriction enzyme or catalytic nucleic acid recognition/cleavage sites in specific target sequences. Primers may also contain additional sequences and/or modified or labelled nucleotides to facilitate capture or detection of amplicons. Repeated cycles of heat denaturation of the DNA, annealing of primers to their complementary sequences and extension of the annealed primers with polymerase result in exponential amplification of the target sequence. The terms target or target sequence or template refer to nucleic acid sequences which are amplified.

Another nucleic acid amplification technique is reverse transcription polymerase chain reaction (RT-PCR). First, complementary DNA (cDNA) is made from an RNA template, using a reverse transcriptase enzyme, and then PCR is performed on the resultant cDNA.

Another method for amplification is the ligase chain reaction ("LCR"), disclosed in EP 0 320 308. In LCR, two complementary probe pairs are prepared, and in the presence of the target sequence, each pair will bind to opposite complementary strands of the target such that they abut. In the presence of a ligase, the two probe pairs will link to form a single unit. By temperature cycling, as in PCR, bound ligated units dissociate from the target and then serve as "target sequences" for ligation of excess probe pairs. U.S. Pat. No. 4,883,750 describes a method similar to LCR for binding probe pairs to a target sequence.

Other methods for amplification of nucleic acid molecules are known to those skilled in the art and include isothermal amplification methods and transcription-based amplification systems. Any suitable method for amplifying a nucleic acid construct, or fragment thereof, or an isolated or exogenous nucleic acid molecule, or a fragment thereof, may be used in the methods of the present invention.

### Vectors and Host Cells

In some instances it may be desirable to insert the nucleic acid construct and/or nucleic acid molecule of the invention into a vector. The vector may be, e.g., a plasmid, virus or artificial chromosome derived from, for example, a bacteriophage, adenovirus, adeno-associated virus, retrovirus, poxvirus or herpesvirus. Such vectors include chromosomal, episomal and virus-derived vectors, e.g., vectors derived from bacterial plasmids, bacteriophages, yeast episomes, yeast chromosomal elements, and viruses, vectors derived from combinations thereof, such as those derived from plasmid and bacteriophage genetic elements, cosmids and phagemids. Thus, one exemplary vector is a double-stranded DNA phage vector. Another exemplary vector is a double-stranded DNA viral vector.

The vector into which the nucleic acid construct is inserted may also include a transposable element, for example a transposon characterized by terminal inverted repeat sequences flanking the open reading frames encoding the double stranded RNA(s). Examples of suitable transposons include Tol2, Mini-Tol2, Sleeping Beauty, Mariner and Galluhop. Reference to a Tol2 tansposon herein includes a transposon derived from Tol2 such as Mini-Tol2.

The present invention also provides a host cell into which the nucleic acid construct, nucleic acid molecule and/or the vector of the present invention has been introduced. The host cell of this invention can be used as, for example, a production system for producing or expressing the dsRNA molecule. For *in vitro* production, eukaryotic cells or prokaryotic cells can be used.

Useful eukaryotic host cells may be animal, plant, or fungal cells. As animal cells, mammalian cells such as CHO, COS, 3T3, DF1, CEF, MDCK myeloma, baby hamster kidney (BHK), HeLa, or Vero cells, amphibian cells such as *Xenopus* oocytes, or insect cells such as Sf9, Sf21, or Tn5 cells can be used. CHO cells lacking DHFR gene (dhfr-CHO) or CHO K-1 may also be used. The vector can be introduced into the host cell by, for example, the calcium phosphate method, the DEAE-dextran method, cationic liposome DOTAP (Boehringer Mannheim) method, electroporation, lipofection, etc.

Useful prokaryotic cells include bacterial cells, such as *E. coli,* for example, JM109, DH5a, and HB101, or *Bacillus subtilis.*

Culture medium such as DMEM, MEM, RPM11640, or IMDM may be used for animal cells. The culture medium can be used with or without serum supplement such as fetal calf serum (FCS). The pH of the culture medium is preferably between about 6 and 8. Cells are typically cultured at about 30° to 40° C for about 15 to 200 hr, and the culture medium may be replaced, aerated, or stirred if necessary.

### Transgenic Non-Human Animals

A "transgenic non-human animal" refers to an animal, other than a human, that contains a nucleic acid construct ("transgene") not found in a wild-type animal of the same species or breed. A "transgene" as referred to herein has the normal meaning in the art of biotechnology and includes a genetic sequence which has been produced or altered by recombinant DNA or RNA technology and which has been introduced into an animal, preferably avian, cell. The transgene may include genetic sequences derived from an animal cell. Typically, the transgene has been introduced into the animal by human manipulation such as, for example, by transformation but any method can be used as one of skill in the art recognizes. A transgene includes genetic sequences that are introduced into a chromosome as well as those that are extrachromosomal.

Techniques for producing transgenic animals are well known in the art. A useful general textbook on this subject is Houdebine, Transgenic animals - Generation and Use (Harwood Academic, 1997).

Heterologous DNA can be introduced, for example, into fertilized ova. For instance, totipotent or pluripotent stem cells can be transformed by microinjection, calcium phosphate mediated precipitation, liposome fusion, retroviral infection or other means, the transformed cells are then introduced into the embryo, and the embryo then develops into a transgenic animal. In one method, developing embryos are infected with a retrovirus containing the desired DNA, and transgenic animals produced from the infected embryo. In an alternative method, however, the appropriate DNAs are coinjected into the pronucleus or cytoplasm of embryos, preferably at the single cell stage, and the embryos allowed to develop into mature transgenic animals.

Another method used to produce a transgenic animal involves microinjecting a nucleic acid into pro-nuclear stage eggs by standard methods. Injected eggs are then cultured before transfer into the oviducts of pseudopregnant recipients.

Transgenic animals may also be produced by nuclear transfer technology. Using this method, fibroblasts from donor animals are stably transfected with a plasmid incorporating the coding sequences for a binding domain or binding partner of interest under the control of regulatory sequences. Stable transfectants are then fused to enucleated oocytes, cultured and transferred into female recipients.

Sperm-mediated gene transfer (SMGT) is another method that may be used to generate transgenic animals. This method was first described by Lavitrano et al. (1989).

Another method of producing transgenic animals is linker based sperm-mediated gene transfer technology (LB-SMGT). This procedure is described in United States Patent 7067308. Briefly, freshly harvested semen is washed and incubated with murine monoclonal antibody mAbC (secreted by the hybridoma assigned ATCC accession number PTA-6723) and then the construct DNA. The monoclonal antibody aids in the binding of the DNA to the semen. The sperm/DNA complex is then artificially inseminated into a female.

Germline transgenic chickens may be produced by injecting replication-defective retrovirus into the subgerminal cavity of chick blastoderms in freshly laid eggs (U.S. Pat. No. 5,162,215; Bosselman et al., 1989; Thoraval et al., 1995). The retroviral nucleic acid carrying a foreign gene randomly inserts into a chromosome of the embryonic cells, generating transgenic animals, some of which bear the transgene in their germ line. Use of insulator elements inserted at the 5' or 3' region of the fused gene construct to overcome position effects at the site of insertion has been described (Chim et al., 1993).

Another method for generating germline transgenic animals is by using a transposon, for example the Tol2 transposon, to integrate a nucleic acid construct of the invention into the genome of an animal. The Tol2 transposon which was first isolated from the medaka fish *Oryzias latipes* and belongs to the hAT family of transposons is described in Koga et al. (1996) and Kawakami et al. (2000). Mini-Tol2 is a variant of Tol2 and is described in Balciunas et al. (2006). The Tol2 and Mini-Tol2 transposons facilitate integration of a transgene into the genome of an organism when co-acting with the Tol2 transposase. By delivering the Tol2 transposase on a separate non-replicating plasmid, only the Tol2 or Mini-Tol2 transposon and transgene is integrated into the genome and the plasmid containing the Tol2 transposase is lost within a limited number of cell divisions. Thus, an integrated Tol2 or Mini-Tol2 transposon will no longer have the ability to undergo a subsequent transposition event. Additionally, as Tol2 is not known to be a naturally occurring avian transposon, there is no endogenous transposase activity in an avian cell, for example a chicken cell, to cause further transposition events.

Any other suitable transposon system may be used in the methods of the present invention. For example, the transposon system may be a Sleeping Beauty, Frog Prince or Mos1 transposon system, or any transposon belonging to the tc1/mariner or hAT family of transposons may be used.

The injection of avian embryonic stem cells into recipient embryos to yield chimeric birds is described in US 7,145,057. Breeding the resulting chimera yields transgenic birds whose genome is comprised of exogenous DNA.

Methods of obtaining transgenic chickens from long-term cultures of avian primordial germ cells (PGCs) are described in US Patent Application 20060206952. When combined with a host avian embryo by known procedures, those modified PGCs are transmitted through the germline to yield transgenic offspring.

A viral delivery system based on any appropriate virus may be used to deliver the nucleic acid constructs of the present invention to a cell. In addition, hybrid viral systems may be of use. The choice of viral delivery system will depend on various parameters, such as efficiency of delivery into the cell, tissue, or organ of interest, transduction efficiency of the system, pathogenicity, immunological and toxicity concerns, and the like. It is clear that there is no single viral system that is suitable for all applications. When selecting a viral delivery system to use in the present invention, it is important to choose a system where nucleic acid construct-containing viral particles are preferably: 1) reproducibly and stably propagated; 2) able to be purified to high titers; and 3) able to mediate targeted delivery (delivery of the nucleic acid expression construct to the cell, tissue, or organ of interest, without widespread dissemination).

### Compositions and Administration

In a preferred embodiment, a composition of the invention is a pharmaceutical composition comprising a suitable carrier. Suitable pharmaceutical carriers, excipients and/or diluents include, but are not limited to, lactose, sucrose, starch powder, talc powder, cellulose esters of alkonoic acids, magnesium stearate, magnesium oxide, crystalline cellulose, methyl cellulose, carboxymethyl cellulose, gelatin, glycerin, sodium alginate, antibacterial agents, antifungal agents, gum arabic, acacia gum, sodium and calcium salts of phosphoric and sulfuric acids, polyvinylpyrrolidone and/or polyvinyl alcohol, saline, and water.

In some embodiments, the nucleic acid construct(s) and/or nucleic acid molecules of the invention are complexed with one or more cationic lipids or cationic amphiphiles, such as the compositions disclosed in US 4,897,355; US 5,264,618; or US 5,459,127. In other embodiments, they are complexed with a liposome/liposomic composition that includes a cationic lipid and optionally includes another component, such as a neutral lipid (see, for example, US 5,279,833; US 5,283,185; and US 5,932,241). In other embodiments, they are complexed with the multifunctional molecular complexes of US 5,837,533; 6,127,170; and 6,379,965 or, desirably, the multifunctional molecular complexes or oil/water cationic amphiphile emulsions of WO 03/093449. The latter application teaches a composition that includes a nucleic acid, an endosomolytic spermine that includes a cholesterol or fatty acid, and a targeting spermine that includes a ligand for a cell surface molecule. The ratio of positive to negative charge of the composition is between 01. to 2.0, preferably 0.5 and 1.5, inclusive; the endosomolytic spermine constitutes at least 20% of the spermine-containing molecules in the composition; and the targeting spermine constitutes at least 10% of the spermine-containing molecules in the composition. Desirably, the ratio of positive to negative charge is between 0.8 and 1.2, inclusive, such as between 0.8 and 0.9, inclusive.

Administration of a nucleic acid construct, nucleic acid molecule and/or composition may conveniently be achieved by injection into an avian egg, and generally injection into the air sac. Notwithstanding that the air sac is the preferred route of *in ovo* administration, other regions such as the yolk sac or chorion allantoic fluid may also be inoculated by injection. The hatchability rate might decrease slightly when the air sac is not the target for the administration although not necessarily at commercially unacceptable levels. The mechanism of injection is not critical to the practice of the present invention, although it is preferred that the needle does not cause undue damage to the egg or to the tissues and organs of the developing embryo or the extra-embryonic membranes surrounding the embryo.

Generally, a hypodermic syringe fitted with an approximately 22 gauge needle is suitable for avian *in ovo* administration. The method of the present invention is particularly well adapted for use with an automated injection system, such as those described in US 4,903,635, US 5,056,464, US 5,136,979 and US 20060075973.

In another embodiment, the nucleic acid construct, nucleic acid molecule and/or composition of the invention is administered via pulmonary delivery, such as by inhalation of an aerosol or spray dried formulation. For example, the aerosol may be administered by an inhalation device or nebulizer (see for example US 4,501,729), providing rapid local uptake of the nucleic acid molecules into relevant pulmonary tissues. Solid particulate compositions containing respirable dry particles of micronized nucleic acid compositions can be prepared by grinding dried or lyophilized nucleic acid compositions, and then passing the micronized composition through, for example, a 400 mesh screen to break up or separate out large agglomerates. A solid particulate composition comprising the nucleic acid compositions of the invention can optionally contain a dispersant which serves to facilitate the formation of an aerosol as well as other therapeutic compounds. A suitable dispersant is lactose, which can be blended with the nucleic acid compound in any suitable ratio, such as a 1 to 1 ratio by weight.

Nebulizers are commercially available devices which transform solutions or suspensions of an active ingredient into a therapeutic aerosol mist either by means of acceleration of a compressed gas, typically air or oxygen, through a narrow venturi orifice or by means of ultrasonic agitation. Suitable formulations for use in nebulizers comprise the active ingredient in a liquid carrier in an amount of up to 40% w/w preferably less than 20% w/w of the formulation. The carrier is typically water or a dilute aqueous alcoholic solution, preferably made isotonic with body fluids by the addition of, for example, sodium chloride or other suitable salts. Optional additives include preservatives if the formulation is not prepared sterile, for example, methyl hydroxybenzoate, anti-oxidants, flavorings, volatile oils, buffering agents and emulsifiers and other formulation surfactants. The aerosols of solid particles comprising the active composition and surfactant can likewise be produced with any solid particulate aerosol generator. Aerosol generators for administering solid particulate therapeutics to a subject produce particles which are respirable, as explained above, and generate a volume of aerosol containing a predetermined metered dose of a therapeutic composition at a rate suitable for human administration.

A nucleic acid construct, nucleic acid molecule and/or composition of the invention can also be added to animal feed or drinking water. It can be convenient to formulate the feed and drinking water compositions so that the animal takes in a therapeutically appropriate quantity along with its diet. It can also be convenient to present the composition as a premix for addition to the feed or drinking water.

### EXAMPLES

### Example 1. Selection of shRNA sequences for inclusion in transgenes

The most highly conserved genes, PB1, PB2, PA, NP and M1, of influenza A were selected for RNAi targeting. The inventors used siVirus (web-based antiviral siRNA design software for highly divergent viral sequences, Naito et al., 2006) to identify highly conserved regions within the selected genes and to also predict siRNA sequences that we could screen for the selection of shRNAs. The software highlighted a number of regions within the analysed Influenza A genomic segments, that were of particular interest for shRNA design, namely the 3' regions of PB1, PB2, PA and NP. More specifically these were: Segment 1 (PB2 gene) nucleotides 2240-2341; Segment 2 (PB1 gene) nucleotides 2257-2341; Segment 3 (PA gene) nucleotides 2087-2233 and; Segment 5 (NP gene) nucleotides 1484-1565.

The inventors selected 29 predicted siRNA sequences from siVirus to screen for the selection of shRNAs (Table 1). There are several algorithms available to select potential siRNA sequences for specific target genes. It has been shown however that many of these predicted siRNAs do not function effectively when processed from expressed shRNAs. Taxman et al. (2006) have specifically designed an algorithm to predict effective shRNA molecules and the present inventors have made our own modification to the algorithm to improve shRNA prediction. The present inventors applied the modified Taxman algorithm to the 29 siVirus selected siRNAs so as to choose sequences for testing as shRNAs for the specific inhibition of Influenza A virus replication.

**Table 1. Algorithm selection of shRNA sequences targeting Influenza A genes.**

| shRNA | 5' end score | ΔG central | 3' end score | A+T in 3' | Score |
|---|---|---|---|---|---|
| PB1-6 | 1 | -13.8 | 1 | 2 | 4 |
| **PB1-129** | **1** | **-12.7** | **-1** | **1** | **1** |
| **PB1-2257** | **1** | **-13.9** | **1** | **2** | **4** |
| | | | | | |
| PB2-2210 | 1 | -14.7 | 1 | 2 | 4 |
| **PB2-2240** | **1** | **-14.1** | **1** | **2** | **4** |
| PB2-8 | 1 | -11.8 | 1 | 1 | 3 |
| PB2-10 | -1 | -12.2 | 1 | 1 | 1 |
| | | | | | |
| PA-44 | -1 | -13.1 | -1 | 2 | 0 |
| PA-739 | 1 | -10.9 | -1 | 0 | 0 |
| **PA-2087** | **1** | **-13.6** | **1** | **0** | **2** |
| PA-2110 | -1 | -17.2 | 1 | 2 | 2 |
| PA-2131 | -1 | -12.6 | -1 | 0 | -2 |
| | | | | | |
| NP-224 | -1 | -15.3 | 1 | 2 | 2 |
| NP-231 | -1 | -12.8 | -1 | 0 | -2 |
| NP-344 | 1 | -13.4 | 1 | 2 | 4 |
| NP-390 | -1 | -13.5 | 1 | 0 | 0 |
| **NP-771** | **1** | **-11.4** | **1** | **1** | **3** |
| NP-778 | -1 | -13.3 | **1** | **1** | **1** |
| **NP-1472** | **1** | **-11.4** | **1** | **2** | **4** |
| **NP-1484** | **-1** | **-8.7** | **1** | **1** | **1** |
| **NP-1496** | **1** | **-8.7** | **-1** | **0** | **0** |
| | | | | | |
| MP-37 | 1 | -13.3 | 1 | 0 | 2 |
| MP-331 | 1 | -13.3 | 1 | 2 | 4 |
| MP-480 | 1 | -14 | -1 | 0 | 0 |
| **MP-554** | **1** | **-12.0** | **1** | **2** | **4** |
| **MP-592** | **1** | **-13.4** | **1** | **1** | **3** |
| MP-598 | -1 | -14.5 | 1 | 0 | 0 |
| MP-934 | 1 | -11 | -1 | 0 | 0 |
| MP-5 | 1 | -10.8 | 1 | 2 | 4 |

There are four criteria for shRNA selection using the Taxman algorithm. Three of the criteria are scored for out of a maximum number of 4 points. These criteria are: 1) C or G on the 5' end of the sequence = 1 point, A or T on 5' end = -1 point; 2) A or T on the 3' end = 1 point, C or G on the 3' end = -1 point; 3) 5 or more A or T in the seven 3' bases = 2 points, 4 A or T in the seven 3' bases = 1 point. shRNA sequences with the highest scores are preferred. The fourth criteria is based on a calculation for the free-energy of the 6 central bases of the shRNA sequence (bases 6-11 of the sense strand hybridised to bases 9-14 of the antisense strand) for a 19 nucleotide sequence. shRNAs with a central duplex ΔG > -12.9 kcal/mol are preferred. The present inventors modification to the Taxman algorithm is to use different free-energy parameters for predictions of RNA duplex stability as published by Freier et al. (1986). Based on the algorithm, the inventors chose 13 of the siVirus siRNA sequences for use in potentially effective shRNAs to test for their ability to inhibit influenza A virus replication. The selected sequences are highlighted in bold in Table 1 and their 5' - 3' sequence is shown in Table 2. These 13 sequences were used to construct ddRNAi plasmids for the expression of the 10 shRNAs.

**Table 2. Sequence of shRNAs selected for virus inhibition assays.**

| shRNA | 5' - 3' Sequence |
|---|---|
| PB1-129 | CAGGAUACACCAUGGAUAC (SEQ ID NO:6) |
| PB1-2257 | GAUCUGUUCCACCAUUGAA (SEQ ID NO:7) |
| PB2-2240 | CGGGACUCUAGCAUACUUA (SEQ ID NO:8) |
| PB2-8 | CAGCGACCAAAAGAATTCGGA (SEQ ID NO:52) |
| PB2-10 | AAGAATTCGGATGGCCATCAA (SEQ ID NO:53) |
| PA-2087 | GCAAUUGAGGAGUGCCUGA (SEQ ID NO:9) |
| NP-771 | CCAGGAAAUGCUGAGAUCGAA (SEQ ID NO:10) |
| NP-1472 | GAGUAAUGAAGGAUCUUAUUU (SEQ ID NO:11) |
| NP-1484 | AUCUUAUUUCUUCGGAGACAA (SEQ ID NO:12) |
| NP-1496 | GGAUCUUAUUUCUUCGGAG (SEQ ID NO:13) |
| MP-554 | CACUAAUCAGACAUGAGAA (SEQ ID NO:14) |
| MP-592 | CUACAGCUAAGGCUAUGGA (SEQ ID NO:15) |
| MP-5 | GTGGATTCTTGATCGTCTT (SEQ ID NO:54) |

### Example 2. Chicken Promoter Sequences

It is known that when designing a transgene construct, it is desirable to include promoters which include upstream sequence to enable efficient attachment of the polymerase enzyme to the promoter sequences. Despite this, chicken U6 promoters were designed and tested to contain the minimum amount of promoter sequence required to elicit transcription of the shRNAs, thus enabling a reduction in the overall size of the transgene construct.

Two versions of constructs, pcU6-4 shNP-1496 and pcU6-4 (+100) shNP-1496, were tested for expression of shRNA via RNAse protesction assays or for virus silencing. The first plasmid contains the minimum chicken U6-4 sequence required to express the shNP-1496 short hairpin RNA. The second plasmid contains 100 bp extra sequence upstream of the cU6-4 promoter. It was expected that the second construct containing the 100 bp extra upstream sequence would provide better expression shRNA.

Table 3 details the results of a hemagglutination assay (HA assay) experiment to measure the inhibition of virus production induced by the shRNA expression from both plasmids. To conduct this assay, MDCK cells were grown to logarithmic-phase and then electroporated with the shRNA plasmids using an Amaxa Nucleofector. The transfected cells were then infected 8 hours later with low pathogenic H1N1 A/PR/8/34 (PR8) Influenza A virus, at a range of multiplicity of infections (moi). Virus titer (HA units) was measured 48 hours after infection by performing HA assays. The assays were carried out in V-bottom 96-well plates. Serial 2-fold dilutions of virus samples were mixed with an equal volume of a 0.5% suspension (vol/vol) of chicken erythrocytes and incubated on ice for 1 hour. Wells containing an adherent, homogenous layer of erythrocytes were scored as positive.

In the HA assay experiment, pcU6-4 shNP-1496 containing the minimal promoter sequence was more effective at silencing the virus at all tested MOI than pcU6-4 (+100) shNP-1496 which contained extra upstream promoter sequence and the mock plasmid.

**Table 3. Results of hemagglutination assay (HA assay)**

| | MOI.001 | MOI.0001 | MOI.00001 |
|---|---|---|---|
| Mock plasmid | 32 | 32 | 16 |
| pcU6-4 (+100) shNP-1496 | 32 | 16 | 4 |
| pcU6-4 shNP-1496 | 4 | 2 | 2 |

### Example 3. Construction of ddRNAi plasmids for expression of selected shRNAs

The chicken polymerase III promoters cU6-1 (GenBank accession number DQ531567), cU6-3 (DQ531569), cU6-4 (DQ531570) and c7SK (EF488955) were used as templates to construct ddRNAi expression plasmids for the selected shRNAs, via a one-step PCR (Figure 1). PCR for the construction of the plasmids used primer TD135 paired with TD218 or TD275 for the cU6-1 promoter; TD175 paired with TD216, TD274 or TD302 for the cU6-4 promoter; TD176 paired with TD217 for the cU6-3 promoter and; TD269 paired with TD307 or TD316 for the c7SK promoter (see Table 4 for primer sequence and details of the specific shRNA amplified). The reverse primers in each PCR were designed to comprise the last 20 nt of each promoter sequence, shRNA sense, loop, and shRNA antisense sequence and were HPLC purified. Full-length amplified expression cassette products were ligated into pGEM-T Easy and then sequenced.

Of the chosen 13 shRNAs, expression plasmids were successfully constructed for 7 of the sequences. The final shRNA expression plasmids used in virus inhibition assays were named pcU6-1-shPB2-2240, pcU6-1-shPA-2087, pcU6-3-shMP-592, pcU6-4-shNP-1496, pcU6-4-shNP-1484, pc7SK-shPB1-129, pcU6-4-shPB1-2257 and pc7SK-shPB1-2257. A cU6-1 irrelevant control plasmid was also constructed and used for mock comparison in virus inhibition assays (see below). For this mock plasmid, forward primer TD135 was paired with reverse primer TD155 comprising the last 20 nt of the chU6-1 promoter and all other irrelevant shRNA (shirr) components. The PCR product was ligated into pGEM-T Easy and sequenced.

Each ddRNAi plasmid was constructed so that the start of each shRNA sequence was at the +1 position of the native U6 or 7SK snRNA transcripts. A *Xho*I restriction enzyme site was engineered downstream of the termination signal to allow screening for full-length shRNA products inserted into pGEM-T Easy. All final shRNA expression vectors consisted of either one of the full length chicken U6 or 7SK promoters, a shRNA sense sequence, a loop sequence, a shRNA antisense sequence, a termination sequence and a *Xho*I site. The loop sequence used in all shRNAs was 5' UUCAAGAGA 3'.

### Example 4. Testing selected shRNAs for virus inhibition

Table 5 summarises the results of hemagglutination assay (HA assay) experiments to measure the inhibition of virus production induced by the shRNA expression plasmids. To conduct these assays, MDCK cells were grown to logarithmic phase and then electroporated with the shRNA plasmids using an Amaxa Nucleofector™. The transfected cells were then infected 8 hours later with Influenza A virus, either low pathogenic H1N1 A/PR/8/34 (PR8) or highly pathogenic H5N1 A/chicken/Vietnam/008/2004 (H5N1), at a range of multiplicity of infections (moi). Virus titer (HA units) was measured 48 hours after infection by performing HA assays. The assays were carried out in V-bottom 96-well plates. Serial 2-fold dilutions of virus samples were mixed with an equal volume of a 0.5% suspension (vol/vol) of chicken erythrocytes and incubated on ice for 1 hour. Wells containing an adherent, homogenous layer of erythrocytes were scored as positive.

**Table 4. Sequence and details of primers used.**

| **Name** | **Sequence 5' - 3'** | **Location/ Feature** |
|---|---|---|
| TD135 | CGAAGAACCGAGCGCTGC (SEQ ID NO:26) | cU6-1 |
| TD155 | GGGCTCGAGTTCCAAAAAAGCGCAGTGTTACTCCACTTCTCTTGAAAGTGGAGTAACACTGCGCTGAA TACCGCTTCCTCCTGAG (SEQ ID NO:27) | cU6-1 shlrr |
| TD175 | GAATTGTGGGACGGCGGAAG (SEQ ID NO:28) | cU6-4 |
| TD176 | CAGACAGACGTCAGGCTTTC(SEQ ID NO:29) | cU6-3 |
| TD216 | CTCGAGTTCCAAAAAAGGATCTTATTTCTTCGGAGTCTCTTGAACTCCGAAGAAATAAGATCCAAACCC CAGTGTCTCTCGGA (SEQ ID NO:30) | cU6-4 shNP-1496 |
| TD217 | CTCGAGTTCCAAAAAACACTACAGCTAAGGCTATGGAGCAAATTCTCTTGAAATTTGCTCCATAGCCTT AGCTGTAGTGGACTAAGAGCATCGAGACTG (SEQ ID NO:31) | cU6-3 shMP-592 |
| TD218 | CTCGAGTTCCAAAAAAGCAATTGAGGAGTGCCTGATCTCTTGAATCAGGCACTCCTCAATTGCGAATA TCTCTACCTCCTAGG (SEQ ID NO:32) | cU6-1 shPA-2087 |
| TD232 | GTCGACCGAAGAACCGAGCGCTGC (SEQ ID NO:33) | TD135 + Sall |
| TD233 | GTCGACGAATTGTGGGACGGCGGAAG (SEQ ID NO:34) | TD175 + Sall |
| TD234 | GTCGACCAGACAGACGTCAGGCTTTC (SEQ ID NO:35) | TD176 + Sall |
| TD269 | GAGGCTCAGTGTCACGCAGA (SEQ ID NO:36) | c7SK |
| TD274 | CTCGAGTTCCAAAAAAGATCTGTTCCACCATTGAATCTCTTGAATTCAATGGTGGAACAGATCAAACCC CAGTGTCTCTCGGA (SEQ ID NO:37) | cU6-4 shPB1-2257 |
| TD275 | CTCGAGTTCCAAAAAACGGGACTCTAGCATACTTATCTCTTGAATAAGTATGCTAGAGTCCCGGAATAT CTCTACCTCCTAGG (SEQ ID NO:38) | cU6-1 shPB2-2240 |
| TD302 | CTCGAGTTCCAAAAAAATCTTATTTCTTCGGAGACAATCTCTTGAATTGTCTCCGAAGAAATAAGATAAA CCCCAGTGTCTCTCGGA (SEQ ID NO:39) | cU6-4 shNP-1484 |
| TD306 | GTCGACGAGGCTCAGTGTCACGCAG (SEQ ID NO:40) | TD269 + Sall |
| TD307 | CTCGAGTTCCAAAAAACAGGATACACCATGGATACTCTCTTGAAGTATCCATGGTGTATCCTGAAAGCT ACGAGCTGCCCCAA (SEQ ID NO:41) | c7SK shPB1-129 |
| TD316 | CTCGAGTTCCAAAAAAGATCTGTTCCACCATTGAATCTCTTGAATTCAATGGTGGAACAGATCAAAGCT ACGAGCTGCCCCAA (SEQ ID NO:42) | c7SK shPB1-2257 |
| TD343 | CTCGAGTTCCAAAAAAATCTTATTTCTTCGGAGACAATCTCTTGAATTGTCTCCGAAGAAATAAGATGA CTAAGAGCATCGAGACTG (SEQ ID NO:51) | cU6-3 shNP-1484 |

In all of the HA assay experiments summarised in Table 5, plasmids expressing shPB1-2257, shNP-1484 and shNP-1496 were able to very effectively inhibit the production of both PR8 and HSN1 viruses compared to the mock plasmid. In the case of shPB1-2257 and shNP-1484, they were able to completely inhibit replication of both viruses in a number of experiments, confirming their effectiveness. Plasmids expressing shPA-2087 and shMP-592 were also able to effectively inhibit production of the viruses, but not as effectively as shPB1-2257, shNP-1484 and shNP-1496. The shPB1-129 molecule inhibited the production of the low pathogenic PR8 strain but did not inhibit the highly pathogenic H5N1 strain. Finally, despite being initially identified as a potential target shRNA sequence, shPB2-2240 was unable to inhibit the replication of either virus tested.

### Example 5. Construction of Multi-Warhead (MWH) transgenes

It has been determined that it is of significant benefit to express multiple shRNAs from the one transgene to further reduce the risk of viral target sequence variability to an RNAi strategy. These "Multi-Warhead" (MWH) transgenes are comprised of multiple transcription units, each with a different chicken pol III promoter (cU6-1, cU6-3, cU6-4 and c7SK) expressing individual shRNA molecules targeting the conserved sequences of different influenza A genes described above. The promoter sequences are native to chickens and the small 21 bp shRNA sequences would already be present in AI infected or vaccinated birds. The RNAi targets are absolutely specific to influenza A viruses and so there would be no off target effects from such a specific transgene.

Four MWH transgenes were constructed from the selected shRNAs as follows:

### a. MWH 1 - cU6-3 shMP-592; c U6-1 shPA-2087; cU6-4 shNP-1496

Each MWH transgene contains 3 transcription units that independently express a single shRNA molecule from a chicken pol III promoter. The 3 individual transcription units were amplified using a one step PCR and the resultant fragments were then ligated together to produce the MWH transgene (Figure 2). The MWH can then express 3 individual shRNAs from a single transgene. The three transcription units for MWH 1 are; cU6-4 shNP-1496; cU6-3 shMP-592 and; cU6-1 shPA-2087. The cU6-4 shNP-1496 transcription unit was amplified using forward primer TD233 and reverse primer TD216, the cU6-3 shMP-592 transcription unit was amplified using forward

**Table 5. Effects of shRNAs on influenza virus production in MDCK cells. Numbers in parentheses are multiplicity of infection (moi) values.**

| | | **Virus production (titer in HA units)** | | | | | | | |
|---|---|---|---|---|---|---|---|---|---|
| **Exp** | **shRNA expression construct** | PR8 (0.1) | PR8 (0.01) | PR8 (0.001) | PR8 (0.0001) | PR8 (0.00001) | H5N1 (0.01) | H5N1 (0.001) | H5N1 (0.0001) |
| 1 | Mock | 512 | 256 | 64 | 16 | | | | |
| | pcU6-4 shNP-1484 | 256 | 128 | 16 | 4 | | | | |
| | pc7SK shPB1-2257 | 128 | 16 | 4 | 0 | | | | |
| | pcU6-4 shPB1-2257 | 128 | 32 | 4 | 0 | | | | |
| | | | | | | | | | |
| 2 | Mock | | | 256 | 128 | 128 | | | |
| | pcU6-4 shNP-1496 | | | 64 | 32 | 8 | | | |
| | pcU6-4 shNP-1484 | | | 32 | 16 | 2 | | | |
| | pcU6-4 shPB1-2257 | | | 0 | 0 | 0 | | | |
| | pcU6-1 shPB2-2240 | | | 256 | 128 | 128 | | | |
| | pc7SK shPB1-129 | | | 128 | 64 | 32 | | | |
| | | | | | | | | | |
| 3 | Mock | | | 64 | 128 | 64 | | | |
| | pcU6-4 shNP-1496 | | | 32 | 8 | 2 | | | |
| | pcU6-4 shNP-1484 | | | 0 | 0 | 0 | | | |
| | pcU6-4 shPB1-2257 | | | 0 | 0 | 0 | | | |
| | pcU6-1 shPA-2087 | | | 128 | 128 | 32 | | | |
| | pcU6-3 shMP-592 | | | 128 | 32 | 32 | | | |
| | | | | | | | | | |
| 4 | Mock | | | | | | 64 | 16 | 4 |
| | pcU6-4 shNP-1484 | | | | | | 0 | 0 | 0 |
| | pcU6-3 shNP-1484 | | | | | | 0 | 0 | 0 |
| | pcU6-4 shPB1-2257 | | | | | | 0 | 0 | 0 |
| | | | | | | | | | |
| 5 | Mock | | | | | | 64 | 32 | 16 |
| | pcU6-4 shNP-1496 | | | | | | 2 | 2 | 2 |
| | pcU6-4 shPB1-2257 | | | | | | 0 | 0 | 0 |
| | pc7SK shPB1-2257 | | | | | | 8 | 16 | 16 |
| | | | | | | | | | |
| 6 | Mock | | | 64 | 32 | 8 | | | |
| | pcU6-4 shNP-1496 | | | 16 | 8 | 2 | | | |
| | pcU6-1 shPA-2087 | | | 32 | 16 | 8 | | | |
| | pcU6-3 shMP-592 | | | 32 | 8 | 4 | | | |
| | pcU6-4 shPB1-2257 | | | 0 | 0 | 0 | | | |
| | | | | | | | | | |
| 7 | Mock | | | | | | 64 | 64 | 32 |
| | pcU6-4 shNP-1496 | | | | | | 8 | 2 | 0 |
| | pcU6-1 shPA-2087 | | | | | | 64 | 32 | 16 |
| | pcU6-3 shMP-592 | | | | | | 32 | 16 | 4 |
| | pcU6-4 shPB1-2257 | | | | | | 0 | 0 | 0 |
| | pcU6-1 shPB2-2240 | | | | | | 64 | 64 | 32 |
| | pc7SK shPB1-129 | | | | | | 64 | 64 | 32 |
| | | | | | | | | | |
| 8 | Mock | | | | | | 64 | 64 | 32 |
| | MWH 1 | | | | | | 32 | 16 | 8 |
| | MWH2 | | | | | | 64 | 64 | 16 |
| | MWH3 | | | | | | 8 | 8 | 4 |
| | MWH4 | | | | | | 4 | 4 | 0 |

primer TD234 and reverse primer TD217, and the cU6-1 shPA-2087 transcription unit was amplified using forward primer TD232 and reverse primer TD218 (primer details are described in Table 4). Each of the PCR products was cloned into pGEM-T Easy and each contain a 5' *SalI* restriction enzyme site and a 3' *XhoI* restriction enzyme site. Both of these restriction sites have compatible overhangs which allowed the sequential ligation of the individual transcription units together, to produce the final MWH transgene (Figure 2).

### b. MWH 2 - cU6-4 shPB1-2257; cU6-1 shPB2-2240; c7SK shPB1-129

The three transcription units for MWH 2 are; cU6-4 shPB1-2257; cU6-1 shPB2-2240 and; c7SK shPB1-129. The cU6-4 shPB1-2257 transcription unit was amplified using forward primer TD233 and reverse primer TD274, the cU6-1 shPB2-2240 transcription unit was amplified using forward primer TD232 and reverse primer TD275, and the c7SK shPB1-129 transcription unit was amplified using forward primer TD306 and reverse primer TD307 (primer details are described in Table 4). Each of the PCR products was cloned into pGEM-T Easy and sequentially ligated to construct the final MWH transgene as described above and in Figure 2.

### c.MWH3 - cU6-4shNP-1484; cU6-1 shPA-2087; c7SK shPB1-2257

The three transcription units for MWH 3 are; cU6-4 shNP-1484; cU6-1 shPA-2087 and; c7SK shPB1-2257. The cU6-4 shNP-1484 transcription unit was amplified using forward primer TD233 and reverse primer TD302, the cU6-1 shPA-2087 transcription unit was amplified using forward primer TD232 and reverse primer TD218, and the c7SK shPB1-2257 transcription unit was amplified using forward primer TD306 and reverse primer TD316 (primer details are described in Table 4). Each of the PCR products was again cloned into pGEM-T Easy and sequentially ligated to construct the final MWH transgene as described above and in Figure 2.

### d.MWH 4 - cU6-4 shPB1-2257; cU6-3 shNP-1484; cU6-1 shPA-2087

The three transcription units for MWH 4 are; cU6-4 shPB1-2257; cU6-3 shNP-1484 and; cU6-1 shPA-2087. The cU6-4 shPB1-2257 transcription unit was amplified using forward primer TD233 and reverse primer TD274, the cU6-3 shNP-1484 transcription unit was amplified using forward primer TD234 and reverse primer TD343, and the cU6-1 shPA-2087 transcription unit was amplified using forward primer TD232 and reverse primer TD218 (primer details are described in Table 4). Each of the PCR products was again cloned into pGEM-T Easy and sequentially ligated to construct the final MWH transgene as described above and in Figure 2.

The four final MWH transgenes were also tested for their ability to inhibit virus production in an HA assay using H5N1 influenza A virus (Table 4, Experiment 8). MWH 3 and 4 were the most effective transgenes. MWH 1 also effectively inhibited H5N1 virus production, while MWH 2 was not as effective as MWH 1.

### Example 6. Cloning of MWH transgenes into pStuffit vector

Each MWH was cloned into the pSuffit plasmid (Figure 3). This plasmid facilitates insertion of the MWH transgenes between stuffer/buffer fragments of chicken genomic DNA to potentially protect the MWH sequences from both the transgene insertion process and external transcription read through. The ME1 and GRM5 stuffer fragments were selected from large intronic sequences from the chicken genome (i.e genomic deserts) and are devoid of transcriptional elements that could interfere with expression of the MWH transgene. The specific regions as described in GenBank are: ME1 1500 (chr3) gb|AADN02002420.1 30995-32489 bp ; ME1 200 (chr 3) gb|AADN02002420.1 5079-5276 bp and; GRM5 1500 (chr 1) gb|AADN02004814.1 13141-13113, 13078-12911, 12848-11638 bp; GRM5 200 (chr 1) gb|AADN02004814.1 10126-9927 bp.

### Plasmid pStuffit construction

Plasmid pStuffit was constructed by cloning four regions of the chicken genome, in a specific order dictated by use of restriction enzyme sites, into the pIC20H cloning vector (Figure 3). Fragments, as listed in Table 6, were first PCR amplified using the primers listed in Table 7 and then cloned individually into pGEM-T Easy (Invitrogen) and sequenced. These fragments were then excised from pGEM-T Easy and cloned sequentially using the restriction enzyme sites listed in Table 5. Firstly, GRM5 200 was cloned into pIC20H followed by ME1 200, GRM5 1500 and ME1 1500. At each cloning stage the resulting plasmid was checked by restriction enzyme digest and DNA sequencing. The final assembled plasmid was designated pStuffit.

**Table 6. pStuffit construction. Cloned fragment designations and the primers used in their amplification.**

| Fragment Name | Primers | Enzyme sites |
|---|---|---|
| GRM5 200 | TD277 / TD278 | *EcoRI* / EcoRV |
| ME1 200 | TD281 / TD282 | *BamHI* / EcoR*I* |
| GRM5 1500 | TD279 / TD280 | EcoRV / *XhoI* |
| ME1 1500 | TD283 / TD284 | *SphI* / BamH*I* |

**Table 7. pStuffit construction. PCR primer designations and sequence. Restriction enzyme sites are underlined.**

| Primer name | Primer sequence | Cloning enzyme |
|---|---|---|
| TD277 | GAA TTC CAT ACC ACT GCG AGG GTG CCA AGT CAT GGG ACT GAT ACT C (SEQ ID NO:43) | EcoRI |
| TD278 | GAT ATC TTA ATT AAC TGG AAG GTT GCA GTA AG (SEQ ID NO:44) | EcoRV |
| TD279 | GAT ATC TTG TCC CTT CCA GGA ACA G (SEQ ID NO:45) | EcoRV |
| TD280 | CTC GAG ATT TAA ATA GAT TGC AGC ACA AGG AG (SEQ ID NO:46) | XhoI |
| TD281 | GGA TCC TTA ATT AAC TGG AAA CTA GGA CGT GGA AG (SEQ ID NO:47) | BamHI |
| TD282 | GAA TTC CGA GAC CAT CCA CGT GCT GCT TAC TGC AGC TAC GTC GAA TG (SEQ ID NO:48) | EcoRI |
| TD283 | GCA TGC ATT TAA ATG ACA GCA GCA GGT GAA AGA C (SEQ ID NO:49) | *Sph*I |
| TD284 | GGA TCC TCA AGT GGG TGC TCA GGA AG (SEQ ID NO:50) | BamHI |

### Insertion of MWH transgenes into pStuffit

The pStuffit vector has a unique EcoRI restriction site located between the GRM5 200 and ME1 200 sequences to permit the insertion of each MWH transgene. Each MWH transgene was inserted into pStuffit by ligation into this EcoRI restriction site. Also included were *PacI* and *Swa*I to allow for the excision of the construct with varying amounts of flanking sequence (Figure 3). The *Hind*III restriction enzyme sites of the pIC20H vector can be used to excise the entire cloned sequence. Therefore the final pStuffit plasmids containing each of the MWH inserts, was digested with HindIII restriction enzyme to release the final insert to be purified and used for the sperm mediated gene transfer (SMGT) process.

### Example 7. Linker based sperm-mediated gene transfer

The process of delivering the construct into a fertilised chicken ovum can be achieved by linker based sperm-mediated gene transfer. This procedure is carried out as described in US 7,067,308. Briefly, freshly harvested chicken semen is washed and incubated with murine monoclonal antibody mAbC (secreted by the hybridoma assigned ATCC accession number PTA-6723) and then the construct DNA. The added monoclonal antibody aids in the binding of the DNA to the semen. The sperm/DNA complex is then artificially inseminated into hens. The process is repeated four times with 72 hours between inseminations. Eggs are collected daily from two days after the first insemination until 3 days after the final insemination.

### Example 8. Insertion of MWH transgenes into Tol2 and delivery to chickens

MWH 3 (SEQ ID NO:21) and MWH 4 (SEQ ID NO:61) transgenes were cloned into the Tol2 transposon vector pminiTol2/MCS ((SEQ ID NO:64); Balciunas et al., 2006). Both transgenes were removed from pGEM-T Easy vector by double digestion with *SalI* and *XhoI.* This fragment was then ligated into the unique *Xho*I site within the multiple cloning site of the Tol2 transposon vector.

The process of delivering the MWH 3 Tol2 construct (SEQ ID NO:62) and MWH 4 Tol 2 construct (SEQ ID NO:63) into a chicken embryo can be achieved by using Primordial Germ Cells (PGCs). Briefly, PGCs are harvested from donor chicken embryos, either from the blood when the embryo is 2 days old or from the gonads of a 5.5 day old embryo. The PGCs are purified from the blood or gonadal tissue by using Magnetic Antibody Cell Separation (MACS). The purified PGCs are then electroporated with the Tol2 constructs and a separate plasmid encoding the Tol2 transposase (pCMV-Tol2; SEQ ID NO:65; Balciunas et al., 2006) using an Amaxa Nucleofector. These cells are then injected back into a recipient embryo that is 2.5 days old. The transformed PGCs migrate to establish the gonads of the developing embryo.

The process of delivering the Tol2 contrsucts into a chicken embryo can also be achieved by direct electroporation of the blastoderm of a freshly laid egg. Briefly, a freshly laid fertilized egg is opened to reveal the blastoderm The blastoderm is injected with Tol2 construct DNA using a microcapillary pipette together with a plasmid encoding the Tol2 transposase. The blastoderm is then electroporated *in ovo* using a BTX ECM830 Electro Square Porator. PGCs are located in the center of the blastoderm and if these cells are transformed with the construct after electroporation, they will proceed to become germ cells within the gonads of a developing embryo.

### Example 9. Screening GO progeny for transgenic

A small quantity of blood is taken from either the wing vein or feather tip of 1 week-old G0 progeny. Genomic DNA is prepared from wing vein blood using a QIAmp DNA Blood Mini kit (Qiagen). DNA from the feather tip blood is prepared using QuickExtract™ DNA Extraction Solution (Epicentre Biotechnologies) Two tests are carried out on these samples to confirm the presence of the construct.

### Southern Blot

PCR is carried out on the genomic samples using the forward and reverse primers listed in Table 8. The PCR mixture is then run on an agarose gel, transferred to a membrane and hybridised with a radioactively labelled locked nucleic acid probe (Table 8). After hybridisation and washing in a high stringency solution, the membrane is exposed to X-ray film. A positive is indicated by a band of the correct size being detected on the resultant autoradiograph.

**Table 8. Oligonucleotides used in Southern blot PCR analysis. Underlined characters indicate the locked nucleic acid bases.**

| Function | Designation | Sequence |
|---|---|---|
| Forward primer | TD320 | TTG CCC CCA AAC AGC AA (SEQ ID NO:55) |
| Reverse primer | TD321 | GAC CAT CCA CGT GCT GCT TA (SEQ ID NO:56) |
| Probe | TD319 | CAT TCG ACG TAG CTG CA (SEQ ID NO:57) |

### Real-Time Quantitative PCR

Real-Time PCR is carried out on the genomic samples using the primers listed in Table 9. The assay uses the binding of SYBR Green reagent to double stranded DNA and subsequent melting curve analysis to determine a positive sample.

**Table 9. Primers used in Real-Time PCR analysis.**

| Function | Designation | Sequence |
|---|---|---|
| Forward primer | BC_F_03 | GCA GCA CGT GGA TGG TCT C (SEQ ID NO:58) |
| Reverse primer (promoter cU6-4) | TD251 | TCT TCC GCC GTC CCA CAA TT (SEQ ID NO:59) |
| Reverse primer (promoter cU6-3) | TD252 | GCT TAG AAA GCC TGA CGT CT (SEQ ID NO:60) |

### Example 10. Testing for transgenic birds

### LB-SMGT

A bird identified as transgenic in the G0 population will be retained and housed until sexually mature. Once sexually mature the bird is used in mating experiments to generate G1 transgenic progeny that have a copy of the construct in every cell. Southern blot and real-time PCR is again used to show the transgenic nature of the G1 progeny.

More detailed analysis using genomic Southern blots and PCR regarding the insertion site and copy number of the construct is carried out on these birds.

G1 birds identified as possessing a relevant construct insertion are raised until sexual maturity. Some of the G2 offspring from these birds are used in animal trials to verify their resistance to various strains of avian influenza. Other G2 birds are analysed for expression of the construct in various tissues and at various ages.

### Tol2 Transposon

G0 embryos that have either received the Tol 2 transformed PGCs or have been electroporated with Tol2 construct will hatch. Only G0 male chicks will be kept and will be raised until sexual maturity. Semen will be collected from the male birds and PCR will be done to confirm if any of the birds contain the relevant construct. Birds that are PCR positive will be used in mating experiments to generate G1 transgenic progeny that have a copy of the construct in every cell. Southern blot and real-time PCR is again used to show the transgenic nature of the G1 progeny.

### Example 11. Model system - Influenza A resistant transgenic mouse

The present inventors constructed two shRNA transgene cassettes for generation of transgenic mice. Each cassette contained the mouse U6 promoter for expression of either shNP-1496 or shEGFP. Both transgenes were then used to generate transgenic mice using lentiviral technology. Briefly, the shNP-1496 and shEGFP shRNA transgene cassettes were cloned into the lentiviral gene transfer vector (AusGene, Bentleigh, Australia). Transgenic viral constructs were then packaged into lentiviral particles. Lentiviral titers were determined and lentiviral particles were injected into the perivitelline space of early stage mouse embryos. The embryos were re-implanted into pseudo-pregnant female mice and the resulting offspring were screened by southern blot analysis. Transgenic founder mice were obtained that had stable integration of either transgene. The founders were then mated with wild type mice to generate F1 progeny. Transgenic F1 mice were then tested in a challenge experiment for resistance to Influenza A infection.

The challenge experiment was made up of 3 groups, each containing 5 mice. Groups 1 and 2 each contained 5 mice with the shNP-1496 shRNA. Group 3 contained 5 mice with the shEGFP shRNA. Groups 2 and 3 received an intranasal challenge of 5 x 10² TCID₅₀ of low pathogenic H1N1 A/PR/8/34 (PR8) Influenza A virus. Group 1 was challenged with phosphate buffered saline with no virus. Body weight was monitored daily for 10 days post challenge and at the end of the experiment, the mice were euthanized and lung sample were taken for qPCR measurement of viral RNA.

As shown in Figure 4, the transgenic mice with the shNP-1496 trangene had excellent levels of resistance to infection compared to mice with the irrelevant shEGFP transgene. The shNP-1496 mice did not lose body weight during the course of the experiment when compared to the PBS control group. By comparison, the shEGFP mice showed a statistically significant decline in body weight, indicating active infection with the influenza virus. When viral RNA was measured in lung samples from mice in Groups 2 and 3, mice with the shNP-1496 transgene had greater than a 90% decrease in viral RNA compared with mice containing the irrelevant shEGFP transgene. Overall these results indicate that transgenic mice containing a shRNA molecule that specifically targets the Influenza A virus, such as shNP-1496, are highly resistant to an experimental challenge with H1N1 A/PR/8/34 (PR8) Influenza A virus.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

All publications discussed and/or referenced herein are incorporated herein in their entirety.

The present application claims priority from US 60/938,315 and AU 2007902616, the entire contents of which are incorporated herein by reference.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

In summary, the invention relates to the following aspects :
1. A nucleic acid construct encoding an RNA molecule comprising a double-stranded region, wherein the RNA molecule reduces influenza A virus replication in an animal cell and/or reduces production of infectious influenza A virus particles in an animal cell and/or reduces the expression of an influenza A virus polypeptide in an influenza A virus infected animal cell when compared to an isogenic influenza A virus infected animal cell lacking the RNA molecule.
2. The nucleic acid construct of aspect 1, wherein the double-stranded region comprises a sequence of nucleotides selected from:
   (i) nucleotides within positions 2240 to 2341 of SEQ ID NO:1,
   (ii) nucleotides within positions 2257 to 2341 of SEQ ID NO:2,
   (iii) nucleotides within positions 2087 to 2233 of SEQ ID NO:3,
   (iv) nucleotides within positions 1484 to 1565 of SEQ ID NO:4,
   (v) a nucleotide sequence of any one of SEQ ID NOs:6 to 15 or 52 to 54,
   (vi) a nucleotide sequence which is at least 90% identical to any one of (i) to (v),
   (vii) a nucleotide sequence which hybridizes to any one of (i) to (v) under stringent conditions.
3. The nucleic acid construct of aspect 2, wherein the RNA molecule reduces influenza A virus replication in an animal cell when compared to an isogenic influenza A virus infected animal cell lacking the RNA molecule.
4. The nucleic acid construct of aspect 2, wherein the RNA molecule reduces production of infectious influenza A virus particles in an animal cell when compared to an isogenic influenza A virus infected animal cell lacking the RNA molecule.
5. The nucleic acid construct of aspect 2, wherein the RNA molecule reduces the expression of an influenza A virus polypeptide in an influenza A virus infected animal cell when compared to an isogenic influenza A virus infected animal cell lacking the RNA molecule.
6. The nucleic acid construct of any one of aspects 1 to 5, wherein the double stranded region is at least 19 basepairs in length.
7. The nucleic acid construct of any one aspects 1 to 6, wherein the double stranded region is less than 100 basepairs in length.
8. The nucleic acid construct of any one of aspects 1 to 7, wherein the RNA molecule is a short hairpin RNA.
9. The nucleic acid construct of any one of aspects 1 to 8, wherein the double-stranded region encoded by the RNA molecule comprises the sequence of nucleotides of SEQ ID NO:7.
10. The nucleic acid construct of any one of aspects 1 to 8, wherein the double-stranded region encoded by the RNA molecule comprises the sequence of nucleotides of SEQ ID NO:9.
11. The nucleic acid construct of any one of aspects 1 to 8, wherein the double-stranded region encoded by the RNA molecule comprises the sequence of nucleotides of SEQ ID NO:12.
12. The nucleic acid construct of any one of aspects 1 to 8, wherein the double-stranded region encoded by the RNA molecule comprises the sequence of nucleotides of SEQ ID NO:6.
13. The nucleic acid construct of any one of aspects 1 to 8, wherein the double-stranded region encoded by the RNA molecule comprises the sequence of nucleotides of SEQ ID NO:8.
14. The nucleic acid construct of any one of aspects 1 to 8, wherein the double-stranded region encoded by the RNA molecule comprises the sequence of nucleotides of SEQ ID NO:13.
15. The nucleic acid construct of any one of aspects 1 to 8, wherein the double-stranded region encoded by the RNA molecule comprises the sequence of nucleotides of SEQ ID NO:15.
16. The nucleic acid construct of any one of the preceding aspects, wherein the construct encodes two or more RNA molecules.
17. The nucleic acid construct of aspect 16, wherein each RNA molecule comprises a nucleotide sequence corresponding to a different influenza A virus gene.
18. The nucleic acid construct of aspect 16 or aspect 17, wherein each RNA molecule is encoded by a nucleotide sequence operably linked to a RNA polymerase II promoter or a RNA polymerase III promoter.
19. The nucleic acid construct of aspect 18, wherein the promoters are RNA polymerase III promoters.
20. The nucleic acid construct of aspect 18 or aspect 19, wherein the promoters are chicken, turkey and/or duck promoters.
21. The nucleic acid construct of aspect 19 or aspect 20, wherein the promoters are selected from a U6, 7SK and/or H1 promoter.
22. The nucleic acid construct of aspect 21, wherein the U6 promoter is cU6-1, cU6-2, cU6-3, and/or cU6-4.
23. The nucleic acid construct of any one of aspects 19 to 22, wherein each nucleotide sequence encoding a RNA molecule is operably linked to a different RNA polymerase III promoter.
24. The nucleic acid construct of any one of aspects 1 to 23, wherein the influenza A virus polypeptide is selected from PB1, PB2, PA, NP and/or M1.
25. The nucleic acid construct of any one of aspects 1 to 24, wherein the influenza A virus polypeptide is encoded by any one of SEQ ID NOs:1 to 5.
26. The nucleic acid construct of any one of aspects 1 to 25, wherein the polypeptide is an avian influenza polypeptide.
27. The nucleic acid construct of aspect 26, wherein the avian influenza is H5N1.
28. The nucleic acid construct of any one of aspects 1 to 27, wherein the construct consists of chicken and influenza A virus nucleotide sequences.
29. The nucleic acid construct of any one of aspects 1 to 28 which encodes three RNA molecules comprising a double-stranded region, wherein the double-stranded regions comprise nucleotide sequences selected from:
   (i) SEQ ID NO:9, SEQ ID NO:13 and SEQ ID NO:15,
   (ii) SEQ ID NO:6, SEQ ID NO:7 and SEQ ID NO:8, and
   (iii) SEQ ID NO:7, SEQ ID NO:9 and SEQ ID NO:12.
30. The nucleic acid construct of any one of aspects 1 to 28 comprising a nucleotide sequence selected from SEQ ID NOs:16 to 21 and 61 to 63, or a fragment thereof, or a sequence that is at least 95% identical to a nucleotide sequence selected from SEQ ID NOs:16 to 21 and 61 to 63.
31. An isolated and/or exogenous nucleic acid molecule comprising a double-stranded region, wherein the double-stranded region comprises a sequence of nucleotides selected from:
   (i) nucleotides within positions 2240 to 2341 of SEQ ID NO:1,
   (ii) nucleotides within positions 2257 to 2341 of SEQ ID NO:2,
   (iii) nucleotides within positions 2087 to 2233 of SEQ ID NO:3,
   (iv) nucleotides within positions 1484 to 1565 of SEQ ID NO:4,
   (v) a nucleotide sequence of any one of SEQ ID NOs:6 to 15 and 52 to 54,
   (vi) a nucleotide sequence which is at least 90% identical to any one of (i) to (v),
   (vii) a nucleotide sequence which hybridizes to any one of (i) to (v) under stringent conditions.
32. The isolated and/or exogenous nucleic acid molecule of aspect 31, wherein the nucleic acid molecule reduces influenza A virus replication in an animal cell when compared to an isogenic influenza A virus infected animal cell lacking the nucleic acid molecule.
33. The isolated and/or exogenous nucleic acid molecule of aspect 31 or aspect 32, wherein the nucleic acid molecule reduces the production of infectious influenza A virus particles in an animal cell when compared to an isogenic influenza A virus infected animal cell lacking the nucleic acid molecule.
34. The isolated and/or exogenous nucleic acid molecule of any one of aspects 31 to 33, wherein the nucleic acid molecule reduces the expression of an influenza A virus polypeptide in an influenza A virus infected animal cell when compared to an isogenic influenza A virus infected animal cell lacking the nucleic acid molecule.
35. The isolated and/or exogenous nucleic acid molecule of aspect 34, wherein the nucleic acid molecule reduces the expression of an influenza A virus polypeptide encoded by any one of SEQ ID NOs:1 to 5.
36. The isolated and/or exogenous nucleic acid molecule of any one of aspects 31 to 35, wherein the double-stranded region is at least 19 nucleotides in length.
37. The isolated and/or exogenous nucleic acid molecule of any one of aspects 31 to 36, wherein the double-stranded region is less than 100 nucleotides in length.
38. The isolated or exogenous nucleic acid molecule of any one of aspects 31 to 37, wherein the molecule comprises double-stranded RNA.
39. The isolated and/or exogenous nucleic acid molecule of any one of aspects 31 to 38 which is a short interfering RNA or a short hairpin RNA.
40. The isolated and/or exogenous nucleic acid molecule of any one of aspects 31 to 39 comprising a nucleotide sequence selected from SEQ ID NOs:16 to 21 and 61 to 63, or a fragment thereof, or a sequence that is at least 95% identical to a nucleotide sequence selected from SEQ ID NOs:16 to 21 and 61 to 63.
41. A vector comprising the nucleic acid construct of any one of aspects 1 to 30 and/or the isolated and/or exogenous nucleic acid of any one of aspects 31 to 40.
42. A cell comprising the nucleic acid construct of any one of aspects 1 to 30, the isolated and/or exogenous nucleic acid or any one of aspects 31 to 40 and/or the vector of aspect 41.
43. The cell of aspect 42 which is a chicken primordial germ cell.
44. A transgenic non-human organism comprising the nucleic acid construct of any one of aspects 1 to 30, the isolated and/or exogenous nucleic acid of any one of aspects 31 to 40 , the vector of aspect 41 and/or the cell of aspect 42 or aspect 43.
45. The transgenic organism of aspect 44 which is a non-human animal.
46. The transgenic organism of aspect 45 which is avian.
47. The transgenic organism of aspect 46 which is poultry.
48. The transgenic organism of aspect 47 which is a chicken, turkey or duck.
49. The transgenic organism of any one of aspects 44 to 48 which comprises a nucleotide sequence selected from SEQ ID NOs:16 to 21 or 61 to 63, or a fragment thereof, or a sequence that is at least 95% identical to SEQ ID NOs:16 to 21 or 61 to 63, encoding at least one RNA molecule comprising a double-stranded region.
50. A composition comprising the nucleic acid construct of any one of aspects 1 to 30, the isolated and/or exogenous nucleic acid molecule of any one of aspects 31 to 40, the vector of aspect 41, and/or the cell of aspect 42 or aspect 43.
51. A method of treating and/or preventing an influenza A virus infection in a subject, the method comprising administering to the subject the nucleic acid construct of any one of aspects 1 to 30, the isolated and/or exogenous nucleic acid molecule of any one of aspects 31 to 40, the vector of aspect 41, and/or the cell of aspect 42 or aspect 43.
52. The method of aspect 51, wherein the method comprises administering the nucleic acid construct, the isolated and/or exogenous nucleic acid molecule, the vector, and/or the cell in drinking water or in an aerosol.
53. The method of aspect 51 or 52, wherein the influenza A virus is avian influenza.
54. The method of any one of aspects 51 to 53, wherein the subj ect is avian.
55. The method of aspect 54, wherein the subject is poultry.
56. The method of aspect 55, wherein the subject is a chicken, turkey or duck.
57. A method of reducing the expression of one or more influenza A virus genes in a cell, the method comprising administering to the cell the isolated and/or exogenous nucleic acid molecule of any one of aspects 31 to 40.
58. Use of the nucleic acid construct of any one of aspects 1 to 30, the isolated and/or exogenous nucleic acid molecule of any one of aspects 31 to 40 , the vector of aspect 41, and/or the cell of aspect 42 or aspect 43 in the manufacture of a medicament for treating and/or preventing influenza A virus infection.
59. A method of identifying an animal comprising the nucleic acid construct of any one of aspects 1 to 30 or the isolated and/or exogenous nucleic acid molecule of any one of aspects 31 to 40, the method comprising:
   determining the presence or absence of the nucleic acid construct of any one of aspects 1 to 30 and/or the isolated and/or exogenous nucleic acid molecule of any one of aspects 31 to 40 in a sample obtained from the animal.
60. The method of aspect 59, wherein the method comprises amplifying the nucleic acid construct or a fragment thereof, or the isolated and/or exogenous nucleic acid molecule or a fragment thereof.
61. The method of aspect 59 or aspect 60, wherein the method comprises:
   contacting a sample obtained from the animal with a probe that hybridizes under stringent conditions with the nucleic acid construct and/or the isolated and/or exogenous nucleic acid molecule to form a complex, and
   determining the presence or absence of the complex.
62. A method of breeding a non-human transgenic animal resistant to influenza, the method comprising:
   (i) introducing the nucleic acid construct of any one of aspects 1 to 30 into a non-human animal cell,
   (ii) selecting a transgenic non-human cell comprising the nucleic acid construct,
   (iii) regenerating a transgenic non-human animal from the transgenic non-human cell,
   (iv) breeding the transgenic non-human animal to produce transgenic progeny, and
   (v) selecting transgenic progeny that are resistant to influenza.
63. A method of producing food, the method comprising
   (i) introducing the nucleic acid construct of any one of aspects 1 to 30 into an animal cell,
   (ii) selecting a transgenic cell comprising the nucleic acid construct,
   (iii) regenerating a transgenic animal from the transgenic cell,
   (iv) breeding the transgenic animal to produce transgenic progeny, and
   (v) producing food from the transgenic progeny.
64. A method of making a transgenic non-human animal, the method comprising:
   (i) introducing a first nucleic acid comprising a transposon into a cell, wherein the nucleic acid encodes a double-stranded RNA molecule,
   (ii) introducing a second nucleic acid encoding a transposase into the cell,
   (ii) selecting a transgenic cell comprising the first nucleic acid in the genome of the cell,
   (iii) regenerating a transgenic non-human animal from the cell, and
   (iv) breeding the transgenic non-human animal.
65. The method of aspect 64, wherein the transposon is a Tol2 transposon and the transposase is Tol2 transposase.
66. The method of aspect 65, wherein the cell is a chicken primordial germ cell.
67. A transgenic non-human animal resistant to influenza.
68. The transgenic non-human animal of aspect 67, wherein the transgenic animal comprises a nucleic acid construct encoding an RNA molecule comprising a double-stranded region, wherein the RNA molecule reduces influenza virus replication in a cell of the animal when compared to an isogenic influenza virus infected animal cell lacking the RNA molecule.
69. The transgenic non-human animal of aspect 67, wherein the transgenic animal comprises a nucleic acid construct encoding an RNA molecule comprising a double-stranded region, wherein the RNA molecule reduces production of infectious influenza virus particles in a cell of the animal when compared to an isogenic influenza virus infected animal cell lacking the RNA molecule.
70. The transgenic non-human animal of aspect 67, wherein the transgenic animal comprises a nucleic acid construct encoding an RNA molecule comprising a double-stranded region, wherein the RNA molecule reduces the expression of an influenza virus polypeptide in an influenza virus infected cell of the animal when compared to an isogenic influenza virus infected animal cell lacking the RNA molecule.
71. The transgenic non-human animal of any one of aspects 67 to 70, wherein the transgenic non-human animal is a chicken and the influenza is influenza A.
72. Use of the non-human transgenic organism of any one of aspects 44 to 49 or the transgenic non-human animal of any one of aspects 67 to 71 for breeding.
73. Use of the non-human transgenic organism of any one of aspects 44 to 49 or the transgenic non-human animal of any one of aspects 67 to 71 for food production.

### REFERENCES

Balciunas et al. (2006) PLoS Genet. 10:e169.
Bosselman et al. (1989) Science, 243:533-534.
Chim et al. (1993) Cell, 74:504-514.
Fire, et al. (1998) Nature, 391:806-811.
Freier, et al. (1986) Proc Natl Acad Sci USA, 83:9373-7.
Higashibata, et al. (2004) J Bone Miner Res, 19:78-88.
Hoggatt, et al. (2002) Circ Res, 91:1151-59.
Kawakami, et al. (2000) Proc Natl Acad Sci USA, 97:11403-11408.
Ketting, et al. (1999) Cell, 99:133-141.
Koga, et al. (1996) Nature, 383:30.
Lavitrano, et al. (1989) Cell, 57:717-723.
Love et al. (1994) Bio/Technology, 12:60-63.
Naito et al. (2006) Nucleic Acids Res, Jul 34 (WebServerIssue):W448-50.
Needleman, S.B. and Wunsch, C.D. (1970) J Mol Biol, 48: 443-453.
Nicholson, et al. (2005) Lancet, 362:1733-1745.
Ratcliff, et al. (1999) Plant Cell, 11:1207-1216.
Tabara, et al, (1999) Cell, 99:123-132.
Taxman, et al. (2006) BMC Biotechnol, Jan 24, 6:7.
Thoraval et al. (1995) Transgenic Research 4:369-36.
Zamore, et al, (2000) Cell, 101:25-33.
Zhang, et al. (2004) Genome Res 14:79-89

## Claims

1. A nucleic acid construct encoding at least three RNA molecules comprising a double-stranded region, wherein the RNA molecules reduce influenza A virus replication in an animal cell and/or reduce production of infectious influenza A virus particles in an animal cell and/or reduce the expression of an influenza A virus polypeptide in an influenza A virus infected animal cell when compared to an isogenic influenza A virus infected animal cell lacking the RNA molecule, wherein the double-stranded regions comprise nucleotide sequences at least 95% identical to sequences selected from:
(i) SEQ ID NO:7, SEQ ID NO:9 and SEQ ID NO:12,
(ii) SEQ ID NO:6, SEQ ID NO:7 and SEQ ID NO:8, and
(iii) SEQ ID NO:9, SEQ ID NO:13 and SEQ ID NO:15.

2. The nucleic acid construct of claim 1, wherein the double-stranded regions comprise nucleotide sequences at least 95% identical to SEQ ID NO:7, SEQ ID NO:9 and SEQ ID NO:12.

3. A composition comprising three isolated and/or exogenous nucleic acid molecule comprising a double-stranded region, wherein the double-stranded regions comprise nucleotide sequences at least 95% identical to sequences selected from:
(i) SEQ ID NO:7, SEQ ID NO:9 and SEQ ID NO:12,
(ii) SEQ ID NO:6, SEQ ID NO:7 and SEQ ID NO:8, and
(iii) SEQ ID NO:9, SEQ ID NO:13 and SEQ ID NO:15.

4. The composition of claim 3, wherein the double-stranded regions comprise nucleotide sequences at least 95% identical to SEQ ID NO:7, SEQ ID NO:9 and SEQ ID NO:12.

5. A vector comprising the nucleic acid construct of claim 1 or claim 2.

6. A cell comprising the nucleic acid construct of claim 1 or claim 2, the composition of claim 3 or claim 4 and/or the vector of claim 5.

7. A transgenic non-human organism comprising the nucleic acid construct of claim 1 or claim 2, the composition of claim 3 or claim 4, the vector of claim 5 and/or the cell of claim 6.

8. The transgenic organism of claim 7 which is a chicken, turkey or duck.

9. A composition comprising the nucleic acid construct of claim 1 or claim 2, the vector of claim 5, and/or the cell of claim 6.

10. The nucleic acid construct of claim 1 or claim 2, the composition of claim 3 or claim 4, the vector of claim 5, and/or the cell of claim 6, for use in treating and/or preventing an influenza A virus infection in a subject.

11. A method of reducing the expression of one or more influenza genes in a cell *in vitro,* the method comprising administering to the cell the composition of claim 3 or claim 4.

12. The composition of claim 3 or claim 4 for use in reducing the expression of one or more influenza A virus genes in a cell.

13. Use of the nucleic acid construct of claim 1 or claim 2, the composition of claim 3 or claim 4, the vector of claim 5, and/or the cell of claim 6 in the manufacture of a medicament for treating and/or preventing influenza A virus infection.

14. A method of identifying an animal comprising the nucleic acid construct of claim 1 or claim 2 or the composition of claim 3 or claim 4, the method comprising:
determining the presence or absence of the nucleic acid construct of claim 1 or claim 2 and/or the composition of claim 3 or claim 4 in a sample obtained from the animal.

15. A method of breeding a non-human transgenic animal resistant to influenza, the method comprising:
(i) introducing the nucleic acid construct of claim 1 or claim 2 into a non-human animal cell,
(ii) selecting a transgenic non-human cell comprising the nucleic acid construct,
(iii) regenerating a transgenic non-human animal from the transgenic non-human cell,
(iv) breeding the transgenic non-human animal to produce transgenic progeny, and
(v) selecting transgenic progeny that are resistant to influenza.

16. A method of producing food, the method comprising
(i) introducing the nucleic acid construct of claim 1 or claim 2 into an animal cell,
(ii) selecting a transgenic cell comprising the nucleic acid construct,
(iii) regenerating a transgenic animal from the transgenic cell,
(iv) breeding the transgenic animal to produce transgenic progeny, and
(v) producing food from the transgenic progeny.

17. A method of making a transgenic non-human animal, the method comprising:
(i) introducing a first nucleic acid comprising a transposon into a cell, wherein the nucleic acid comprises the nucleic acid construct of claim 1 or claim 2,
(ii) introducing a second nucleic acid encoding a transposase into the cell,
(ii) selecting a transgenic cell comprising the first nucleic acid in the genome of the cell,
(iii) regenerating a transgenic non-human animal from the cell, and
(iv) breeding the transgenic non-human animal.

18. A transgenic non-human animal resistant to influenza, wherein the transgenic non-human animal comprises the nucleic acid construct of claim 1 or claim 2.

19. Use of the transgenic non-human organism of claim 7 or claim 8 or the transgenic non-human animal of claim 17 for breeding and/or for food production.
